# EUROPÄISCHE PATENTSCHRIFT

(11) **EP 3 806 176 B1**
(45) Veröffentlichungstag und Bekanntmachung des Hinweises auf die Patenterteilung: **25.06.2025**
(21) Anmeldenummer: 20211768.5
(22) Anmeldetag: 11.10.2013
(51) Int. Cl.: H10K 85/60, C07D 209/86, C07D 307/91, H10K 50/11, H10K 50/15

(54) **ELEKTRONISCHE VORRICHTUNG**
ELECTRONIC DEVICE
DISPOSITIF ÉLECTRONIQUE

(30) Priorität: 31.10.2012 EP 12007456
(43) Veröffentlichungstag der Anmeldung: 14.04.2021
(62) Teilanmeldung aus: 13776421.3
(73) Patentinhaber: Merck Patent GmbH, 64293 Darmstadt (DE)
(72) Erfinder: Parham, Amir Hossain, 64293 Darmstadt (DE); Martynova, Irina, 64293 Darmstadt (DE); Montenegro, Elvira, 64293 Darmstadt (DE); Mujica-Fernaud, Teresa, 64293 Darmstadt (DE); Voges, Frank, 64293 Darmstadt (DE); Buesing, Arne, 64293 Darmstadt (DE)
(74) Vertreter: Merck Patent Association

(56) Entgegenhaltungen:
- JP-A- 2012 049 518
- US-A1- 2011 006 670
- GAO-QIANG LI ET AL: "Double-coupling of dibromo arenes with aryltriolborates for synthesis of diaryl-substituted planar frameworks", TETRAHEDRON, ELSEVIER SIENCE PUBLISHERS, AMSTERDAM, NL, vol. 67, no. 36, 24 June 2011 (2011-06-24), pages 6804 - 6811, XP028264271, ISSN: 0040-4020, [retrieved on 20110706], DOI: 10.1016/J.TET.2011.06.083

## Beschreibung

Die vorliegende Anmeldung betrifft eine elektronische Vorrichtung, welche mindestens eine Verbindung der Formel (I-f), (I-k) oder (I-l) in einer organischen Schicht enthält.

Unter elektronischen Vorrichtungen im Sinne dieser Anmeldung werden insbesondere sogenannte organische elektronische Vorrichtungen verstanden (organic electronic devices), welche organische Halbleitermaterialien als Funktionsmaterialien enthalten. Nochmals insbesondere werden darunter organische Elektrolumineszenzvorrichtungen (OLEDs) und andere elektronische Vorrichtungen verstanden, welche weiter unten aufgeführt sind.

Der Aufbau von OLEDs, in denen organische Halbleiter als funktionelle Materialien eingesetzt werden, ist beispielsweise in US 4539507, US 5151629, EP 0676461 und WO 98/27136 beschrieben. Allgemein werden unter der Bezeichnung OLED elektronische Vorrichtungen verstanden, welche organisches Material enthalten und unter Anlegen einer elektrischen Spannung Licht emittieren.

Bei elektronischen Vorrichtungen, insbesondere OLEDs, besteht großes Interesse an der Verbesserung der Leistungsdaten, insbesondere Lebensdauer und Effizienz. Eine wichtige Rolle spielen dabei Schichten mit lochtransportierender Funktion in der elektronischen Vorrichtung, wie beispielsweise lochinjizierende Schichten, Lochtransportschichten, Elektronenblockierschichten und emittierende Schichten.

Hierfür werden kontinuierlich neue Materialien mit lochtransportierenden Eigenschaften gesucht. Sie können in den genannten Schichten als Reinmaterialien, als Hauptkomponenten oder als Mindermengenkomponenten in Kombination mit weiteren Materialien eingesetzt werden. In lochinjizierenden Schichten, Lochtransportschichten und Elektronenblockierschichten werden die Materialien mit lochtransportierenden Eigenschaften typischerweise als Reinstoffe eingesetzt. Sie können jedoch in solchen Schichten auch in Mischung mit einem eindotierten weiteren Material eingesetzt werden. In emittierenden Schichten, insbesondere in phosphoreszierenden emittierenden Schichten, werden die Materialien mit lochtransportierenden Eigenschaften bevorzugt als Hauptkomponente der Schicht in Kombination mit weiteren Materialien, beispielsweise Emittermaterialien eingesetzt. In diesem Fall werden sie als Hostmaterialien der emittierenden Schicht bezeichnet.

Es ist im Stand der Technik bekannt, Triarylamine als Materialien mit lochtransportierenden Eigenschaften in den oben genannten Schichten einzusetzen. Diese können Mono-Triarylamine darstellen, wie beispielsweise in JP 1995/053955, WO 2006/123667 und JP 2010/222268 beschrieben, oder Bis- oder höherwertige Amine darstellen, wie beispielsweise in US 7504163 oder US 2005/0184657 beschrieben. Bekannte Beispiele für solche Triarylamin-Verbindungen sind unter anderem Tris-p-biphenyl-amin, N,N'-Di-1-Naphthyl-N,N'-diphenyl-1,1'-biphenyl-4,4'-diamin (NPB) und 4,4',4"-Tris-(3-methylphenylphenylamino)-triphenylamin (MTDATA).

Weiterhin bekannt ist die Verwendung von Triarylamin-Verbindungen mit relativ komplexer, asymmetrischer Struktur als lochtransportierende Verbindungen in OLEDs (WO 2011/021520). Diese Verbindungen weisen eine Triarylaminogruppe auf, die an eine Dibenzofuran- oder ähnliche Arylgruppe gebunden ist. Es ist dabei in jedem Fall nur eine einzige Triarylaminogruppe in der Verbindung vorhanden.

Weiterhin im Stand der Technik bekannt sind Verbindungen, in denen zwei Carbazolgruppen einander gegenüberliegend an eine Dibenzofuran-Gruppe gebunden sind, beispielsweise aus WO 2011/004639. Solche Verbindungen sind jedoch weniger gut als Lochtransportmaterialien geeignet, aufgrund der Lage ihres HOMOs.

Des Weiteren sind OLEDs mit Dibenzofuran-Verbindungen, an die Carbazolgruppen gebunden sind, bekannt, z.B. aus JP2012049518.

Es besteht daher trotz dieser Fortschritte weiterhin Bedarf an Verbindungen mit lochtransportierenden Eigenschaften zur Verwendung in elektronischen Vorrichtungen. Insbesondere besteht Bedarf an Verbindungen, welche ausgezeichnete Lochmobilität aufweisen sowie ein geeignetes Triplettniveau, um in phosphoreszierenden OLEDs verwendet zu werden. Weiterhin sollten die Verbindungen eine hohe Glasübergangstemperatur besitzen. Mit Verbindungen mit den oben genannten Eigenschaften können OLEDs erhalten werden, die gute Leistungsdaten, inbesondere hohe Effizienz und lange Lebensdauer, aufweisen.

Überraschend wurde nun gefunden, dass Verbindungen, in denen zwei Triarylaminogruppen bzw. eine Triarylaminogruppe und eine Carbazolgruppe "face-to-face", d.h. einander gegenüberliegend an eine Dibenzofuran- oder ähnliche Arylgruppe gebunden sind, hervorragende lochtransportierende Eigenschaften aufweisen. Weiterhin weisen sie eine hervorragende Lochmobilität und ein geeignetes Triplettniveau auf, um in phosphoreszierenden OLEDs eingesetzt zu werden. Nochmals weiterhin weisen sie eine hohe Glasübergangstemperatur auf, was sie besonders geeignet zur Verwendung in dünnen amorphen organischen Schichten in OLEDs macht.

Einzelne Verbindungen mit derartiger Struktur sind im Stand der Technik bekannt (G.-Q. Li et al., Tetrahedron, 2011, 67, 6804-6811), jedoch ist die technische Lehre dort auf die Synthese der Verbindungen beschränkt. Eine Anregung, die Verbindungen als funktionelle Materialien in elektronischen Vorrichtungen einzusetzen, findet sich dort nicht.

Gegenstand der Erfindung ist somit eine elektronische Vorrichtung gemäß Anspruch 1.

Unter einer kondensierten Arylgruppe wird dabei eine Arylgruppe verstanden, welche zwei oder mehr aromatische Ringe enthält, die miteinander kondensiert sind, d. h. eine oder mehr aromatische Bindungen miteinander teilen. Eine entsprechende Definition gilt für Heteroarylgruppen. Beispiele für kondensierte Arylgruppen ungeachtet der Zahl ihrer Ringatome sind Naphthyl, Anthracenyl, Pyrenyl, Phenanthrenyl und Perylenyl. Beispiele für kondensierte Heteroarylgruppen sind Chinolinyl, Indolyl, Carbazolyl, und Acridinyl.

Es folgen allgemeine Definitionen für chemische Gruppen im Rahmen der vorliegenden Anmeldung:
Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Unter einer Aryloxygruppe gemäß der Definition der vorliegenden Erfindung wird eine Arylgruppe, wie oben definiert, verstanden, welche über ein Sauerstoffatom gebunden ist. Eine analoge Definition gilt für Heteroaryloxygruppen.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, Si-, N- oder O-Atom, ein sp²-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Bevorzugt ist die Gruppe E bei jedem Auftreten gleich oder verschieden gewählt aus einer Einfachbindung, C(R¹)₂, C=O, O, S und NR¹. Besonders bevorzugt ist sie gewählt aus einer Einfachbindung, O und S.

Es ist bevorzugt, dass nicht mehr als zwei benachbarte Gruppen Z gleich N sind. Weiterhin bevorzugt sind nicht mehr als zwei Gruppen Z in einem aromatischen Ring gleich N. Besonders bevorzugt ist Z allgemein gleich CR¹.

Die Gruppe W ist bevorzugt bei jedem Auftreten gleich oder verschieden gewählt aus O, S und NR¹. Bevorzugt ist sie gewählt aus O und S.

Die Gruppe Ar¹ ist bevorzugt gleich oder verschieden gewählt aus Aryl- oder Heteroarylgruppen mit 6 bis 10 aromatischen Ringatomen, die mit einem oder mehreren Resten R¹ substituiert sein können. Besonders bevorzugt ist Ar¹ gewählt aus Phenyl, Naphthyl, Pyridyl, Pyrimidyl, Pyridazyl, Pyrazinyl und Triazinyl, die jeweils mit einem oder mehreren Resten R¹ substituiert sein können.

Die Gruppe Ar² ist bevorzugt gleich oder verschieden gewählt aus Aryl- oder Heteroarylgruppen mit 6 bis 10 aromatischen Ringatomen, die mit einem oder mehreren Resten R¹ substituiert sein können. Besonders bevorzugt ist Ar² gewählt aus Phenyl, Naphthyl, Pyridyl, Pyrimidyl, Pyridazyl, Pyrazinyl und Triazinyl, die jeweils mit einem oder mehreren Resten R¹ substituiert sein können.

Die Gruppe X ist bevorzugt bei jedem Auftreten gleich oder verschieden gewählt aus C(R¹)₂, C=O, O, S und NR¹.

R¹ ist bevorzugt bei jedem Auftreten gleich oder verschieden H, D, F, CN, Si(R²)₃, N(R²)₂, eine geradkettige Alkyl- oder Alkoxygruppe mit 1 bis 20 C-Atomen oder eine verzweigte oder cyclische Alkyl- oder Alkoxygruppe mit 3 bis 20 C-Atomen, wobei die oben genannten Gruppen jeweils mit einem oder mehreren Resten R² substituiert sein können und wobei in den oben genannten Gruppen eine oder mehrere CH₂-Gruppen durch -C=C-, - R²C=CR²-, Si(R²)₂, C=O, C=NR², -NR²-, -O-, -S-, -C(=O)O- oder - C(=O)NR²- ersetzt sein können, oder ein aromatisches oder heteroaromatisches Ringsystem mit 5 bis 20 aromatischen Ringatomen, das mit einem oder mehreren Resten R² substituiert sein kann, wobei zwei oder mehr Reste R¹ miteinander verknüpft sein können und einen Ring bilden können.

Der Index n ist bevorzugt bei jedem Auftreten gleich oder verschieden 0 oder 1, besonders bevorzugt 0.

Der Index i ist bevorzugt gleich 0.

Weiterhin ist es für Verbindungen der Formeln (I-f), (I-k) oder (I-I) bevorzugt, dass an den mit * gekennzeichneten Positionen eine Gruppe gewählt aus Gruppen der Formel (A-1) und (A-2) gebunden ist.

Weiterhin ist es für Verbindungen der Formeln (I-f), (I-k) oder (I-I) bevorzugt, dass sie keine kondensierten Arylgruppen mit mehr als 10 aromatischen Ringatomen enthalten.

Weiterhin ist es für Verbindungen der Formeln (I-f), (I-k) oder (I-l) bevorzugt, dass sie zusätzlich zu den Gruppen (A-1), (A-2), (C-1) und (C-2) keine weiteren Arylaminogruppen aufweisen. Besonders bevorzugt weisen sie keine weiteren Aminogruppen auf.

Weiterhin ist es für Verbindungen der Formeln (I-f), (I-k) oder (I-I) bevorzugt, dass sie zusätzlich zu den Gruppen (A-1), (A-2), (C-1) und (C-2) keine weiteren Carbazolgruppen aufweisen.

Weiterhin ist es für Verbindungen der Formeln (I-f), (I-k) oder (I-I) bevorzugt, dass sie keine elektronenarmen Heteroarylgruppen aufweisen. Unter elektronenarmen Heteroarylgruppen werden im Rahmen der vorliegenden Erfindung insbesondere heteroaromatische Sechsringe mit einem oder mehreren Stickstoffatomen und heteroaromatische Fünfringe mit zwei oder mehr Heteroatomen, insbesondere Heteroatomen ausgewählt aus N, O und S, verstanden.

Die folgenden Verbindungen werden beispielhaft genannt, wobei nur die Verbindungen der Formeln (I-f), (I-k) und (I-I) unter die Erfindung fallen. 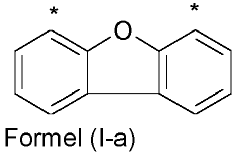 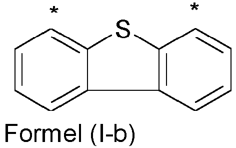 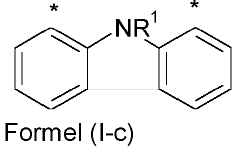 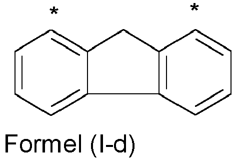 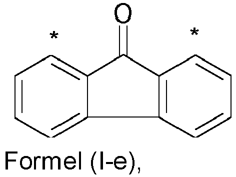 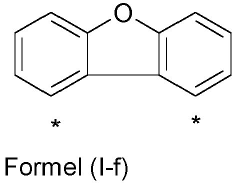 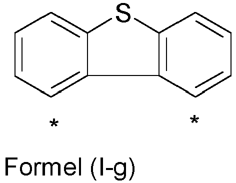 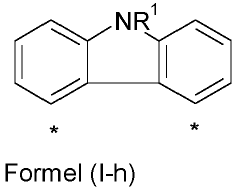 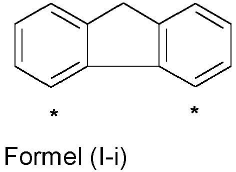 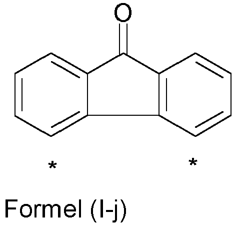 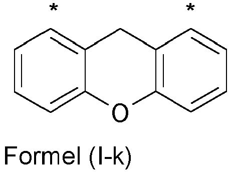 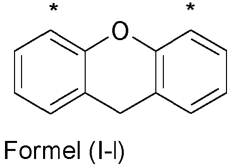 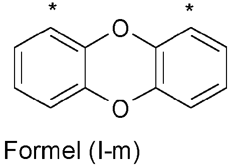 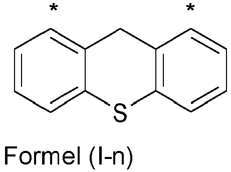 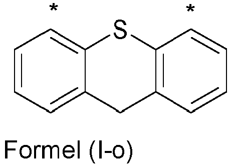 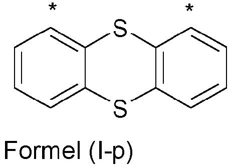 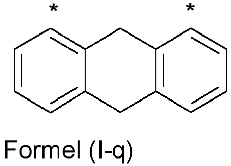 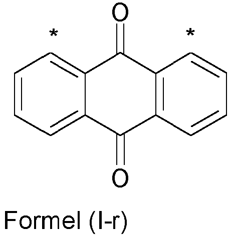 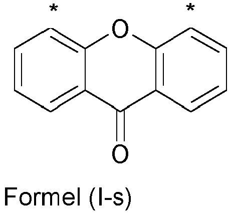 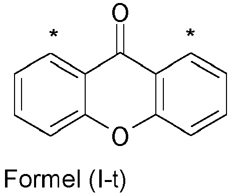 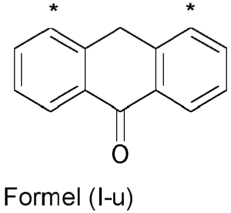 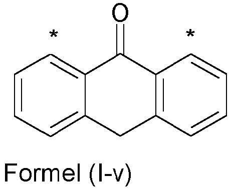 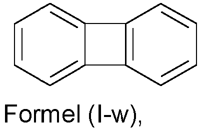
wobei die Verbindungen an allen freien Positionen mit Resten R¹ substituiert sein können, und
wobei an die mit * gekennzeichneten Positionen eine Gruppe gewählt aus den Gruppen der Formeln (A-1), (A-2), (C-1) und (C-2) gebunden ist und
wobei an mindestens eine der mit * gekennzeichneten Positionen eine Gruppe gewählt aus Gruppen der Formeln (A-1) und (A-2) gebunden ist.

Die in der Anmeldung angegebenen bevorzugten Ausführungsformen von Gruppen sind ebenfalls bevorzugt.

Eine Gruppe der Formel (A-1) entspricht bevorzugt einer Gruppe der folgenden Formeln (A-1-1) bis (A-1-6) 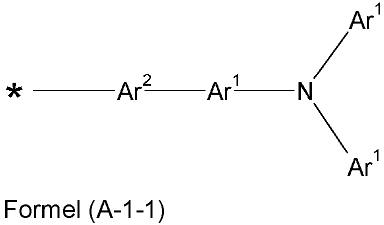 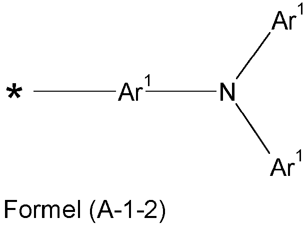 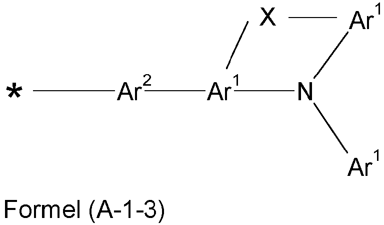 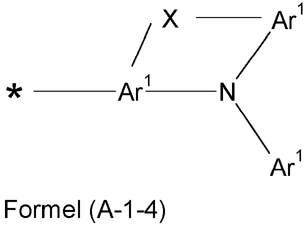 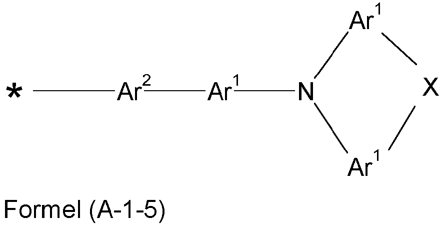 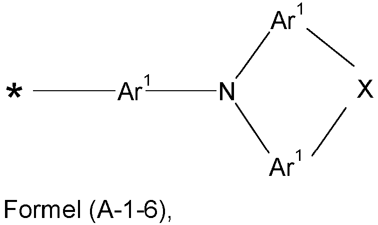 wobei die auftretenden Symbole wie oben definiert sind. Die in der Anmeldung angegebenen bevorzugten Ausführungsformen von Gruppen gelten ebenfalls als bevorzugt.

Insbesondere ist es bevorzugt, dass in den Formeln (A-1-1) bis (A-1-6) Ar¹ und Ar² gleich oder verschieden gewählt sind aus Phenyl, Naphthyl, Pyridyl, Pyrimidyl, Pyridazyl, Pyrazinyl und Triazinyl, die jeweils mit einem oder mehreren Resten R¹ substituiert sein können. Weiterhin ist es bevorzugt, dass die Gruppe X bei jedem Auftreten gleich oder verschieden gewählt ist aus C(R¹)₂, C=O, O, S und NR¹.

Besonders bevorzugt sind die Formeln (A-1-1) und (A-1-2).

Eine Gruppe der Formel (A-2) entspricht bevorzugt einer Gruppe der folgenden Formeln (A-2-1) bis (A-2-2) 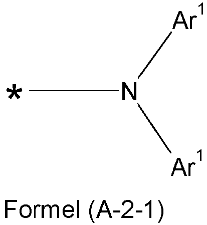 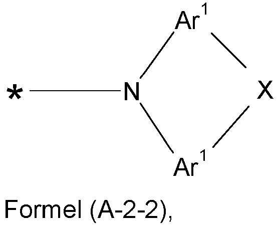 wobei die auftretenden Symbole wie oben definiert sind. Die in der Anmeldung angegebenen bevorzugten Ausführungsformen von Gruppen gelten ebenfalls als bevorzugt.

Insbesondere ist es bevorzugt, dass in den Formeln (A-2-1) bis (A-2-2) Ar¹ gleich oder verschieden gewählt ist aus Phenyl, Naphthyl, Pyridyl, Pyrimidyl, Pyridazyl, Pyrazinyl und Triazinyl, die jeweils mit einem oder mehreren Resten R¹ substituiert sein können. Weiterhin ist es bevorzugt, dass die Gruppe X bei jedem Auftreten gleich oder verschieden gewählt ist aus C(R¹)₂, C=O, O, S und NR¹.

Besonders bevorzugt ist die Formel (A-2-1).

Eine Gruppe der Formel (C-1) entspricht bevorzugt einer Gruppe der folgenden Formeln (C-1-1) bis (C-1-4) 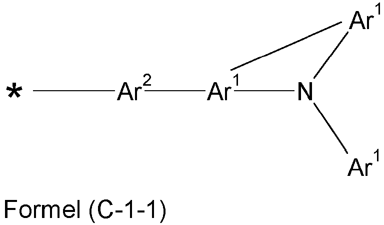 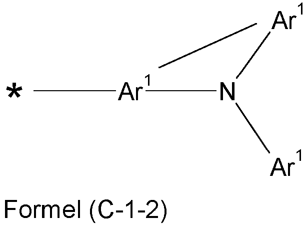 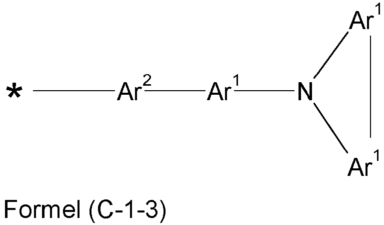 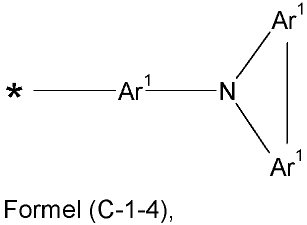 wobei die auftretenden Symbole wie oben definiert sind. Die in der Anmeldung angegebenen bevorzugten Ausführungsformen von Gruppen gelten ebenfalls als bevorzugt.

Insbesondere ist es bevorzugt, dass in den Formeln (C-1-1) bis (C-1-4) Ar¹ und Ar² gleich oder verschieden gewählt sind aus Phenyl, Naphthyl, Pyridyl, Pyrimidyl, Pyridazyl, Pyrazinyl und Triazinyl, die jeweils mit einem oder mehreren Resten R¹ substituiert sein können.

Eine Gruppe der Formel (C-2) entspricht bevorzugt einer Gruppe der folgenden Formel (C-2-1) 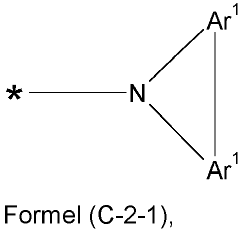 wobei Ar¹ gleich oder verschieden gewählt ist aus Phenyl, Naphthyl, Pyridyl, Pyrimidyl, Pyridazyl, Pyrazinyl und Triazinyl, die jeweils mit einem oder mehreren Resten R¹ substituiert sein können.

Folgende Strukturen werden beispielhaft genannt, wobei nur die Strukturen mit einem Grundkörper der Formeln (I-f), (I-k) oder (I-l) unter die Erfindung fallen (Schema unten in Kombination mit Tabelle):

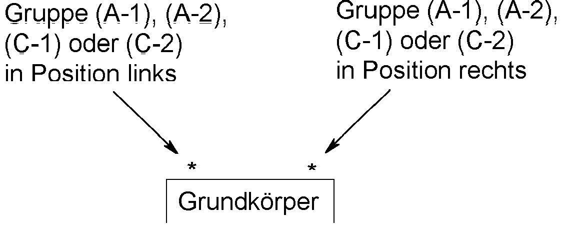

| Struktur | Grundkörper der Formel | Gruppe in Position * links | Gruppe in Position * rechts |
|---|---|---|---|
| (I-a-1) | (I-a) | (A-1) | (A-1) |
| (I-a-2) | " | (A-1) | (A-2) |
| (I-a-3) | " | (A-1) | (C-1) |
| (I-a-4) | " | (A-1) | (C-2) |
| (I-a-5) | " | (A-2) | (A-2) |
| (I-a-6) | " | (A-2) | (C-1) |
| (I-a-7) | " | (A-2) | (C-2) |
| (I-b-1) | (I-b) | (A-1) | (A-1) |
| (I-b-2) | " | (A-1) | (A-2) |
| (I-b-3) | " | (A-1) | (C-1) |
| (I-b-4) | " | (A-1) | (C-2) |
| (I-b-5) | " | (A-2) | (A-2) |
| (I-b-6) | " | (A-2) | (C-1) |
| (I-b-7) | " | (A-2) | (C-2) |
| (I-c-1) | (I-c) | (A-1) | (A-1) |
| (I-c-2) | " | (A-1) | (A-2) |
| (I-c-3) | " | (A-1) | (C-1) |
| (I-c-4) | " | (A-1) | (C-2) |
| (I-c-5) | " | (A-2) | (A-2) |
| (I-c-6) | " | (A-2) | (C-1) |
| (I-c-7) | " | (A-2) | (C-2) |
| (I-d-1) | (I-d) | (A-1) | (A-1) |
| (I-d-2) | " | (A-1) | (A-2) |
| (I-d-3) | " | (A-1) | (C-1) |
| (I-d-4) | " | (A-1) | (C-2) |
| (I-d-5) | " | (A-2) | (A-2) |
| (I-d-6) | " | (A-2) | (C-1) |
| (I-d-7) | " | (A-2) | (C-2) |
| (I-e-1) | (I-e) | (A-1) | (A-1) |
| (I-e-2) | " | (A-1) | (A-2) |
| (I-e-3) | " | (A-1) | (C-1) |
| (I-e-4) | " | (A-1) | (C-2) |
| (I-e-5) | " | (A-2) | (A-2) |
| (I-e-6) | " | (A-2) | (C-1) |
| (I-e-7) | " | (A-2) | (C-2) |
| (I-f-1) | (I-f) | (A-1) | (A-1) |
| (I-f -2) | " | (A-1) | (A-2) |
| (I-f-3) | " | (A-1) | (C-1) |
| (I-f-4) | " | (A-1) | (C-2) |
| (I-f-5) | " | (A-2) | (A-2) |
| (I-f-6) | " | (A-2) | (C-1) |
| (I-f-7) | " | (A-2) | (C-2) |
| (I-g-1) | (I-g) | (A-1) | (A-1) |
| (I-g-2) | " | (A-1) | (A-2) |
| (I-g-3) | " | (A-1) | (C-1) |
| (I-g-4) | " | (A-1) | (C-2) |
| (I-g-5) | " | (A-2) | (A-2) |
| (I-g-6) | " | (A-2) | (C-1) |
| (I-g-7) | " | (A-2) | (C-2) |
| (I-h-1) | (I-h) | (A-1) | (A-1) |
| (I-h-2) | " | (A-1) | (A-2) |
| (I-h-3) | " | (A-1) | (C-1) |
| (I-h-4) | " | (A-1) | (C-2) |
| (I-h-5) | " | (A-2) | (A-2) |
| (I-h-6) | " | (A-2) | (C-1) |
| (I-h-7) | " | (A-2) | (C-2) |
| (I-i-1) | (I-i) | (A-1) | (A-1) |
| (I-i-2) | " | (A-1) | (A-2) |
| (I-i-3) | " | (A-1) | (C-1) |
| (I-i-4) | " | (A-1) | (C-2) |
| (I-i-5) | " | (A-2) | (A-2) |
| (I-i-6) | " | (A-2) | (C-1) |
| (I-i-7) | " | (A-2) | (C-2) |
| (I-j-1) | (I-j) | (A-1) | (A-1) |
| (I-i-2) | " | (A-1) | (A-2) |
| (I-i-3) | " | (A-1) | (C-1) |
| (I-i-4) | " | (A-1) | (C-2) |
| (I-i-5) | " | (A-2) | (A-2) |
| (I-i-6) | " | (A-2) | (C-1) |
| (I-j-7) | " | (A-2) | (C-2) |
| (I-k-1) | (I-k) | (A-1) | (A-1) |
| (I-k-2) | " | (A-1) | (A-2) |
| (I-k-3) | " | (A-1) | (C-1) |
| (I-k-4) | " | (A-1) | (C-2) |
| (I-k-5) | " | (A-2) | (A-2) |
| (I-k-6) | " | (A-2) | (C-1) |
| (I-k-7) | " | (A-2) | (C-2) |
| (I-l-1) | (I-I) | (A-1) | (A-1) |
| (I-l-2) | " | (A-1) | (A-2) |
| (I-l-3) | " | (A-1) | (C-1) |
| (I-l-4) | " | (A-1) | (C-2) |
| (I-l-5) | " | (A-2) | (A-2) |
| (I-l-6) | " | (A-2) | (C-1) |
| (I-l-7) | " | (A-2) | (C-2) |
| (I-m-1) | (I-m) | (A-1) | (A-1) |
| (I-m-2) | " | (A-1) | (A-2) |
| (I-m-3) | " | (A-1) | (C-1) |
| (I-m-4) | " | (A-1) | (C-2) |
| (I-m-5) | " | (A-2) | (A-2) |
| (I-m-6) | " | (A-2) | (C-1) |
| (I-m-7) | " | (A-2) | (C-2) |
| (I-n-1) | (I-n) | (A-1) | (A-1) |
| (I-n-2) | " | (A-1) | (A-2) |
| (I-n-3) | " | (A-1) | (C-1) |
| (I-n-4) | " | (A-1) | (C-2) |
| (I-n-5) | " | (A-2) | (A-2) |
| (I-n-6) | " | (A-2) | (C-1) |
| (I-n-7) | " | (A-2) | (C-2) |
| (I-o-1) | (I-0) | (A-1) | (A-1) |
| (I-o-2) | " | (A-1) | (A-2) |
| (I-o-3) | " | (A-1) | (C-1) |
| (I-o-4) | " | (A-1) | (C-2) |
| (I-o-5) | " | (A-2) | (A-2) |
| (I-o-6) | " | (A-2) | (C-1) |
| (I-o-7) | " | (A-2) | (C-2) |
| (I-p-1) | (I-p) | (A-1) | (A-1) |
| (I-p-2) | " | (A-1) | (A-2) |
| (I-p-3) | " | (A-1) | (C-1) |
| (I-p-4) | " | (A-1) | (C-2) |
| (I-p-5) | " | (A-2) | (A-2) |
| (I-p-6) | " | (A-2) | (C-1) |
| (I-p-7) | " | (A-2) | (C-2) |
| (I-q-1) | (I-q) | (A-1) | (A-1) |
| (I-q-2) | " | (A-1) | (A-2) |
| (I-q-3) | " | (A-1) | (C-1) |
| (I-q-4) | " | (A-1) | (C-2) |
| (I-q-5) | " | (A-2) | (A-2) |
| (I-q-6) | " | (A-2) | (C-1) |
| (I-q-7) | " | (A-2) | (C-2) |
| (I-r-1) | (I-r) | (A-1) | (A-1) |
| (I-r-2) | " | (A-1) | (A-2) |
| (I-r-3) | " | (A-1) | (C-1) |
| (I-r-4) | " | (A-1) | (C-2) |
| (I-r-5) | " | (A-2) | (A-2) |
| (I-r-6) | " | (A-2) | (C-1) |
| (I-r-7) | " | (A-2) | (C-2) |
| (I-s-1) | (I-s) | (A-1) | (A-1) |
| (I-s-2) | " | (A-1) | (A-2) |
| (I-s-3) | " | (A-1) | (C-1) |
| (I-s-4) | " | (A-1) | (C-2) |
| (I-s-5) | " | (A-2) | (A-2) |
| (I-s-6) | " | (A-2) | (C-1) |
| (I-s-7) | " | (A-2) | (C-2) |
| (I-t-1) | (I-t) | (A-1) | (A-1) |
| (I-t-2) | " | (A-1) | (A-2) |
| (I-t-3) | " | (A-1) | (C-1) |
| (I-t-4) | " | (A-1) | (C-2) |
| (I-t-5) | " | (A-2) | (A-2) |
| (I-t-6) | " | (A-2) | (C-1) |
| (I-t-7) | " | (A-2) | (C-2) |
| (I-u-1) | (I-u) | (A-1) | (A-1) |
| (I-u-2) | " | (A-1) | (A-2) |
| (I-u-3) | " | (A-1) | (C-1) |
| (I-u-4) | " | (A-1) | (C-2) |
| (I-u-5) | " | (A-2) | (A-2) |
| (I-u-6) | " | (A-2) | (C-1) |
| (I-u-7) | " | (A-2) | (C-2) |
| (I-v-1) | (I-v) | (A-1) | (A-1) |
| (I-v-2) | " | (A-1) | (A-2) |
| (I-v-3) | " | (A-1) | (C-1) |
| (I-v-4) | " | (A-1) | (C-2) |
| (I-v-5) | " | (A-2) | (A-2) |
| (I-v-6) | " | (A-2) | (C-1) |
| (I-v-7) | " | (A-2) | (C-2) |
| (I-w-1) | (I-w) | (A-1) | (A-1) |
| (I-w-2) | " | (A-1) | (A-2) |
| (I-w-3) | " | (A-1) | (C-1) |
| (I-w-4) | " | (A-1) | (C-2) |
| (I-w-5) | " | (A-2) | (A-2) |
| (I-w-6) | " | (A-2) | (C-1) |
| (I-w-7) | " | (A-2) | (C-2) |
| (II-a-1) | (II-a) | (A-1) | (A-1) |
| (II-a-2) | " | (A-1) | (A-2) |
| (II-a-3) | " | (A-1) | (C-1) |
| (II-a-4) | " | (A-1) | (C-2) |
| (II-a-5) | " | (A-2) | (A-2) |
| (II-a-6) | " | (A-2) | (C-1) |
| (II-a-7) | " | (A-2) | (C-2) |
| (II-b-1) | (II-b) | (A-1) | (A-1) |
| (II-b-2) | " | (A-1) | (A-2) |
| (II-b-3) | " | (A-1) | (C-1) |
| (II-b-4) | " | (A-1) | (C-2) |
| (II-b-5) | " | (A-2) | (A-2) |
| (II-b-6) | " | (A-2) | (C-1) |
| (II-b-7) | " | (A-2) | (C-2) |

Es gelten für die Strukturen aus der obenstehenden Tabelle die in der Anmeldung angegebenen bevorzugten Ausführungsformen, insbesondere die bevorzugten Ausführungsformen der Gruppen (A-1), (A-2), (C-1) und (C-2).

Folgende Verbindungen sind Beispiele für Verbindungen, wobei alle Verbindungen, mit Ausnahme der Verbindungen 37-45, nicht unter Anspruch 1 fallen.

| | |
|---|---|
| 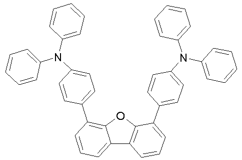 | 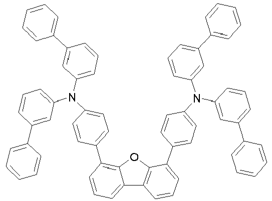 |
| 1 | 2 |
| 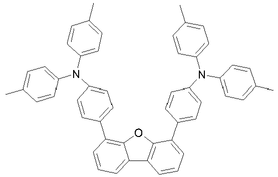 | 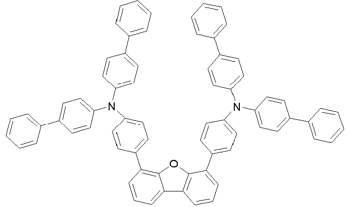 |
| 3 | 4 |
| 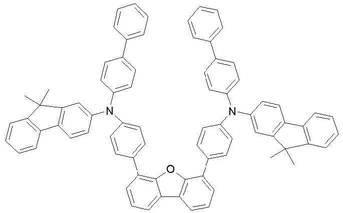 | 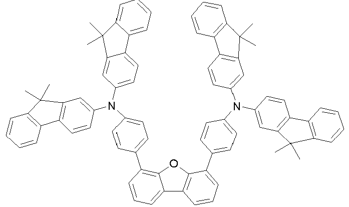 |
| 5 | 6 |
| 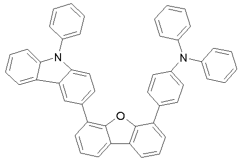 | 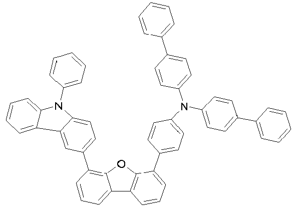 |
| 7 | 8 |
| 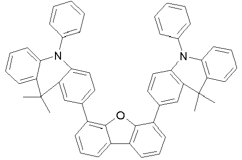 | 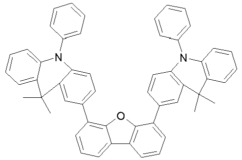 |
| 9 | 10 |
| 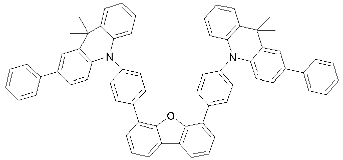 | 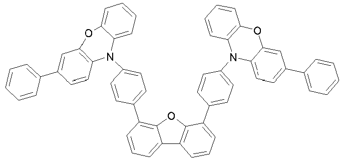 |
| 11 | 12 |
| 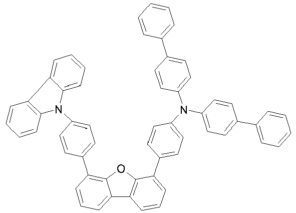 | 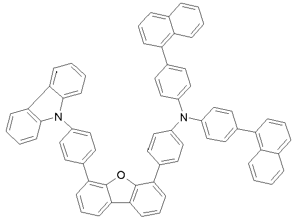 |
| 13 | 14 |
| 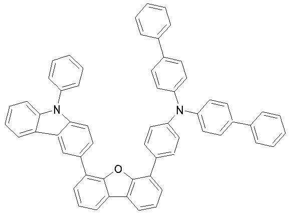 | 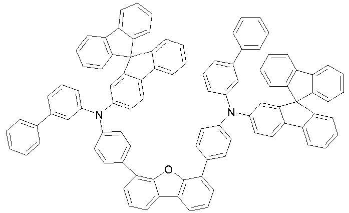 |
| 15 | 16 |
| 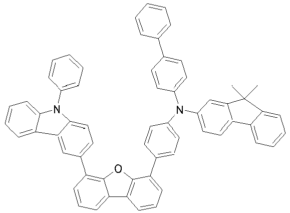 | 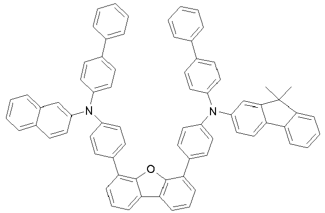 |
| 17 | 18 |
| 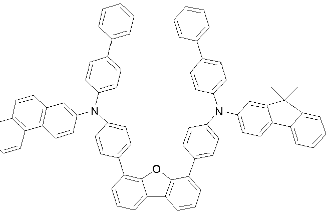 | 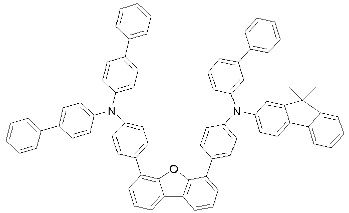 |
| 19 | 20 |
| 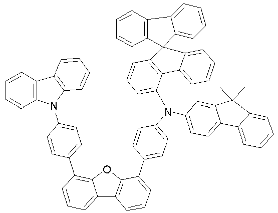 | 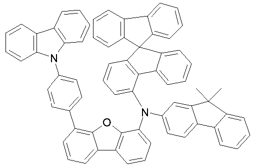 |
| 21 | 22 |
| 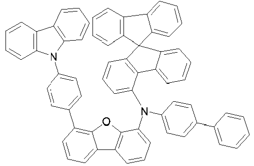 | 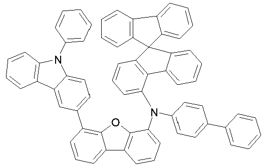 |
| 23 | 24 |
| 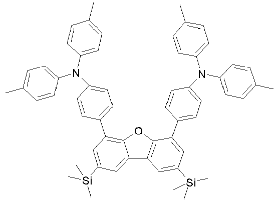 | 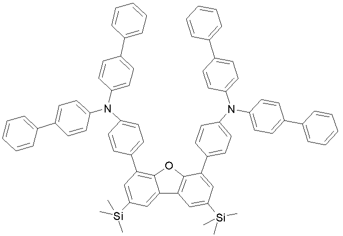 |
| 25 | 26 |
| 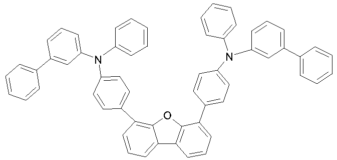 | 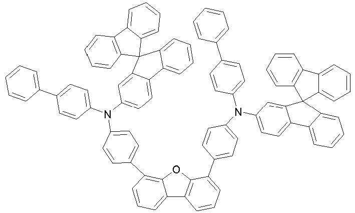 |
| 27 | 28 |
| 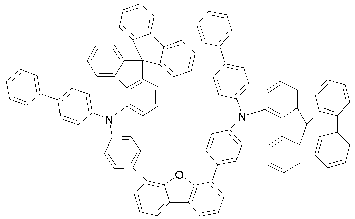 | 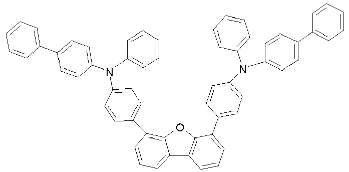 |
| 29 | 30 |
| 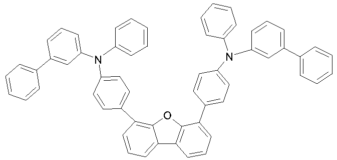 | 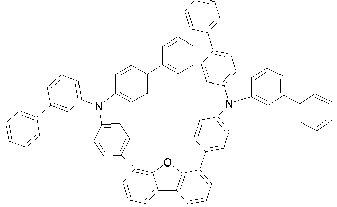 |
| 31 | 32 |
| 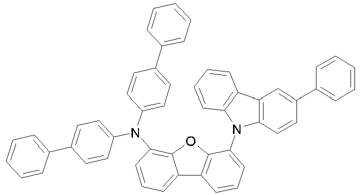 | 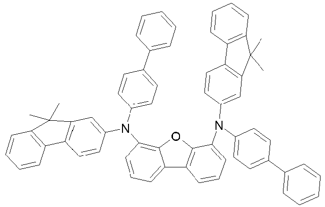 |
| 33 | 34 |
| 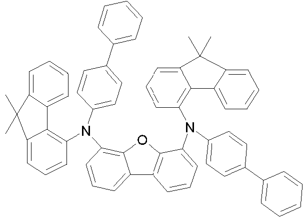 | 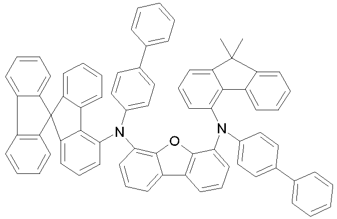 |
| 35 | 36 |
| 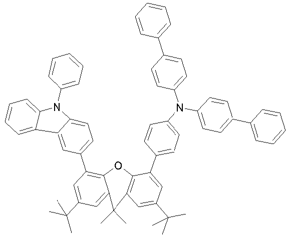 | 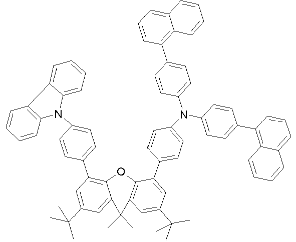 |
| 37 | 38 |
| 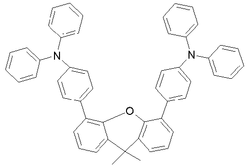 | 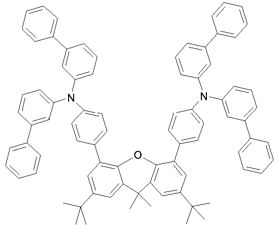 |
| 39 | 40 |
| 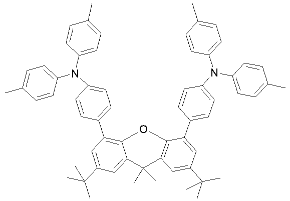 | 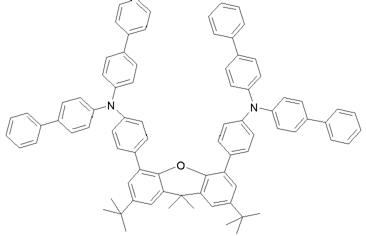 |
| 41 | 42 |
| 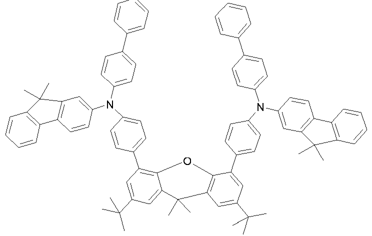 | 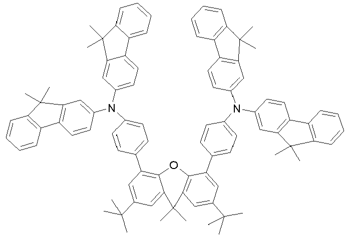 |
| 43 | 44 |
| 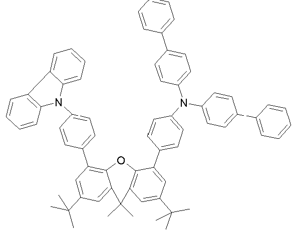 | 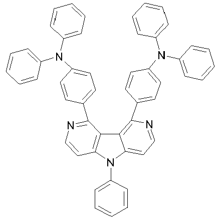 |
| 45 | 46 |
| 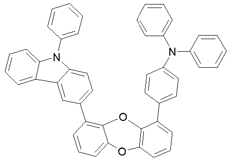 | 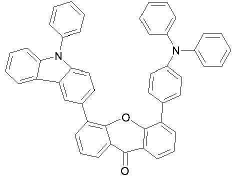 |
| 47 | 48 |
| 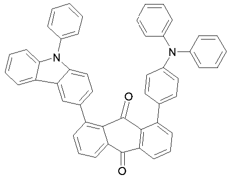 | 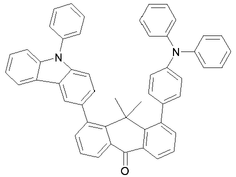 |
| 49 | 50 |
| 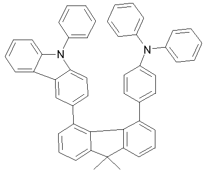 | 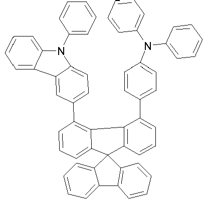 |
| 51 | 52 |
| 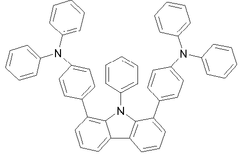 | 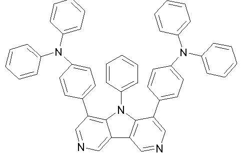 |
| 53 | 54 |
| 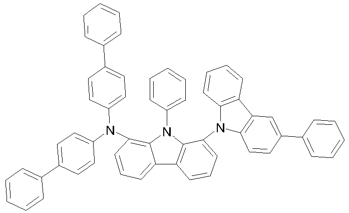 | 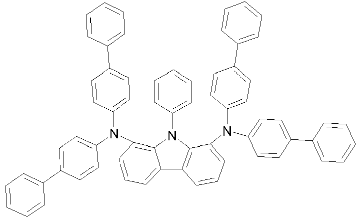 |
| 55 | 56 |
| 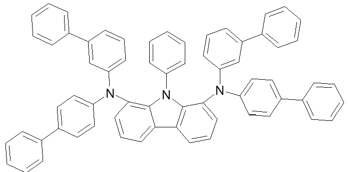 | 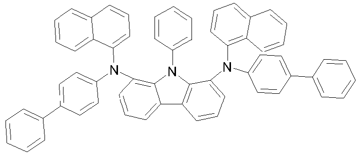 |
| 57 | 58 |
| 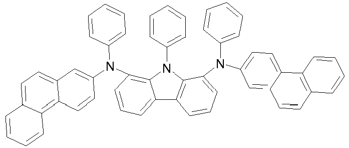 | 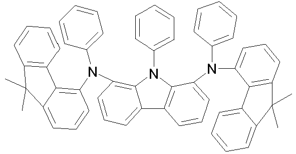 |
| 59 | 60 |
| 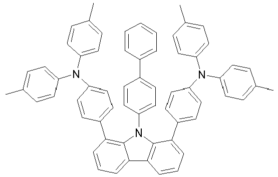 | 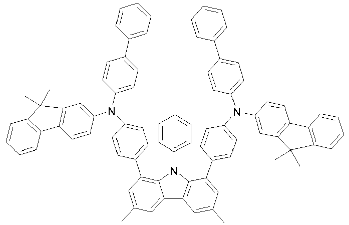 |
| 61 | 62 |
| 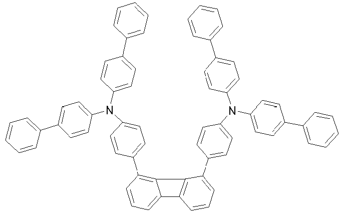 | 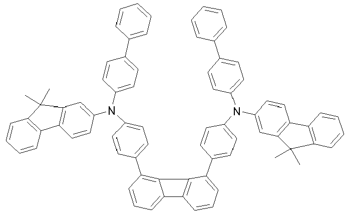 |
| 63 | 64 |
| 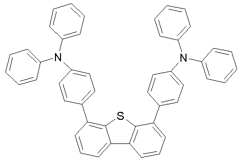 | 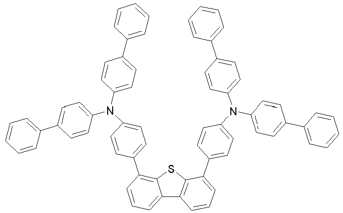 |
| 65 | 66 |
| 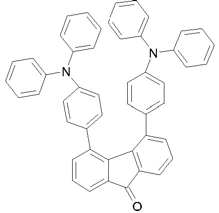 | 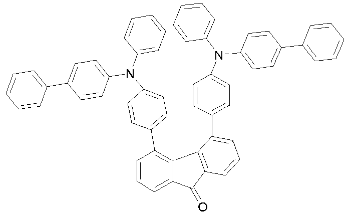 |
| 67 | 68 |
| 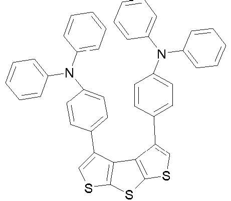 | 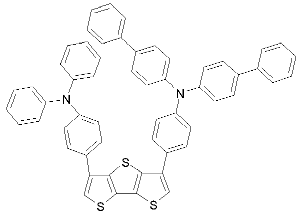 |
| 69 | 70 |
| 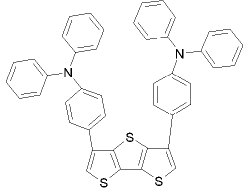 | |
| 71 | |

Die Verbindungen können gemäß Schemata 1-4 hergestellt werden.

Die entsprechenden Monoboronsäuren können durch Suzuki-Kupplung (Schema 1) oder Buchwald-Kupplung (Schema 2) und anschließende Silylierung (a oder b) hergestellt werden. Reaktion von diesen Monoboronsäuren via Suzuki-Kupplung mit entsprechenden Arylbromiden oder Arylchloriden führt zu entsprechenden Zielverbindungen. 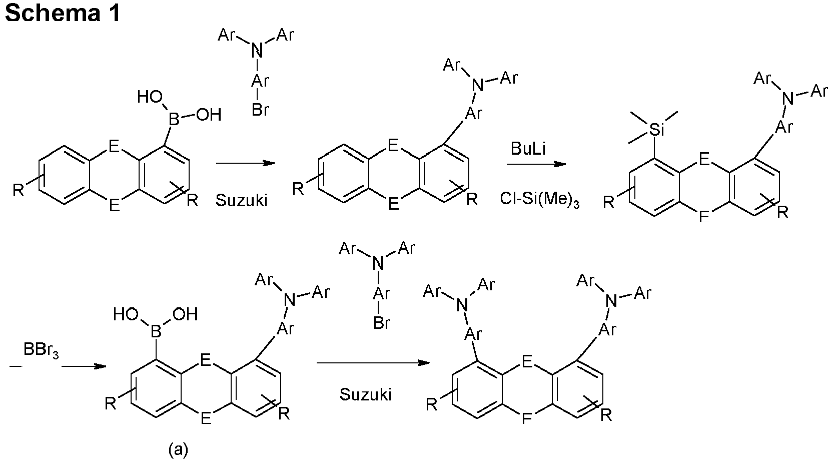
R = organischer Rest
Ar = aromatisches oder heteroaromatisches Ringsystem
E = Einfachbindung, O, S, C=O... 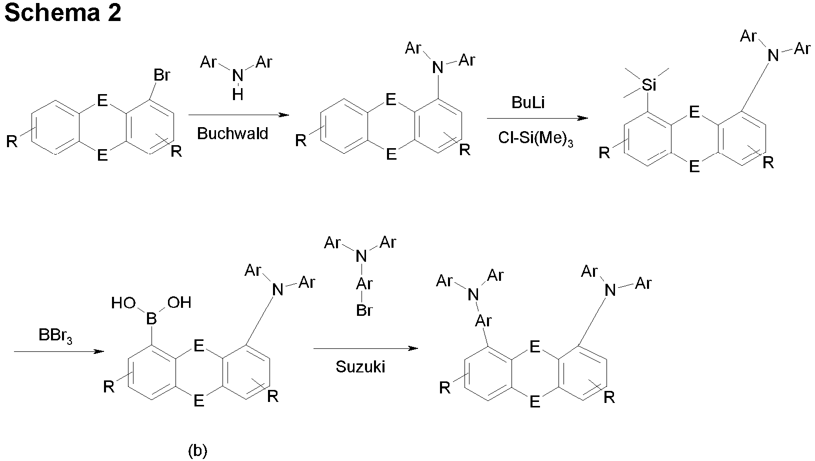
   R = organischer Rest
   Ar = aromatisches oder heteroaromatisches Ringsystem
   E = Einfachbindung, O, S, C=O...

Eine weitere Möglichkeit ist die Umsetzung eines Dihalogenids mit 1 Äq. der entsprechenden Arylboronsäure und anschließende Suzuki-Kupplung zum gewünschten Produkt (Schema 3, Alternative a), wobei die Synthese ähnliche Schritte wie in Schema 1 gezeigt umfasst.

Eine weitere Möglichkeit ist die Umsetzung eines Dihalogenids mit 2 Äq. einer entsprechenden Boronsäure (Schema 3, Alternative b) 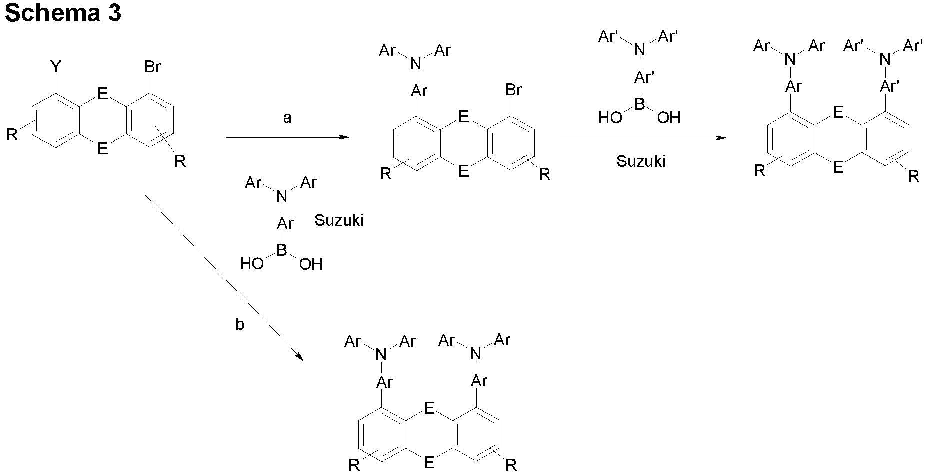
R = organischer Rest
Ar, Ar' = aromatisches oder heteroaromatisches Ringsystem
E = Einfachbindung, O, S, C=O...
Y= I,Br, ...

Ein Syntheseweg ausgehend von einer unfunktionalisierten Ausgangsverbindung wie beispielsweise einem Dibenzofuran-Derivat ist unten gezeigt (Schema 4).

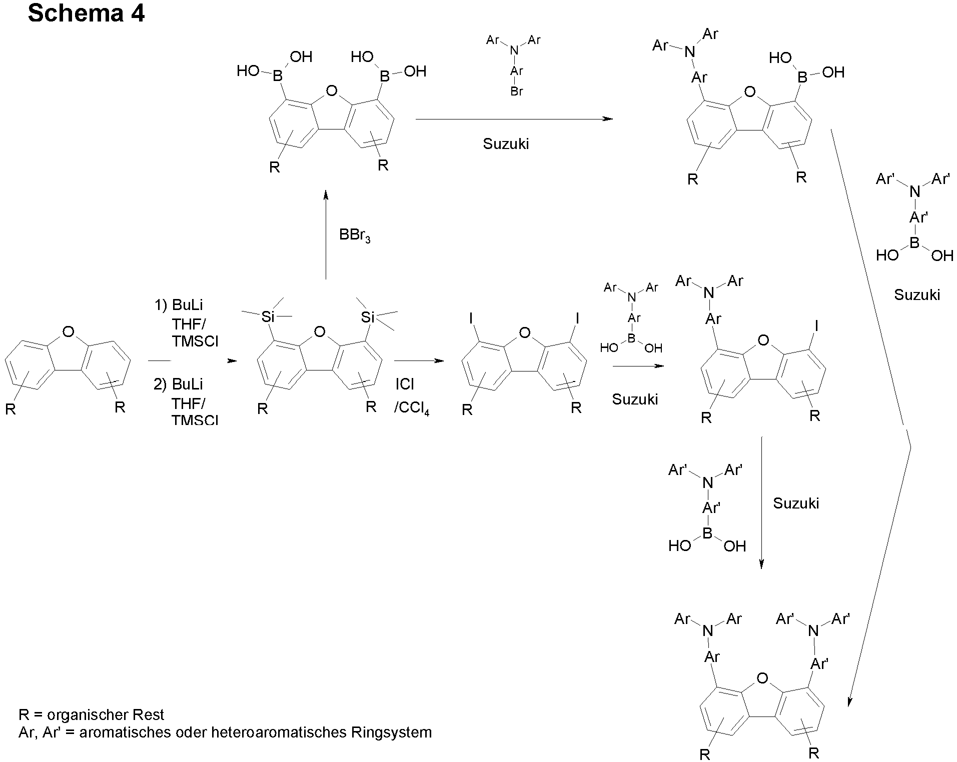

Die gezeigten Verfahren zur Synthese der Verbindungen sind exemplarisch zu verstehen. Der Fachmann kann alternative Synthesewege im Rahmen seines allgemeinen Fachwissens entwickeln.

Weiterer Gegenstand der Erfindung ist die Verwendung einer Verbindung der Formel (I-f), (I-k) oder (I-l) in einer elektronischen Vorrichtung, bevorzugt in einer lochtransportierenden und/oder in einer emittierenden Schicht.

Die erfindungsgemäße elektronische Vorrichtung ist bevorzugt eine organische Elektrolumineszenzvorrichtung (OLED).

Die organische Schicht enthaltend die Verbindung der Formel (I-f), (I-k) oder (I-l) ist bevorzugt eine Schicht mit lochtransportierender Funktion. Besonders bevorzugt ist sie eine Lochinjektionsschicht, eine Lochtransportschicht, eine Elektronenblockierschicht oder eine emittierende Schicht.

Eine Lochtransportschicht gemäß der vorliegenden Anmeldung ist eine Schicht mit lochtransportierender Funktion, welche sich zwischen Anode und emittierender Schicht befindet.

Lochinjektionsschichten und Elektronenblockierschichten werden im Sinne der vorliegenden Anmeldung als spezielle Ausführungsformen von Lochtransportschichten verstanden. Eine Lochinjektionsschicht ist dabei im Fall von mehreren Lochtransportschichten zwischen Anode und emittierender Schicht eine Lochtransportschicht, welche sich direkt an die Anode anschließt oder nur durch eine einzelne Beschichtung der Anode von ihr getrennt ist. Eine Elektronenblockierschicht ist im Fall von mehreren Lochtransportschichten zwischen Anode und emittierender Schicht diejenige Lochtransportschicht, welche sich direkt anodenseitig an die emittierende Schicht anschließt.

Wird die Verbindung gemäß Formel (I-f), (I-k) oder (I-I) als Lochtransportmaterial in einer Lochtransportschicht, einer Lochinjektionsschicht oder einer Elektronenblockierschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen eingesetzt werden. Gemäß einer bevorzugten Ausführungsform enthält die organische Schicht enthaltend die Verbindung der Formel (I-f), (I-k) oder (I-I) dann zusätzlich einen oder mehrere p-Dotanden.

Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Besonders bevorzugte Ausführungsformen von p-Dotanden sind die in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600 und WO 2012/095143 offenbarten Verbindungen.

In einer weiteren bevorzugten Ausführungsform der Erfindung ist die Verbindung gemäß Formel (I-f), (I-k) oder (I-l) als Lochtransportmaterial in einer lochtransportierenden Schicht in Kombination mit einem Hexaazatriphenylenderivat, wie in US 2007/0092755 beschrieben, in der elektronischen Vorrichtung vorhanden. Besonders bevorzugt wird das Hexaazatriphenylenderivat dabei in einer separaten Schicht eingesetzt.

Es ist weiterhin bevorzugt, dass die organische Schicht enthaltend die Verbindung der Formel (I-f), (I-k) oder (I-I) eine emittierende Schicht ist.

In einer weiteren Ausführungsform der vorliegenden Erfindung ist die Verbindung der Formel (I-f), (I-k) oder (I-I) in der elektronischen Vorrichtung als Matrixmaterial in Kombination mit einem oder mehreren Dotanden, vorzugsweise phosphoreszierenden Dotanden, vorhanden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Dotanden Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Anmeldung alle lumineszierenden Iridium-, Platin- oder Kupferkomplexe als phosphoreszierende Verbindungen angesehen. Beispiele für phosphoreszierende Dotanden sind in einem folgenden Abschnitt aufgeführt.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindungen gemäß Formel (I-f), (I-k) oder (I-I) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende Dotanden oder den bevorzugten Matrixmaterialien für fluoreszierende Dotanden, je nachdem welche Art von Dotand im mixed-Matrix-System eingesetzt wird.

Bevorzugte phosphoreszierende Dotanden zur Verwendung in Mixed-Matrix-Systemen sind die im Folgenden angebenen bevorzugten phosphoreszierenden Dotanden.

Außer Kathode, Anode und der Schicht enthaltend die Verbindung der Formel (I-f), (I-k) oder (I-I) kann die elektronische Vorrichtung noch weitere Schichten enthalten. Diese sind beispielsweise gewählt aus jeweils einer oder mehreren Lochinjektionsschichten, Lochtransportschichten, Lochblockierschichten, emittierenden Schichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer*)* und/oder organischen oder anorganischen p/n-Übergängen. Es sei aber darauf hingewiesen, dass nicht notwendigerweise jede dieser Schichten vorhanden sein muss.

Die Abfolge der Schichten der organischen Elektrolumineszenzvorrichtung ist bevorzugt die folgende:
Anode-Lochinjektionsschicht-Lochtransportschicht-emittierende Schicht-Elektronentransportschicht-Elektroneninjektionsschicht-Kathode.

Dabei soll erneut darauf hingewiesen werden, dass nicht alle der genannten Schichten vorhanden sein müssen, und/oder dass zusätzlich weitere Schichten vorhanden sein können.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei die drei Schichten blaue, grüne und orange oder rote Emission zeigen (für den prinzipiellen Aufbau siehe z. B. WO 2005/011013). Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Es folgen bevorzugte Ausführungsformen der Funktionsschichten und der Funktionsmaterialien der erfindungsgemäßen Vorrichtung:
Beispiele für phosphoreszierende Dotanden können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen.

Explizite Beispiele für phosphoreszierende Dotanden sind in der folgenden Tabelle aufgeführt.

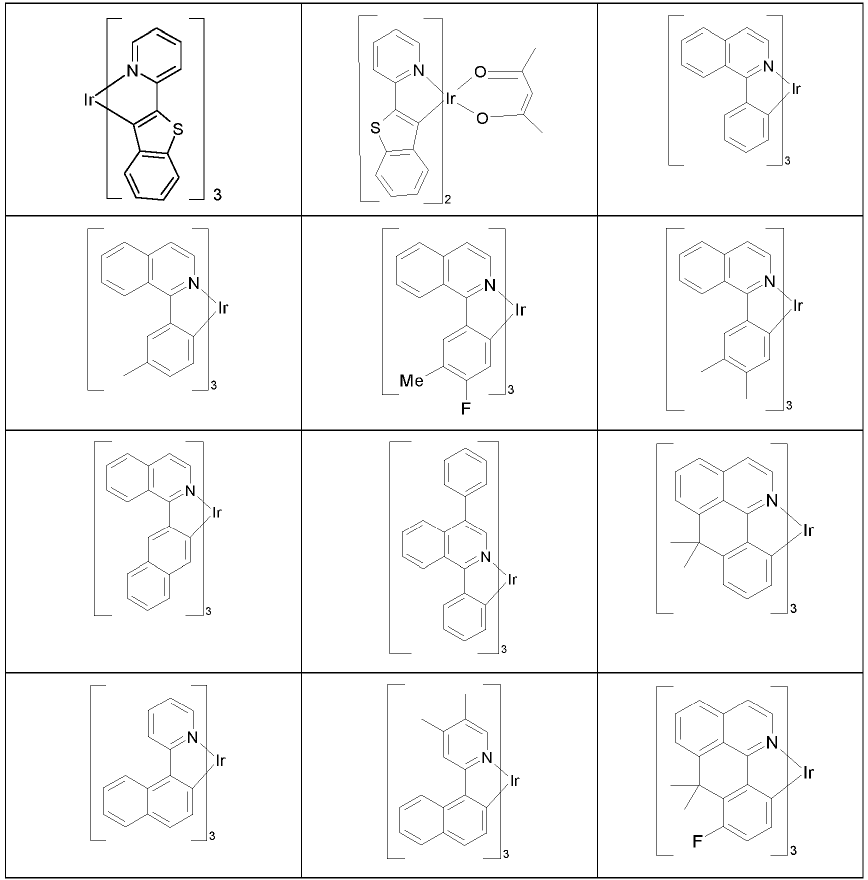

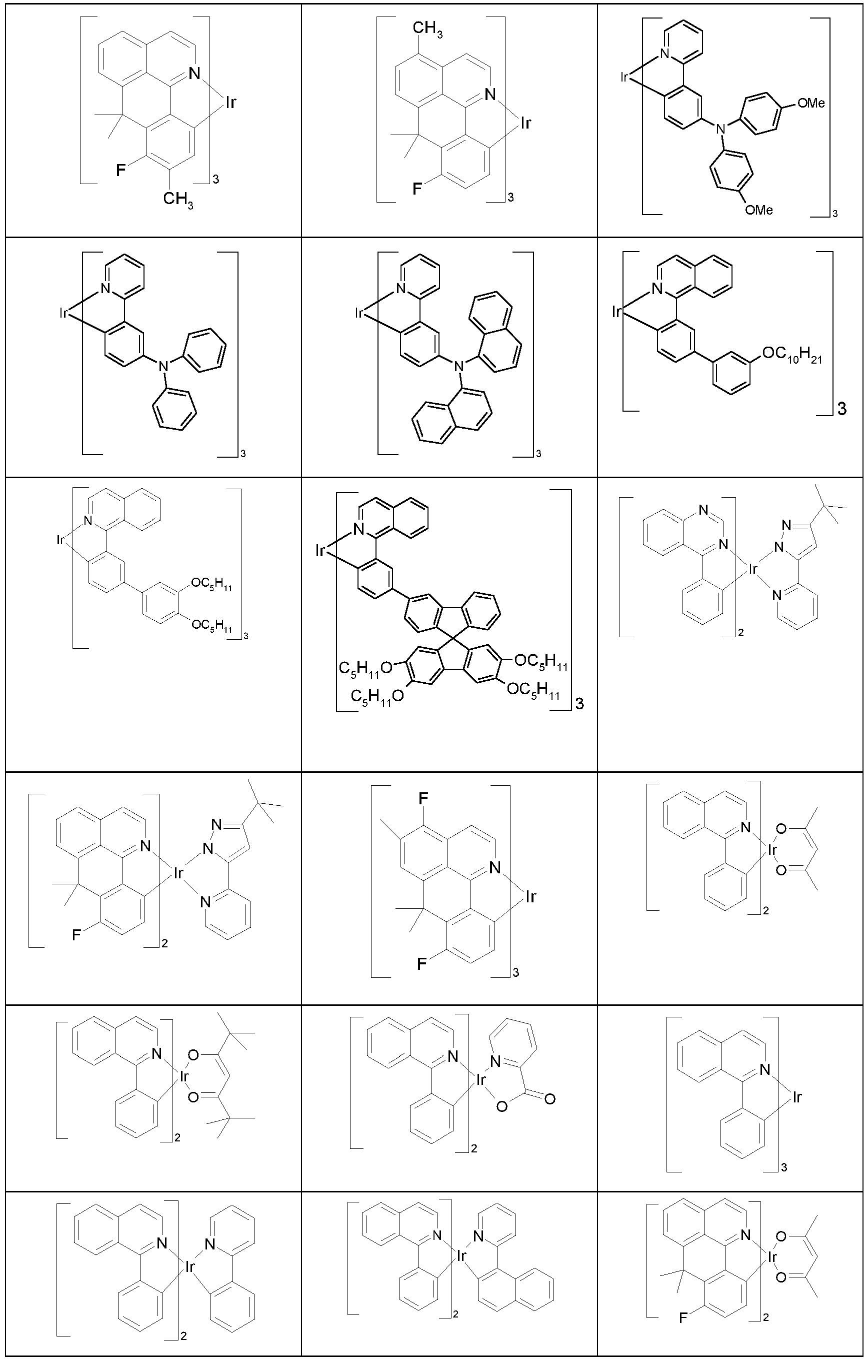

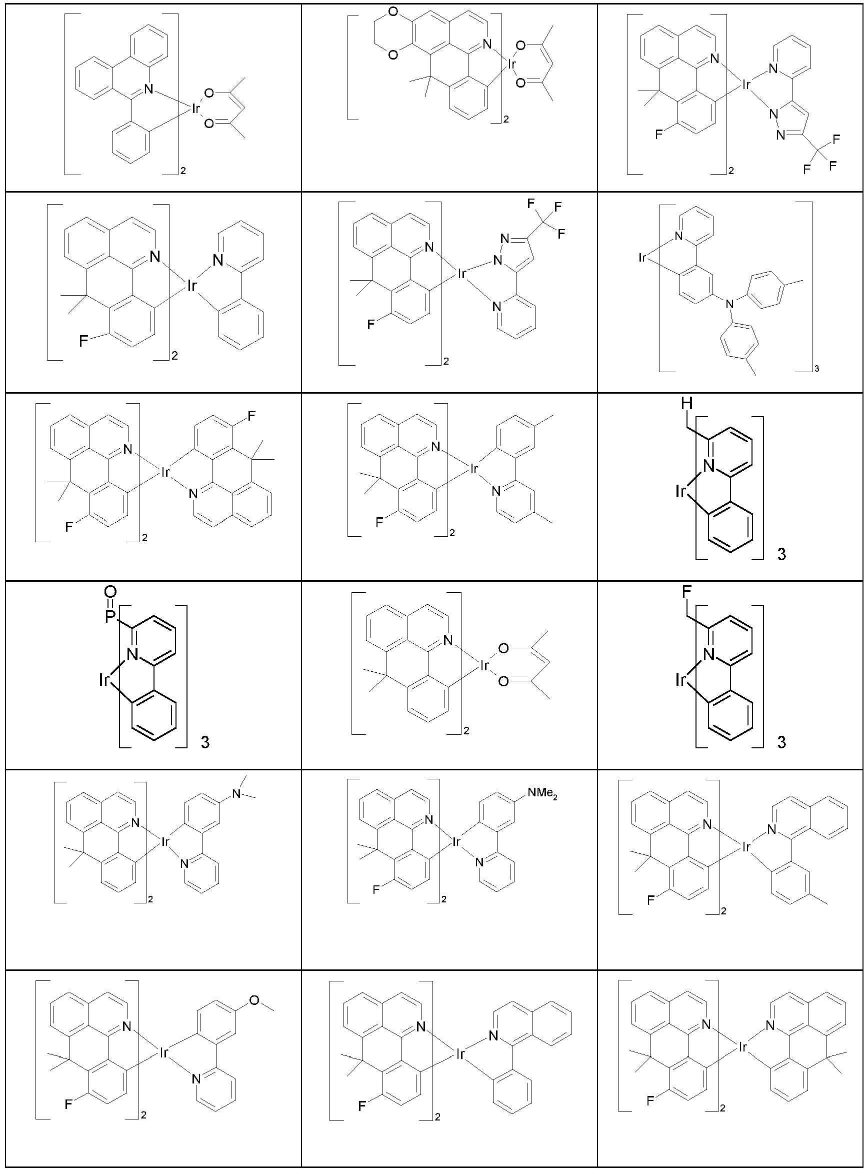

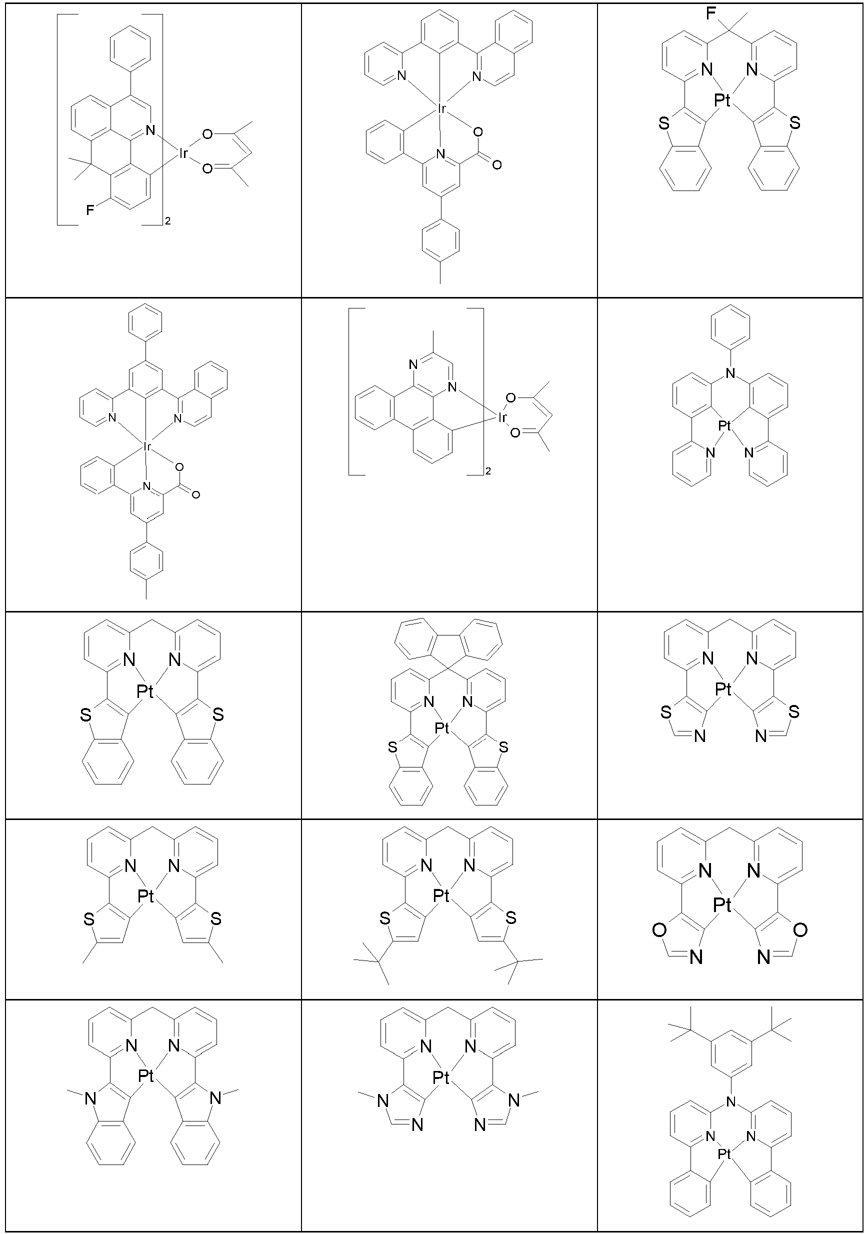

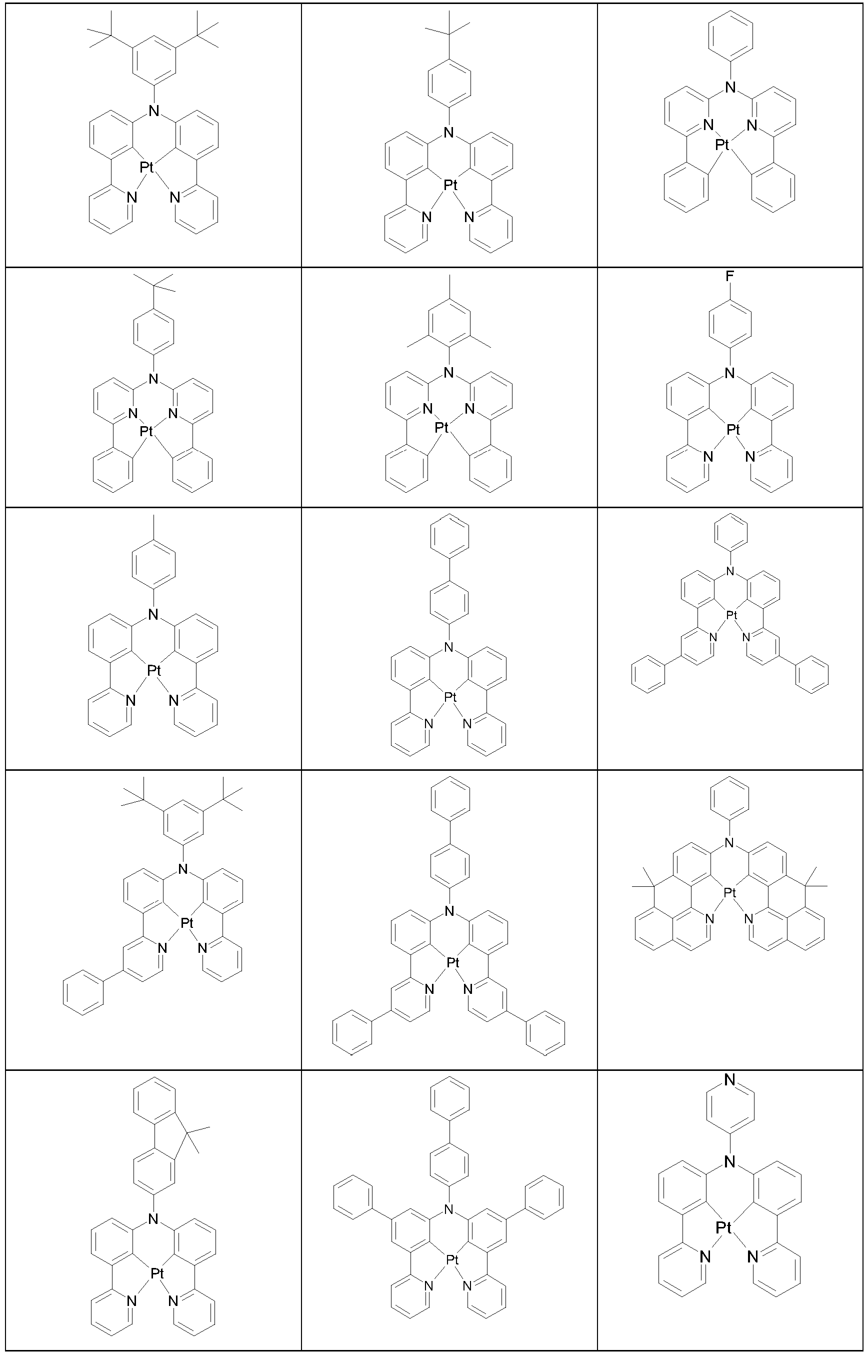

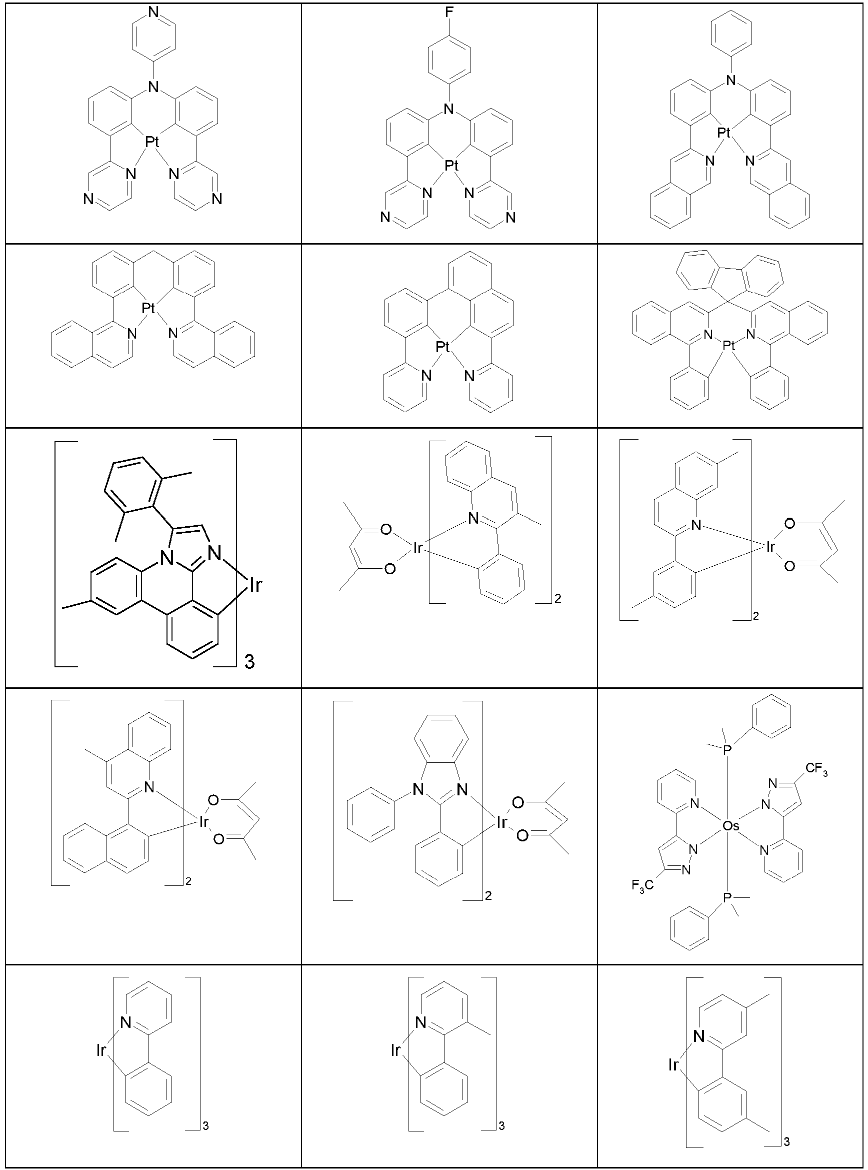

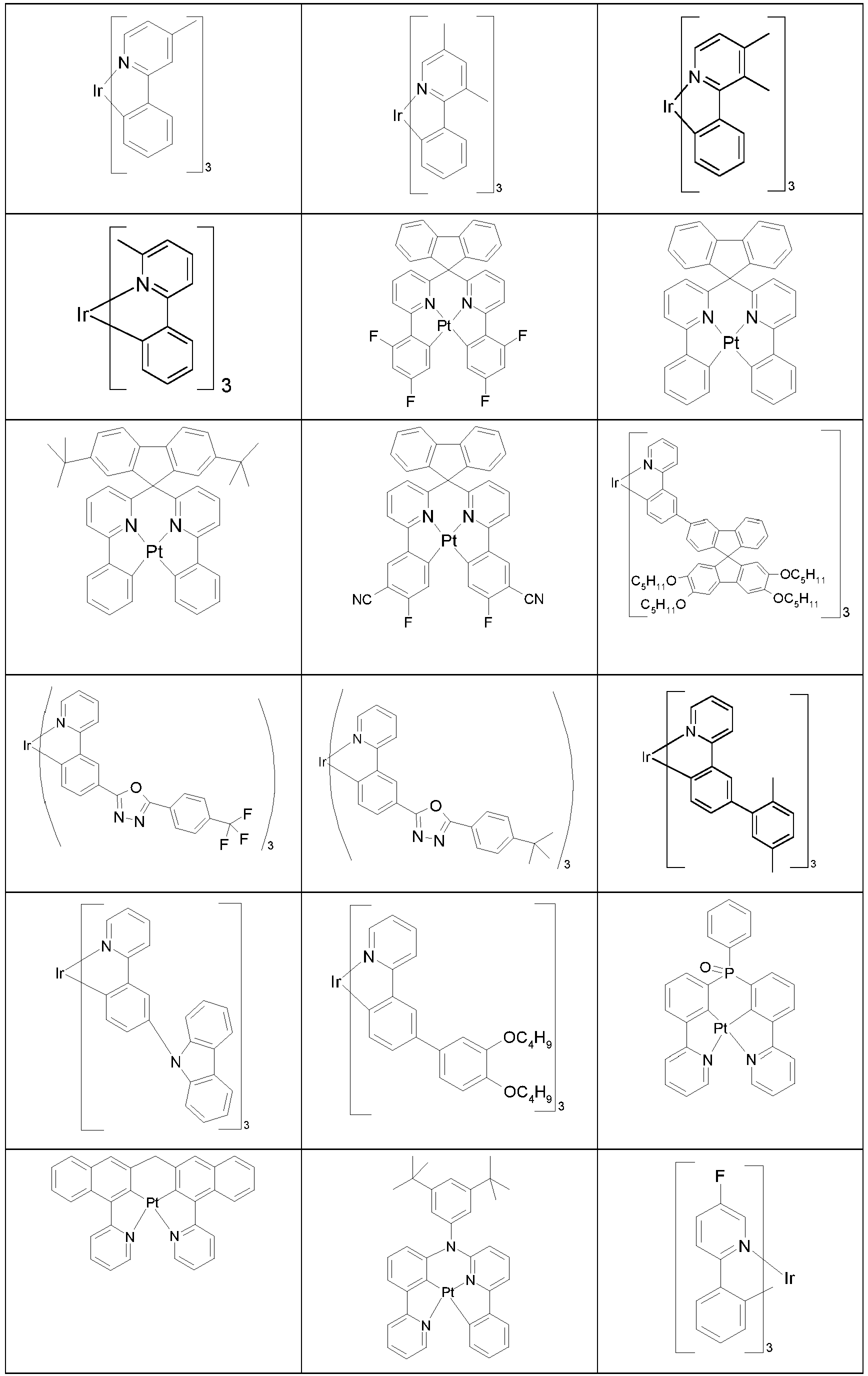

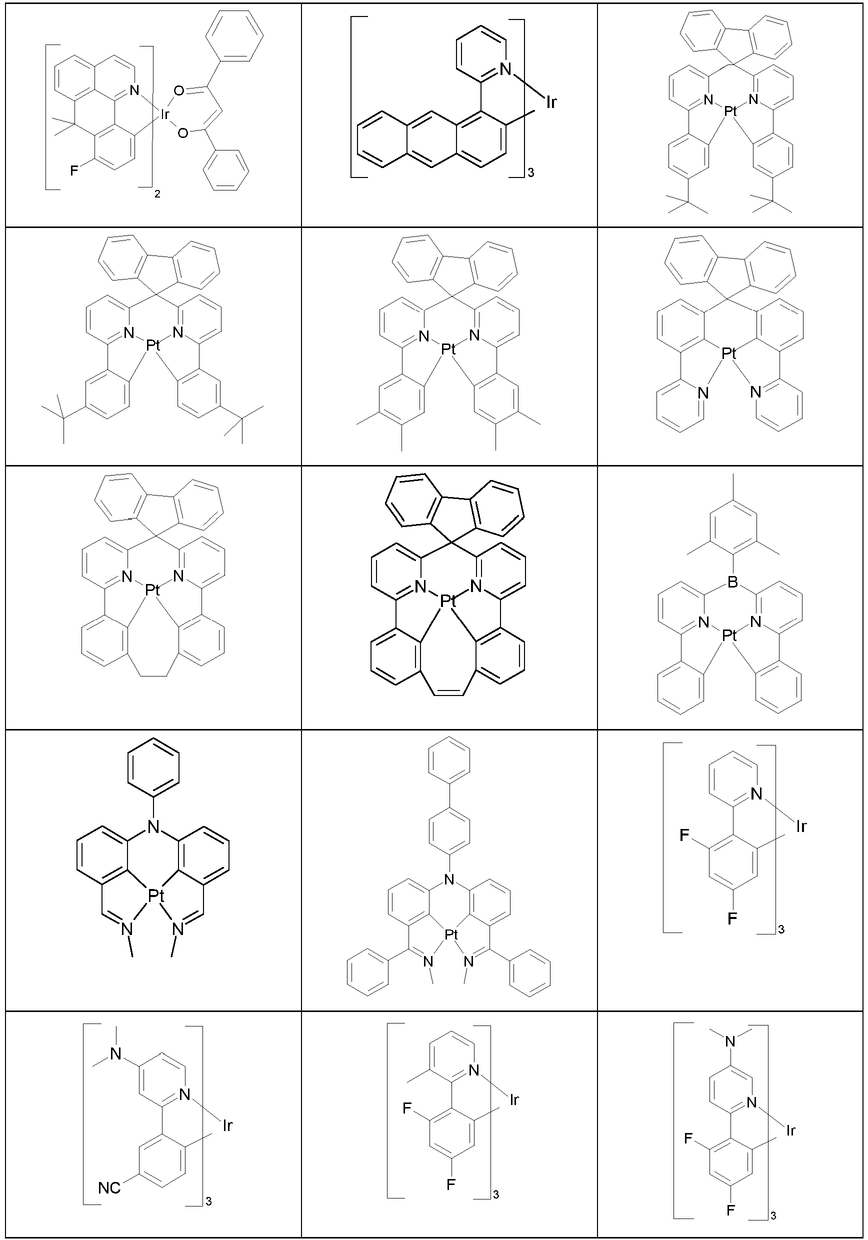

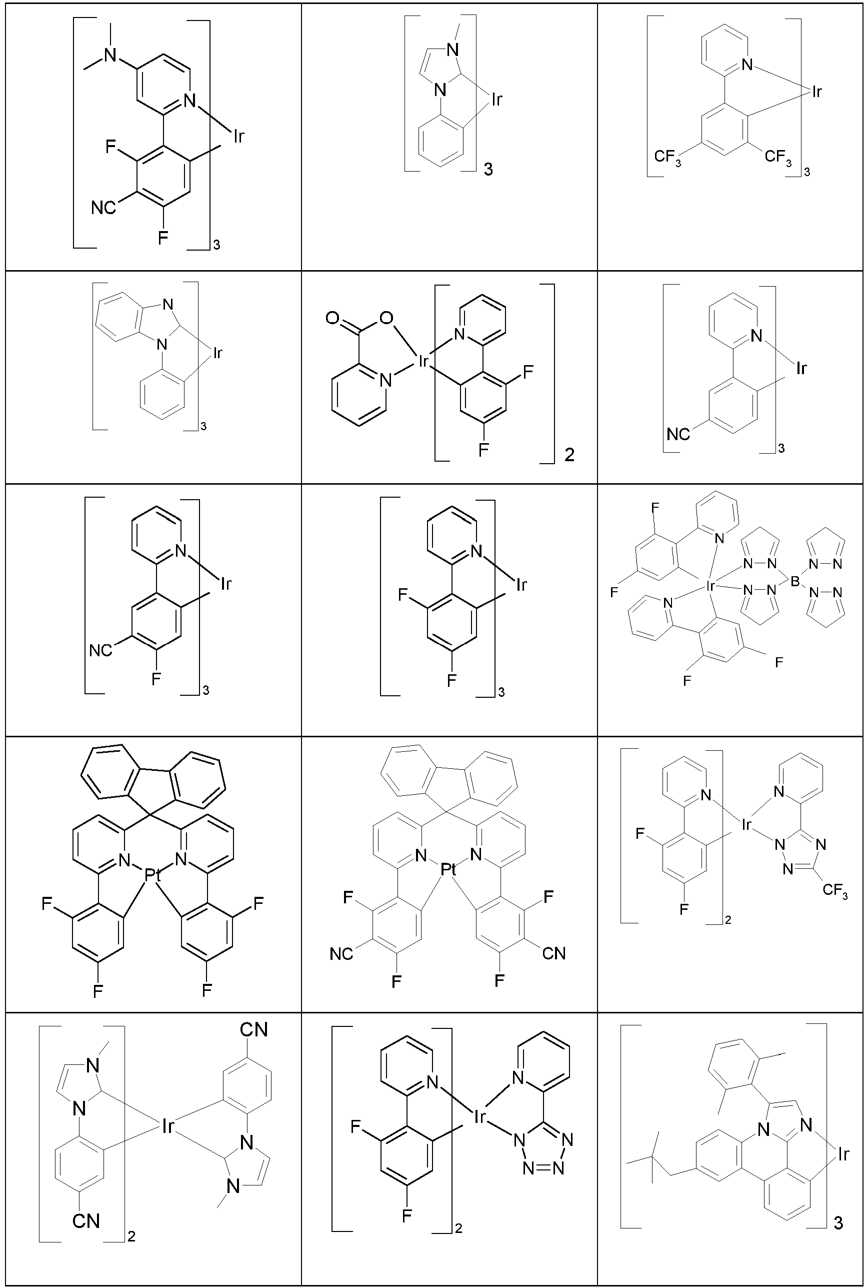

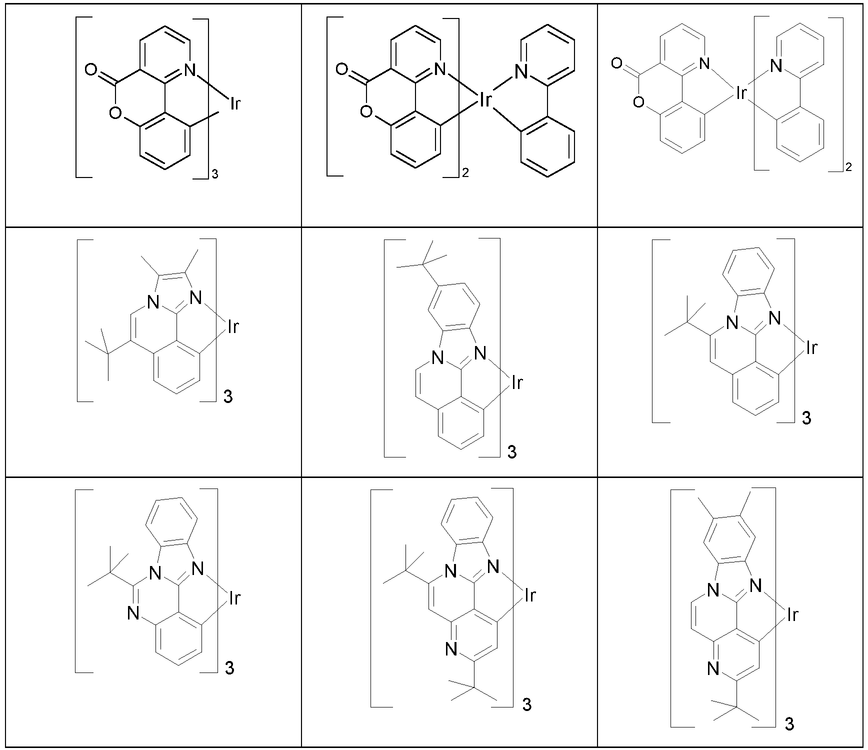

Bevorzugte fluoreszierende Dotanden sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte Dotanden sind Indenofluorenamine bzw. -diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen. Ebenfalls bevorzugt sind die in WO 2012/048780 offenbarten Pyren-Arylamine.

Als Matrixmaterialien, bevorzugt für fluoreszierende Dotanden, kommen neben den Verbindungen der Formel (I-f), (I-k) oder (I-l) Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw.

Arylengruppen aneinander gebunden sind. Bevorzugt sind weiterhin die in WO 2006/097208, WO 2006/131192, WO 2007/065550, WO 2007/110129, WO 2007/065678, WO 2008/145239, WO 2009/100925, WO 2011/054442, und EP 1553154 offenbarten Anthracenderivate, sowie die in EP 1749809, EP 1905754 und US 2012/0187826 offenbarten Pyren-Verbindungen. Bevorzugte Matrixmaterialien für phosphoreszierende Dotanden sind neben den Verbindungen der Formel (I-f), (I-k) oder (I-I) aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Bis-carbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109, WO 2011/000455 oder WO 2013/041176, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinckomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte CarbazolDerivate, z. B. gemäß US 2009/0136779, WO 2010/050778, WO 2011/042107, WO 2011/088877 oder WO 2012/143080, Triphenylenderivaten, z. B. gemäß WO 2012/048781, oder Lactame, z. B. gemäß WO 2011/116865 oder WO 2011/137951.

Geeignete Ladungstransportmaterialien, wie sie in der Lochinjektions- bzw.

Lochtransportschicht bzw. Elektronenblockierschicht oder in der Elektronentransportschicht der erfindungsgemäßen organischen Elektrolumineszenzvorrichtung verwendet werden können, sind neben den Verbindungen der Formel (I-f), (I-k) oder (I-I) beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Benzimidazolderviate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Als Lochtransportmaterialien sind insbesondere bevorzugt die Verbindungen der Formel (I-f), (I-k) oder (I-I). Bevorzugt sind als Lochtransportmaterialien, die in einer Lochtransport-, Lochinjektions- oder Elektronenblockierschicht in der erfindungsgemäßen Elektrolumineszenzvorrichtung verwendet werden können, Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder der noch nicht offengelegten EP 12000929.5), Fluoren-Amine (z. B. gemäß den noch nicht offengelegten Anmeldungen EP 12005369.9, EP 12005370.7 und EP 12005371.5), Spiro-Dibenzopyran-Amine (z. B. gemäß WO 2013/083216) und Dihydroacridin-Derivate (z. B. gemäß WO 2012/150001).

Als Kathode der elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die elektronische Vorrichtung wird bei der Herstellung entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße elektronische Vorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I-f), (I-k) oder (I-I) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Gegenstand der Erfindung ist somit weiterhin ein Verfahren zur Herstellung der erfindungsggemäßen elektronischen Vorrichtung, dadurch gekennzeichnet, dass mindestens eine organische Schicht durch Gasphasenabscheidung oder aus Lösung aufgebracht wird.

Erfindungsgemäß können die elektronischen Vorrichtungen enthaltend eine oder mehrere Verbindungen gemäß Formel (I-f), (I-k) oder (I-I) in Displays, als Lichtquellen in Beleuchtungsanwendungen sowie als Lichtquellen in medizinischen und/oder kosmetischen Anwendungen (z.B. Lichttherapie) eingesetzt werden.

### Ausführungsbeispiele zum besseren Verständnis der Erfindung, die gezeigten Verbindungen, mit Ausnahme der im folgenden gezeigten Verbindungen 6o und 6s mit der Vorläuferverbindung Int-5c, fallen nicht unter die Erfindung

### A) Synthesebeispiele:

Die nachfolgenden Synthesen werden, sofern nicht anders angegeben, unter einer Schutzgasatmosphäre in getrockneten Lösungsmitteln durchgeführt. Die Metallkomplexe werden zusätzlich unter Ausschluss von Licht gehandhabt. Die Lösungsmittel und Reagenzien können z. B. von Sigma-ALDRICH bzw. ABCR bezogen werden. Die Angaben in eckigen Klammern zu literaturbekannten chemischen Verbindungen beziehen sich auf die CAS-Nummer.

### Synthese von Vorstufen:

### Beispiel Int-1: 3-Dibenzofuran-4-yl-9-phenyl-9H-carbazol

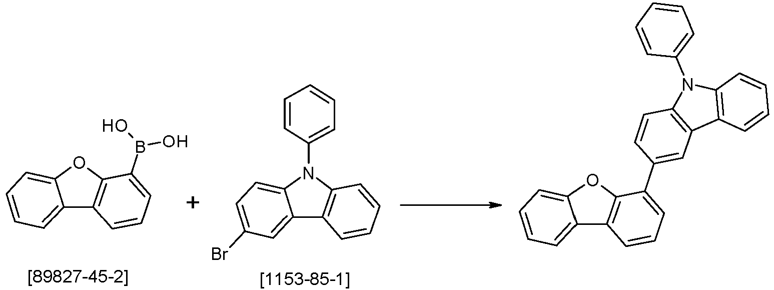

43,35 g (204,1 mmol) Dibenzofuran-4-boronsäure, 60 g (186,2 mmol) 3-Bromo-9-phenyl-9H-carbazol, 118,4 ml (237 mmol) Na₂CO₃ (2M-Lösung) werden in 180 mL Toluol, 180 mL Ethanol und 150 mL Wasser suspendiert. Zu dieser Suspension werden 3,9 g (3,3 mmol) Pd(PPh₃)₄ gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockne eingeengt. Der Rückstand wird aus Toluol umkristallisiert. Die Ausbeute beträgt 75,7 g (181 mmol), entsprechend 96 % der Theorie.

Analog können folgende Verbindungen erhalten werden:

| | Edukt 1 | Edukt 2 | Produkt | Ausbe ute |
|---|---|---|---|---|
| Int-1a | 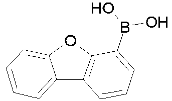 [89827-45-2] | 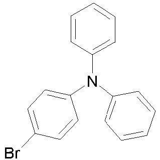 [36809-26-4 ] | 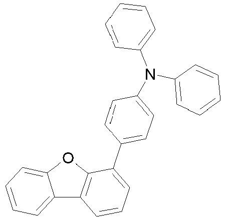 | 90% |
| Int-1b | 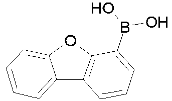 [89827-45-2] | 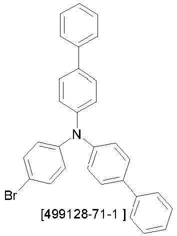 | 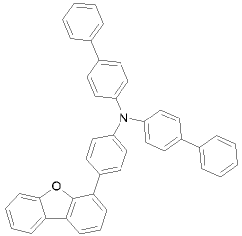 | 92% |
| Int-1c | 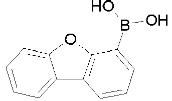 [89827-45-2] | 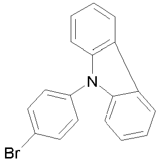 [57102-42-8] | 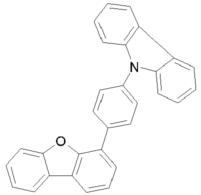 | 89% |
| Int-1d | 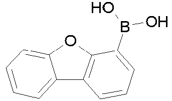 [89827-45-2] | 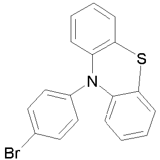 [63524-03-8] | 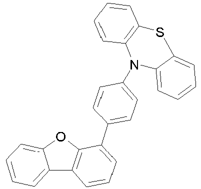 | 92% |
| Int-1e | 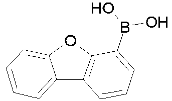 [89827-45-2] | 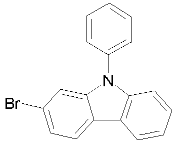 [94994-62-4 ] | 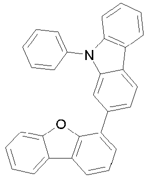 | 86% |
| Int-1f | 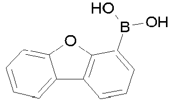 [89827-45-2] | 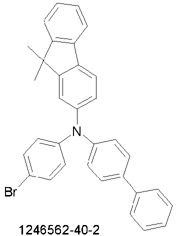 | 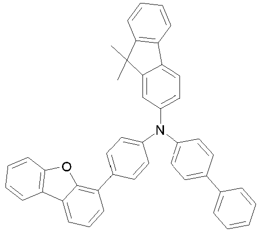 | 96% |
| Int-1g | 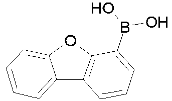 [89827-45-2] | 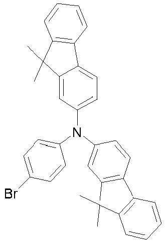 [313050-71-4 ] | 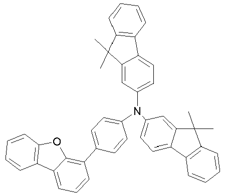 | 90% |

### Beispiel Int-2: Bis-biphenyl-4-yl-dibenzofuran-4-yl-amin

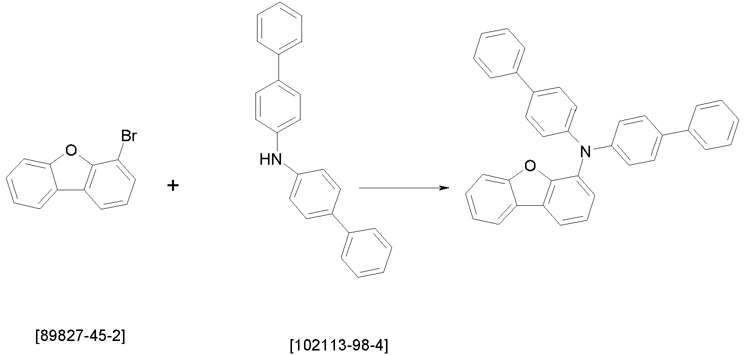

Eine entgaste Lösung von 43,92 g (176 mmol) 4-Brom-Dibenzofuran und 47,41 g (148 mmol) Bis-biphenyl-4-yl-amin in 700 mL Toluol wird 30 min mit N₂ gesättigt. Danach wird die Mischung zuerst mit 2,51 mL (10,3 mmol) 1M Lösung in Toluol P(*t*Bu)₃, dann mit 1,66 g (7,3 mmol) Palladium(II)acetat versetzt und anschließend 21,24 g (222 mmol) NaOtBu im festen Zustand zugegeben. Die Reaktionsmischung wird 6 h unter Rückfluss erhitzt. Nach Abkühlen auf Raumtemperatur werden vorsichtig 500 mL Wasser zugegeben. Die wässrige Phase wird mit 3x 70 mL Toluol gewaschen, über MgSO₄ getrocknet und das Lösungsmittel im Vakuum entfernt. Danach wird das Rohprodukt über Kieselgel mit Heptan/Essigsäureester (20:1) chromatographisch gereinigt. Die Ausbeute beträgt 70,91 g (142,8 mmol), entsprechend 94 % der Theorie.

Analog können folgende Verbindungen erhalten werden:

| | Edukt 1 | Edukt 2 | Produkt | Ausbeute |
|---|---|---|---|---|
| Int-2a | 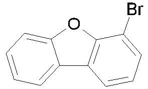 [89827-45-2] | 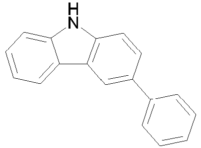 [103012-26-6 ] | 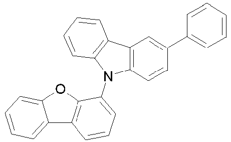 | 90% |
| Int-2b | 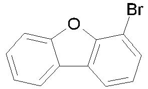 [89827-45-2] | 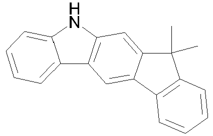 [1257220-47-5] | 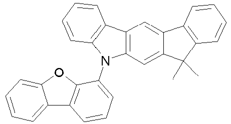 | 87% |
| Int-2c | 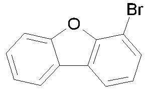 [89827-45-2] | 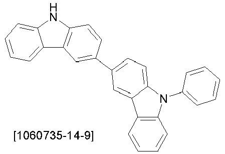 | 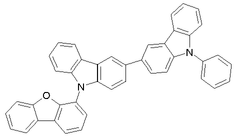 | 83% |
| Int-2d | 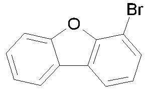 [89827-45-2] | 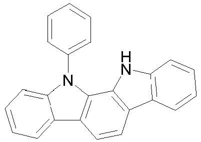 [1024598-06-8] | 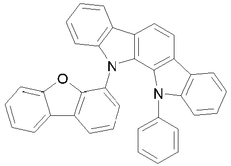 | 57% |
| Int-2e | 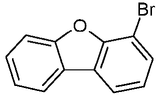 [89827-45-2] | 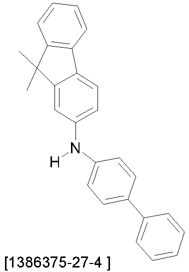 | 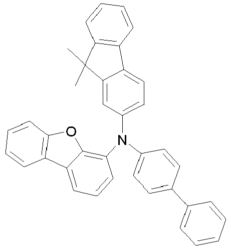 | 86% |
| Int-2f | [89827-45-2] | 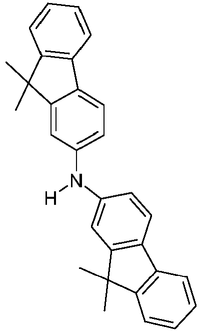 [1386375-16-1 ] | 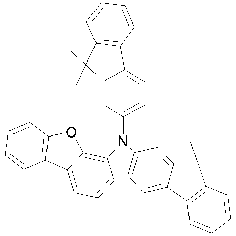 | 85% |

### Beispiel Int-3: 9-Phenyl-3-(6-trimethylsilanyl-dibenzofuran-4-yl)-9H-carbazol

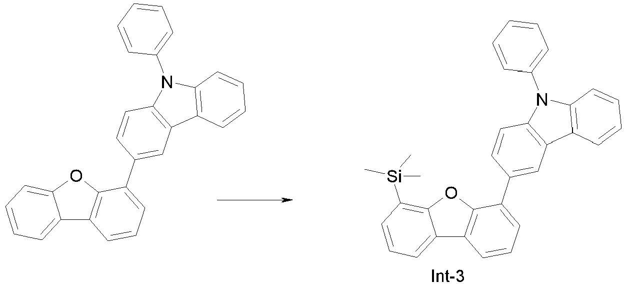

Eine auf 15 °C gekühlte Lösung von 49 g (121 mmol) 3-Dibenzofuran-4-yl-9-phenyl-9H-carbazol und 28g (242 mmol)TMEDA in 1000 ml THF wird tropfenweise mit 127 ml (225,4 mmol) n-Butyllithium (2.5 M in Hexan) versetzt. Die Reaktionsmischung wird 3h bei Raumtemperatur gerührt, dann kühlt man auf 0 °C und tropft innerhalb 30 min. 26 g (242 mmol) Chlortrimethylsilan hinzu und rührt 16 h bei Raumtemperatur. Anschließend wird das Lösungsmittel im Vakuum entfernt und der Rückstand chromatographisch über Kieselgel mit Toluol:Dichloromethan 2:1 gereinigt. Ausbeute: 34 g (72 mmol), 60 % der Theorie.

Analog können folgende Verbindungen erhalten werden:

| | Edukt 1 | Produkt | Ausbeute |
|---|---|---|---|
| Int-3a | 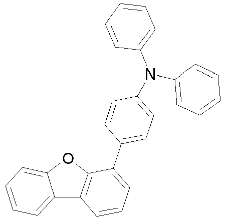 | 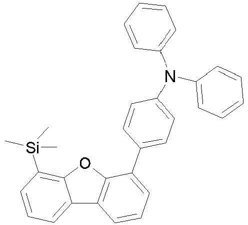 | 81% |
| Int-3b | 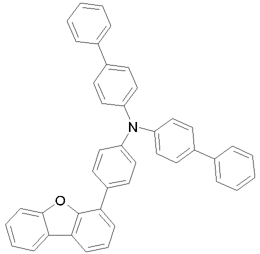 | 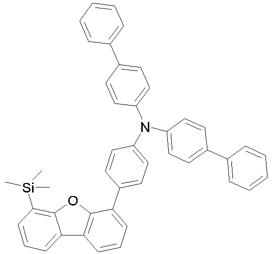 | 88% |
| Int-3c | 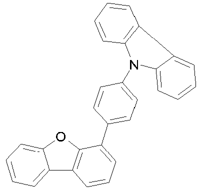 | 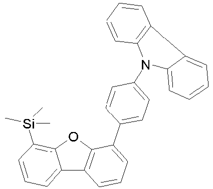 | 84% |
| Int-3d | 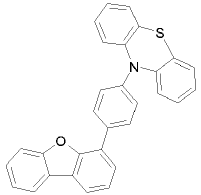 | 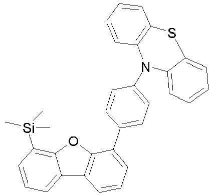 | 88% |
| Int-3e | 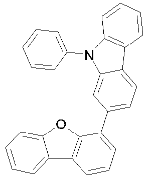 | 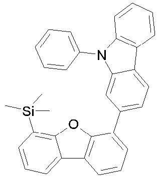 | 70% |
| Int-3f | 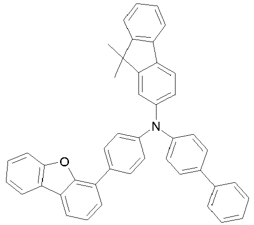 | 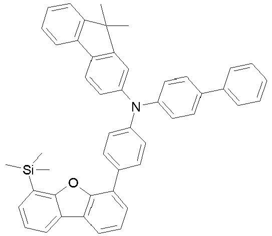 | 86% |
| Int-3g | 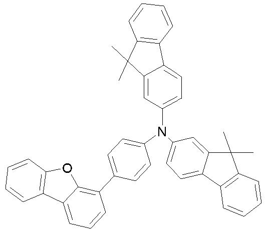 | 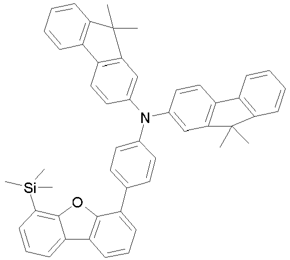 | 79% |
| Int-3h | 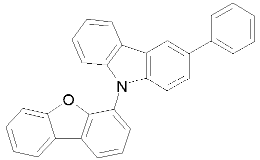 | 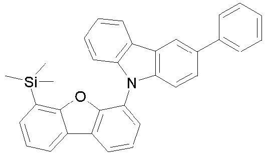 | 75% |
| Int-3i | 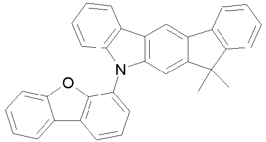 | 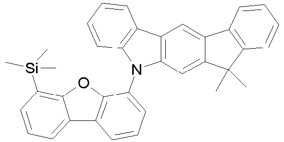 | 72% |
| Int-3j | 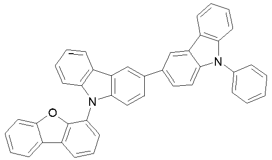 | 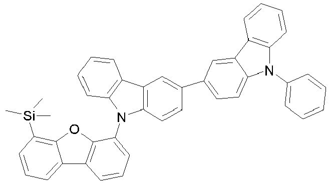 | 81% |
| Int-3k | 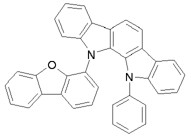 | 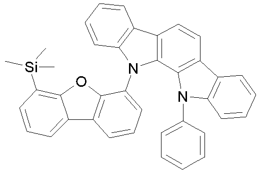 | 83% |
| Int-3l | 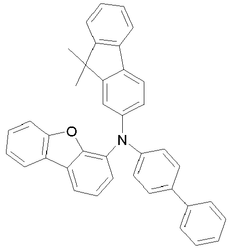 | 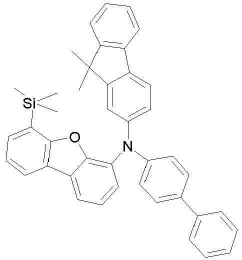 | 88% |
| Int-3m | 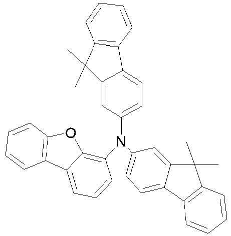 | 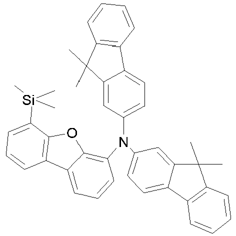 | 84% |

### Beispiel Int-4: B-[6-(Phenyl-9H-carbazol-3-yl)-4-dibenzofuranyl]-boronsäure

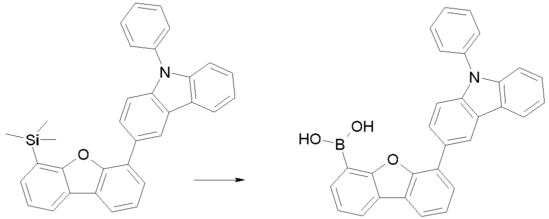

Unter Schutzgas wird eine Lösung von 34 g (72 mmol) B-[6-(Phenyl-9H-carbazol-3-yl)-4-dibenzofuranyl]-boronsäure in 500 ml Dichloromethan tropfenweise mit 21 g (86 mmol) Bromtribromid versetzt und 10 h bei Raumtemperatur gerührt. Danach wird die Mischung langsam mit etwas Wasser versetzt und der ausgefallene Rückstand abfiltriert und mit Heptan gewaschen. Die Ausbeute beträgt 28 g (62 mmol), entsprechend 86 % der Theorie.

Analog können folgende Verbindungen erhalten werden:

| | Edukt 1 | Produkt | Ausbeute |
|---|---|---|---|
| Int-4a | 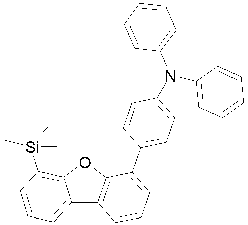 | 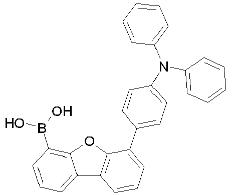 | 82% |
| Int-4b | 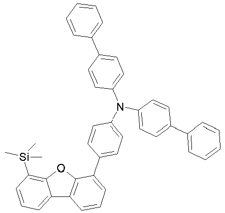 | 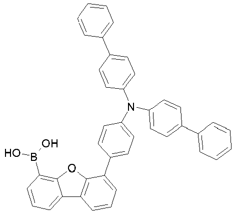 | 81% |
| Int-4c | 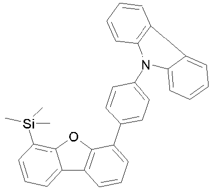 | 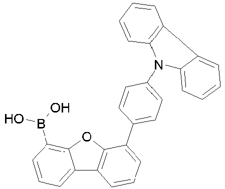 | 87% |
| Int-4d | 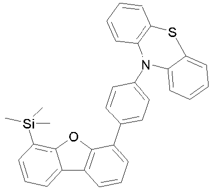 | 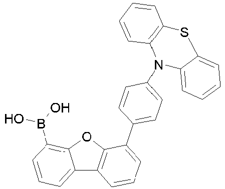 | 86% |
| Int-4e | 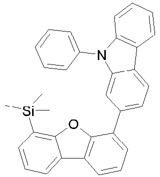 | 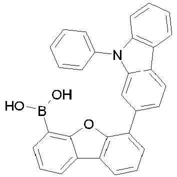 | 79% |
| Int-4f | 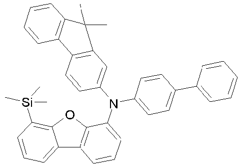 | 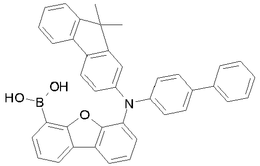 | 78% |
| Int-4g | 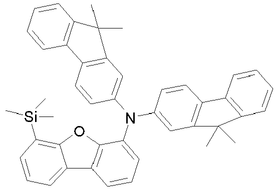 | 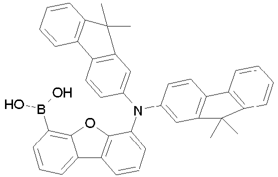 | 83% |
| Int-4h | 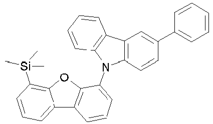 | 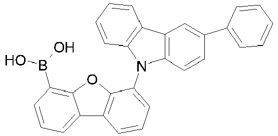 | 85% |
| Int-4i | 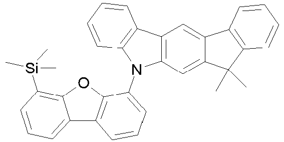 | 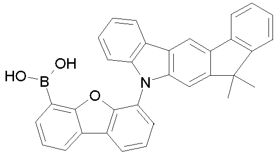 | 78% |
| Int-4j | 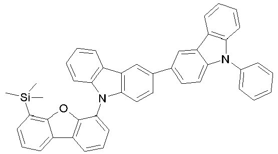 | 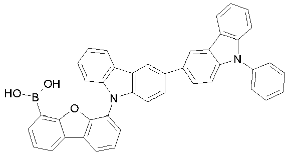 | 84% |
| Int-4k | 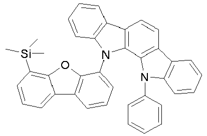 | 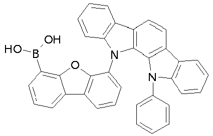 | 87% |
| Int-4l | 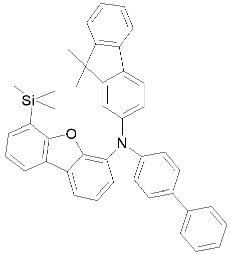 | 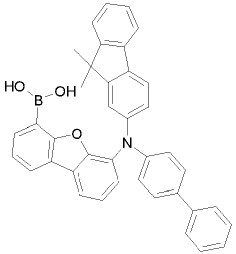 | 80% |
| Int-4m | 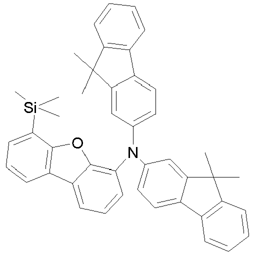 | 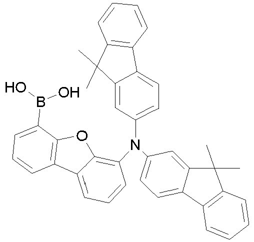 | 86% |

### Beispiel Int-5: B-[6-(Phenyl-9H-carbazol-3-yl)-4-dibenzofuranyl]-boronsäure

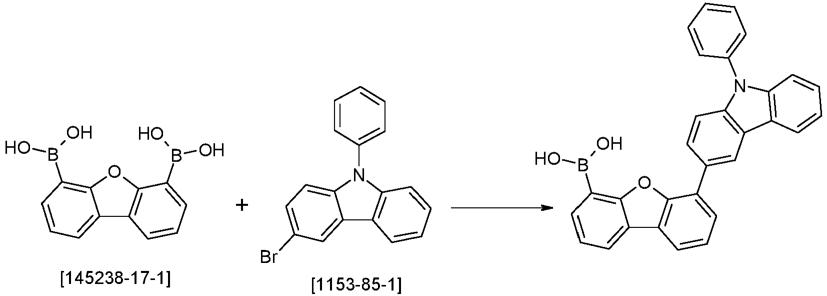

9 g (32 mmol) B,B'-4,6-dibenzofurandiylbisboronsäure, 15 g (31,6 mmol) 3-Bromo-9-phenyl-9H-carbazol, 31 ml (63 mmol) Na₂CO₃ (2M-Lösung) werden in 120 mL Toluol und 120 mL Ethanol suspendiert. Zu dieser Suspension werden 0,73 g (0,63 mmol) Pd(PPh₃)₄ gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockne eingeengt. Der Rückstand wird aus Toluol umkristallisiert. Die Ausbeute beträgt 11,1 g (24 mmol), entsprechend 70 % der Theorie.

Analog können folgende Verbindungen erhalten werden:

| | Edukt 1 | Edukt 2 | Produkt | Ausbeute |
|---|---|---|---|---|
| Int-5b | 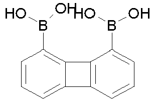 [480438-76-4] | 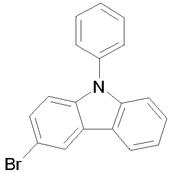 [1153-85-1] | 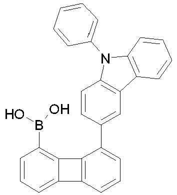 | 69% |
| Int-5c | 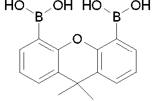 [862159-27-1 ] | 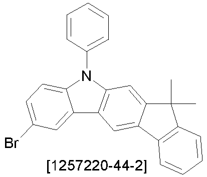 | 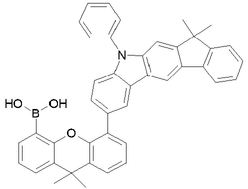 | 74% |

### Synthese von weiteren Verbindungen:

### Beispiel 6: Biphenyl-4-yl-(9,9-dimethyl-9H-fluoren-2-yl)-{4-[6-(9-phenyl-9H-carbazol-3-yl)-dibenzofuran-4-yl]-phenyl}-amin

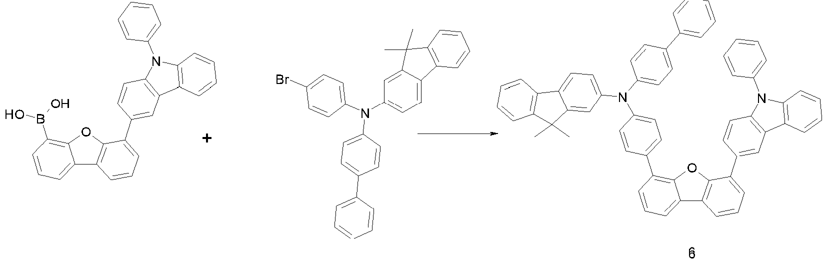

32,1 g (70 mmol) B-[6-(Phenyl-9H-carbazol-3-yl)-4-dibenzofuranyl]-boronsäure, 36,12 g (70 mmol) Biphenyl-4-yl-(4-bromo-phenyl)-(9,9-dimethyl-9H-fluoren-2-yl)-amin, und 78,9 ml (158 mmol) Na₂CO₃ (2M-Lösung) werden in 120 mL Ethanol und 100 mL Wasser suspendiert. Zu dieser Suspension werden 1,3 g (1,1 mmol) Pd(PPh₃)₄ gegeben, und die Reaktionsmischung wird 16 h unter Rückfluss erhitzt. Nach Erkalten wird die Mischung mit Dichloromethan versetzt, die organische Phase abgetrennt und über Kieselgel filtriert. Die Ausbeute beträgt 40 g (47 mmol), entsprechend 67,8 % der Theorie. Der Rückstand wird aus Toluol umkristallisiert und abschließend im Hochvakuum (p = 5 x 10⁻⁶ mbar) sublimiert. Die Reinheit beträgt 99.9%.

Analog können folgende Verbindungen erhalten werden:

| | Edukt 1 | Edukt 2 | Produkt |
|---|---|---|---|
| 6a | 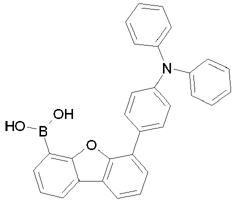 | 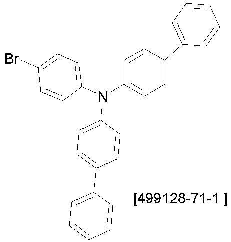 | 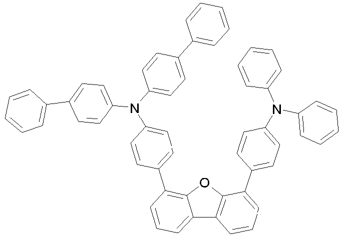 |
| 6b | 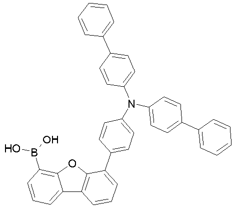 | 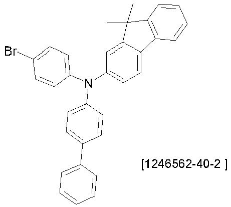 | 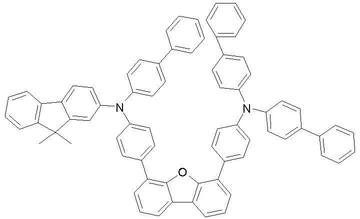 |
| 6c | 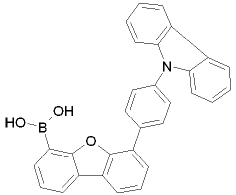 | 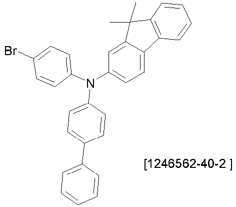 | 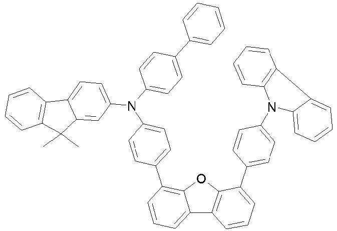 |
| 6d | 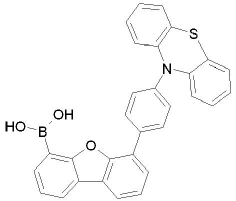 | 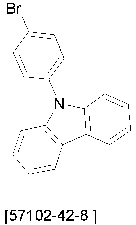 | 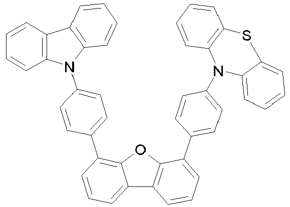 |
| 6e | 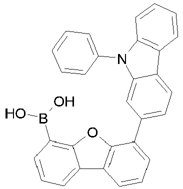 | 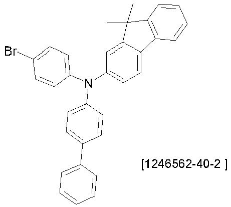 | 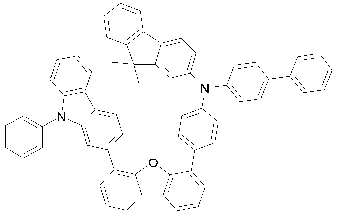 |
| 6f | 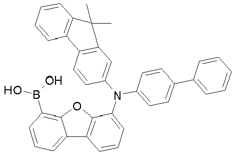 | 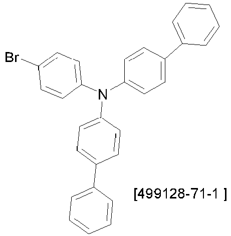 | 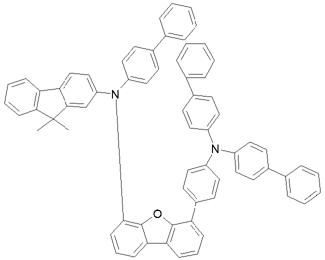 |
| 6g | 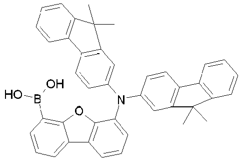 | 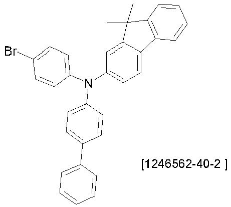 | 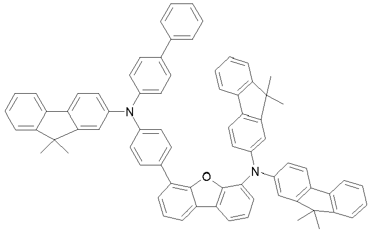 |
| 6h | 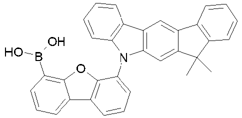 | 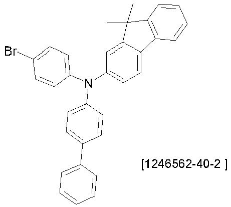 | 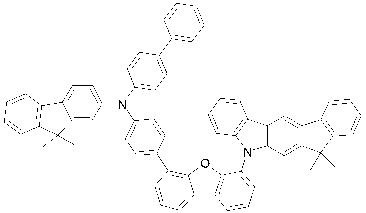 |
| 6i | 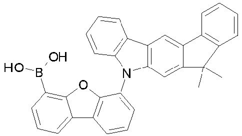 | 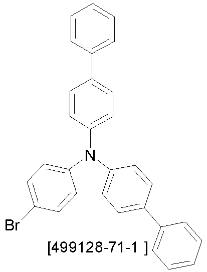 | 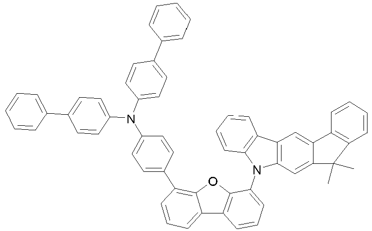 |
| 6j | 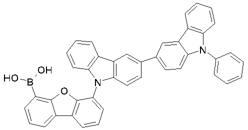 | 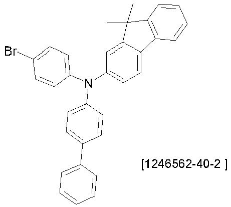 | 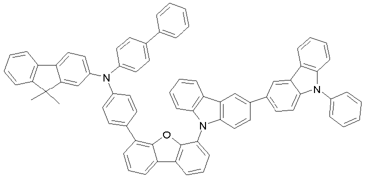 |
| 6k | 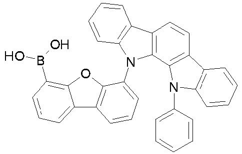 | 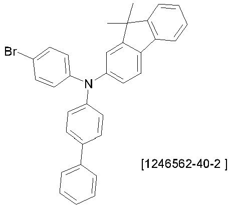 | 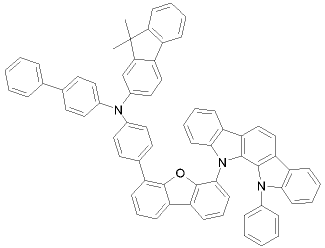 |
| 6l | 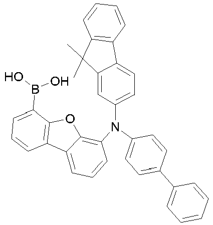 | 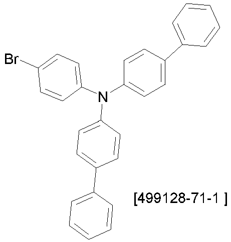 | 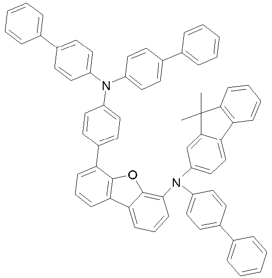 |
| 6n | 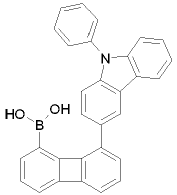 | 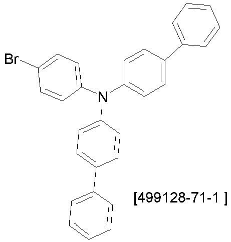 | 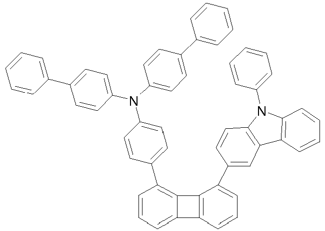 |
| 6o | 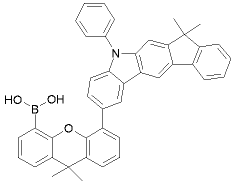 | 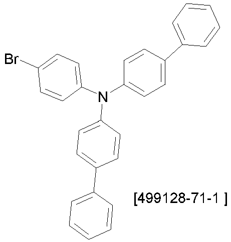 | 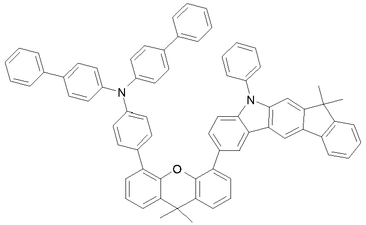 |
| 6p | 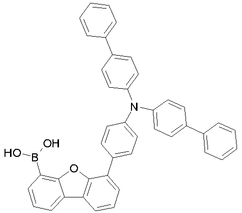 | 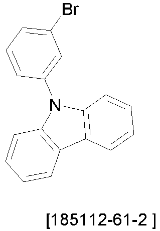 | 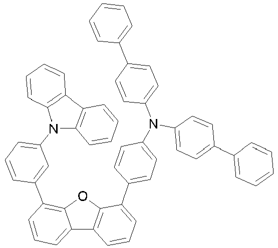 |
| 6r | 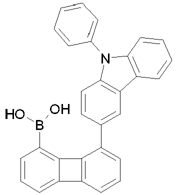 | 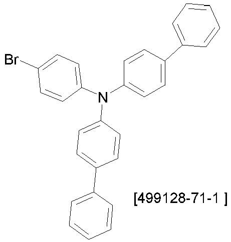 | 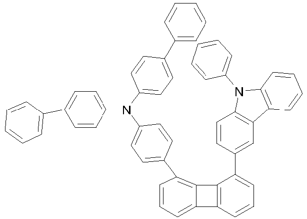 |
| 6s | 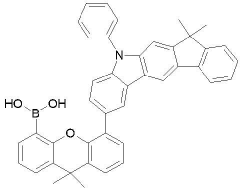 | 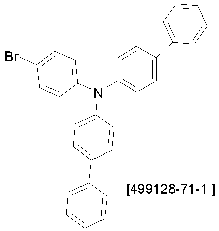 | 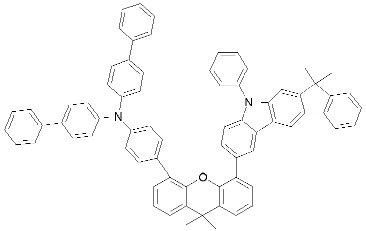 |
| 6t | 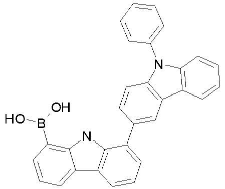 | 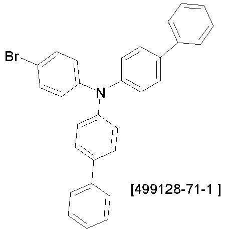 | 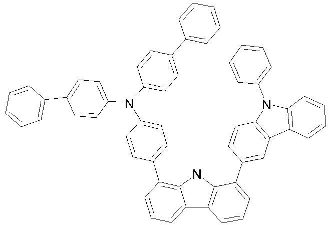 |

| | Ausbeute |
|---|---|
| 6a | 60 % |
| 6b | 77% |
| 6c | 82% |
| 6d | 78% |
| 6e | 84% |
| 6f | 63% |
| 6g | 52% |
| 6h | 71% |
| 6i | 69% |
| 6j | 73% |
| 6k | 52% |
| 6l | 48% |
| 6n | 56% |
| 6o | 67% |
| 6p | 69% |
| 6r | 45% |
| 6s | 64% |
| 6t | 60 % |

### Synthese von Vorstufen:

### Beispiel Int-7: 3-(6-Bromo-dibenzofuran-4-yl)-9-phenyl-9H-carbazol

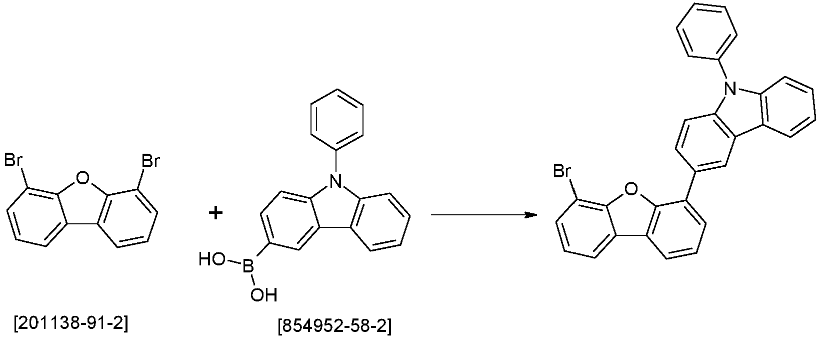

10,43 g (32 mmol) B-(9-Phenyl-9H-carbazol-3-yl)boronsäure, 8,9 g (31,6 mmol) 4,6-Dibromdibenzofuran und 31 ml (63 mmol) Na₂CO₃ (2M-Lösung) werden in 120 mL Toluol und 120 mL Ethanol suspendiert. Zu dieser Suspension werden 0,73 g (0,63 mmol) Pd(PPh₃)₄ gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockne eingeengt. Der Rückstand wird aus Toluol umkristallisiert. Die Ausbeute beträgt 11,4 g (23 mmol), entsprechend 73 % der Theorie.

Analog können folgende Verbindungen erhalten werden:

| | Edukt 1 | Edukt 2 | Produkt | Ausbeute |
|---|---|---|---|---|
| Int-7a | 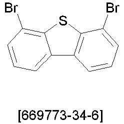 | 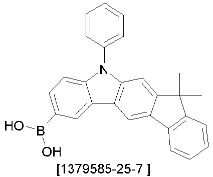 | 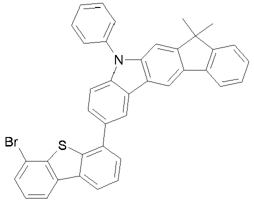 | 51% |
| Int-7b | 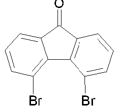 [69414-97-7] | 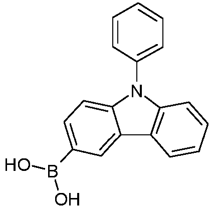 [854952-58-2] | 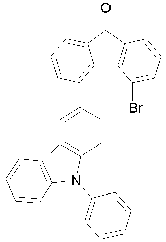 | 65% |
| Int-7c | 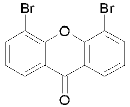 [1262398-42-4 ] | 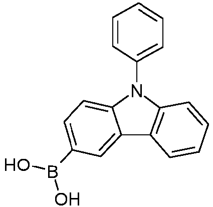 [854952-58-2] | 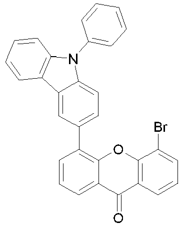 | 69% |
| Int-7d | 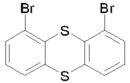 [176646-34-7 ] | 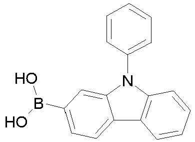 [1001911-63-2] | 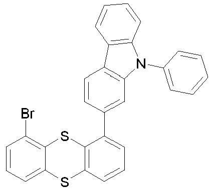 | 67% |
| Int-7e | 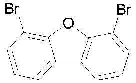 [201138-91-2] | 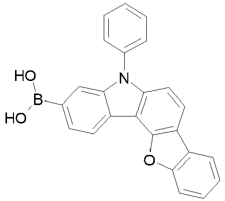 [1391729-62-6] | 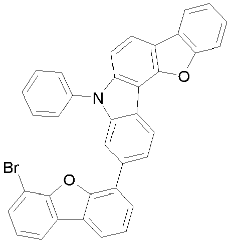 | 62% |
| Int-7f | 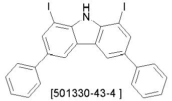 | 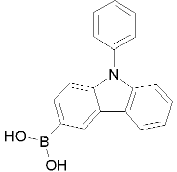 [854952-58-2] | 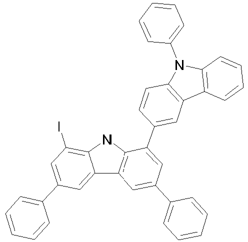 | 62% |
| Int-7g | 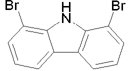 [553663-65-3] | 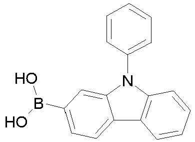 [1001911-63-2] | 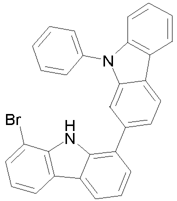 | 61% |
| Int-7h | 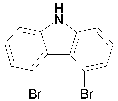 [905702-33-2] | 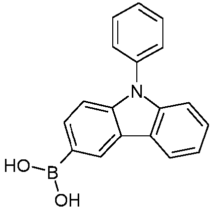 [854952-58-2] | 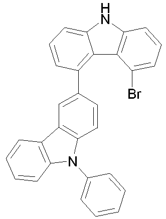 | 64% |
| Int-7i | 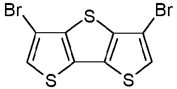 [502764-54-7] | 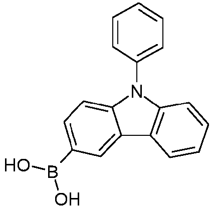 [854952-58-2] | 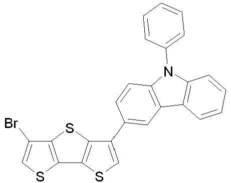 | 66% |
| Int-7j | 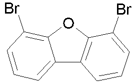 [201138-91-2] | 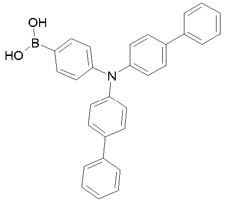 [943836-24-6 ] | 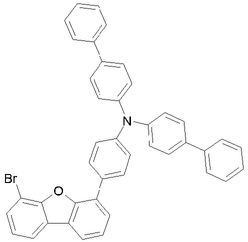 | 62% |
| Int-7k | 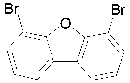 [201138-91-2] | 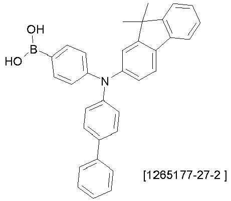 | 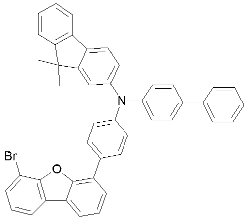 | 65% |

### Synthese von weiteren Verbindungen:

Analog können folgende Verbindungen durch zweifache Addition mit entsprechenden Boronsäuren erhalten werden:

| | Edukt 1 | Edukt 2 | Produkt |
|---|---|---|---|
| 7l | 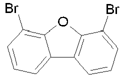 [201138-91-2] | 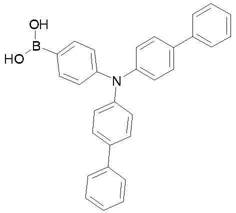 [943836-24-6 ] | 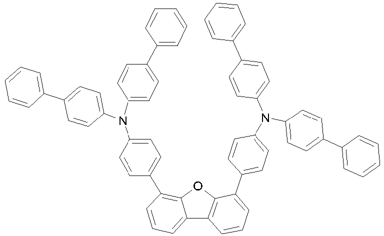 |
| 7m | 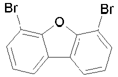 [201138-91-2] | 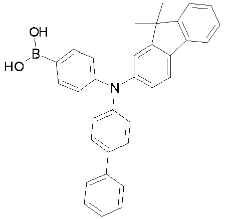 [1265177-27-2 ] | 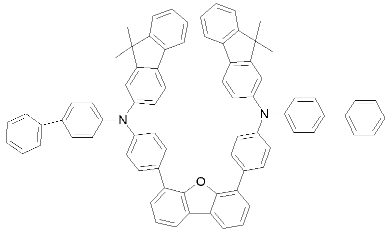 |
| 7n | 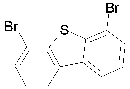 | 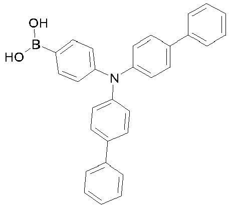 [943836-24-6 ] | 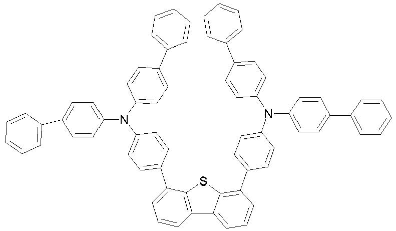 |
| 7o | 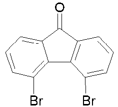 | 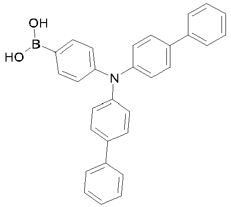 [943836-24-6 ] | 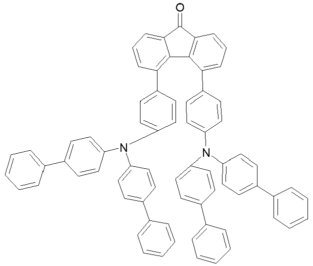 |
| 7p | 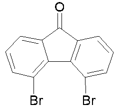 | 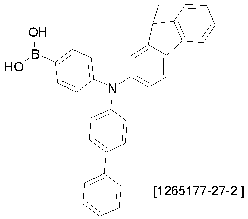 | 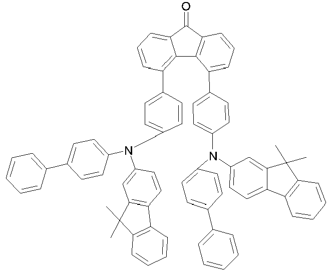 |
| 7q | 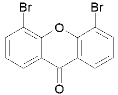 | 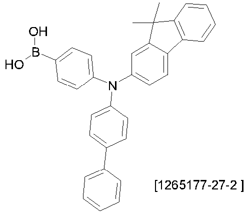 | 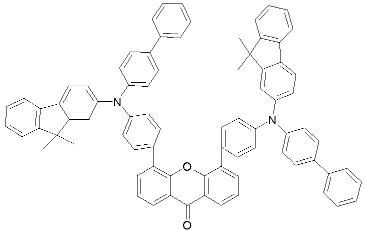 |
| 7r | 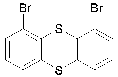 | 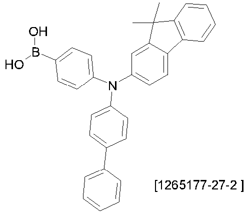 | 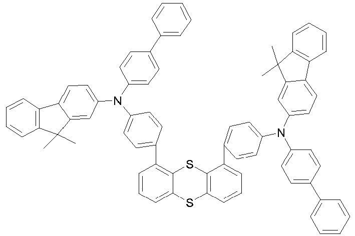 |
| 7s | 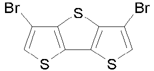 | 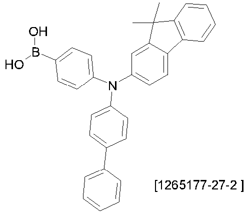 | 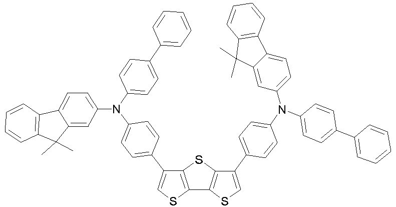 |
| 7t | 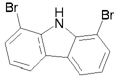 | 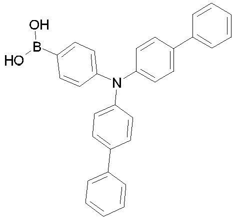 [943836-24-6 ] | 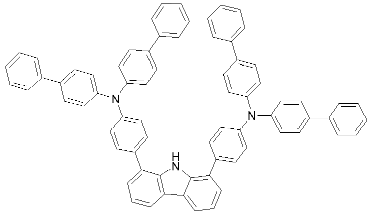 |
| 7u | 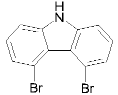 | 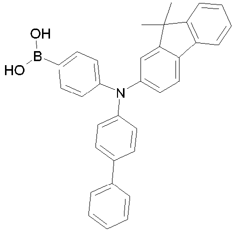 [1265177-27-2 ] | 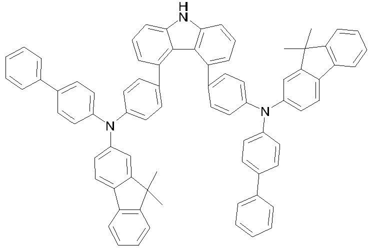 |
| 7v | 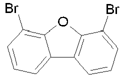 [201138-91-2] | 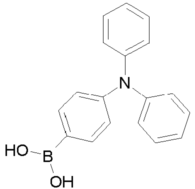 [201802-67-7] | 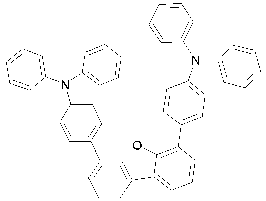 |

| | Ausbeute |
|---|---|
| 7l | 52% |
| 7m | 67% |
| 7n | 80% |
| 7o | 79% |
| 7p | 58% |
| 7q | 78% |
| 7r | 87% |
| 7s | 87% |
| 7t | 83% |
| 7u | 80% |
| 7v | 87% |

### Beispiel 8: Bis-biphenyl-4-yl-(4-{1-[3-eth-(Z)-ylidene-7-(9-phenyl-9H-carbazol-3-yl)-3H-benzofuran-(2Z)-yliden]-ethyl}-phenyl)-amin

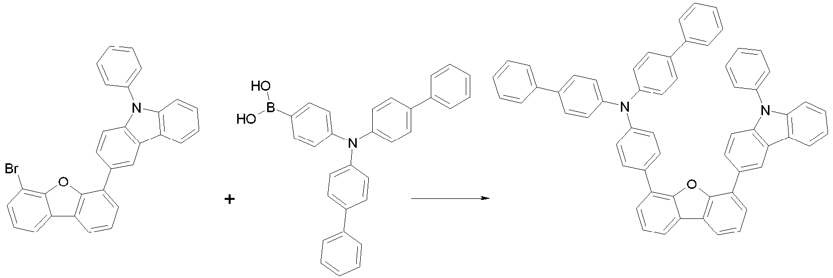

87g (180,1 mmol) 3-(6-Bromo-dibenzofuran-4-yl)-9-phenyl-9H-carbazol, 79 g (180,7 mmol) B-[4-[Bis([1,1'-biphenyl]-4-yl]amino]phenylboronsäure und 38,3 g (180,7 mmol) Kaliumphosphat werden in 500 ml Toluol, 250 ml 1,4-Dioxan und 120 ml Wasser suspendiert. Zu der Mischung werden 1,3 g (4,1 mmol) Tri(o-tolyl)phosphin und dann 461 mg (2 mmol) Palladium(II)acetat gegeben und die Reaktionsmischung 48 h unter Rückfluss erhitzt. Nach dem Erkalten wird die organische Phase abgetrennt, dreimal mit je 100 ml Wasser gewaschen und eingeengt. Nach der säulenchromatographischen Reinigung (SiO₂, *n*-Heptan/Dichlormethan 3/1) wird der erhaltene Schaum in Dichlormethan gelöst und mit Ethanol gefällt. Der Rückstand wird aus Toluol und aus Dichlormethan umkristallisiert und abschließend im Hochvakuum (p = 5 x 10⁻⁵ mbar) sublimiert. Ausbeute: 129 g (160 mmol), 90 %. Reinheit ca. 99 % n. HPLC.

Analog können folgende Verbindungen erhalten werden:

| | Edukt 1 | Edukt 2 | Produkt |
|---|---|---|---|
| 8a | 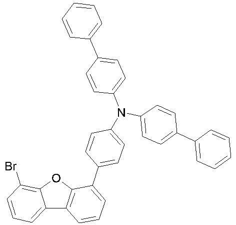 | 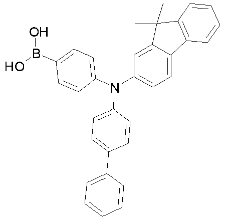 [1265177-27-2 ] | 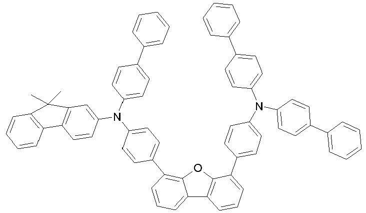 |
| 8b | 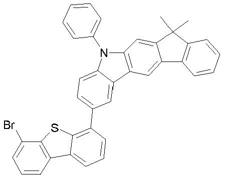 | 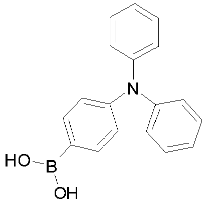 [201802-67-7] | 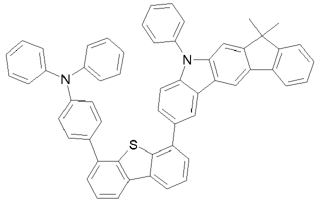 |
| 8c | 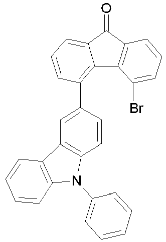 | 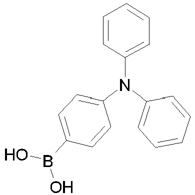 [201802-67-7] | 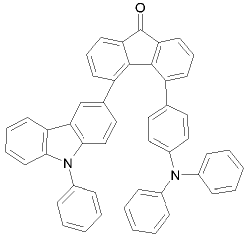 |
| 8d | 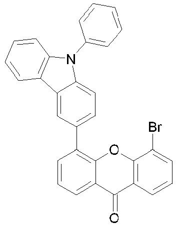 | 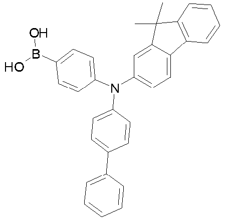 [1265177-27-2 ] | 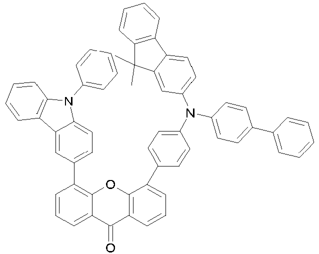 |
| 8e | 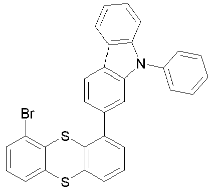 | 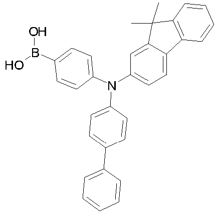 [1265177-27-2 ] | 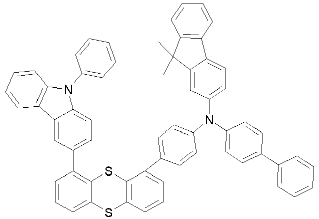 |
| 8f | 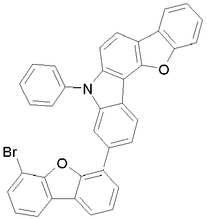 | 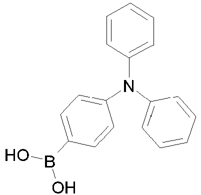 [201802-67-7] | 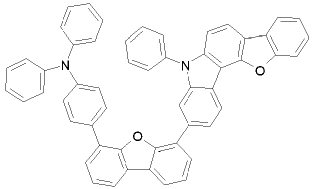 |
| 8g | 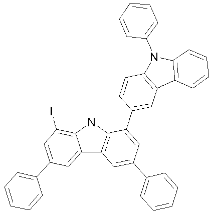 | 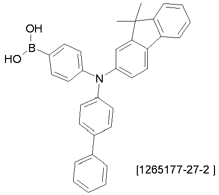 | 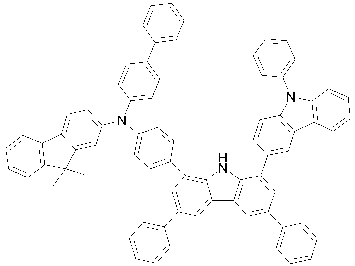 |
| 8h | 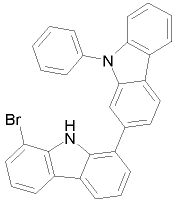 | 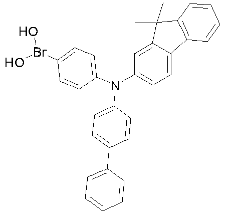 | 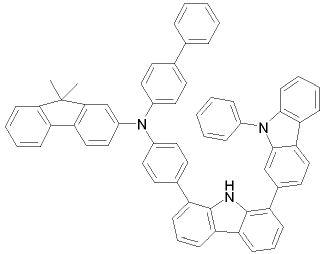 |
| 8i | 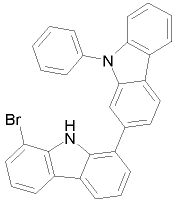 | 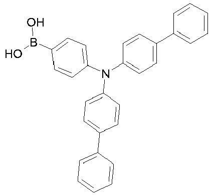 [943836-24-6 ] | 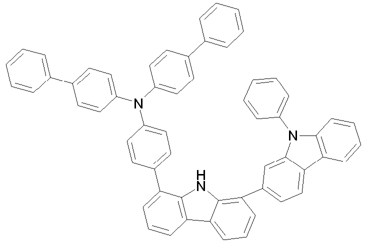 |
| 8j | 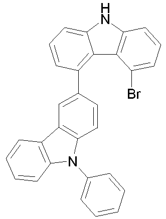 | 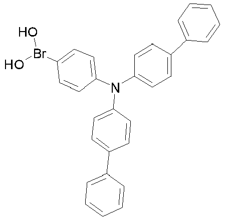 | 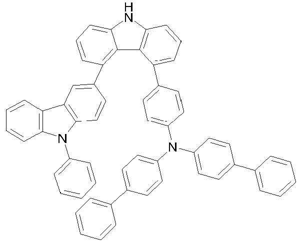 |
| 8k | 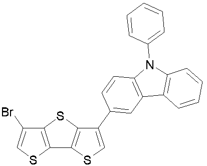 | 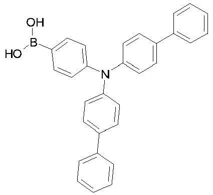 [943836-24-6 ] | 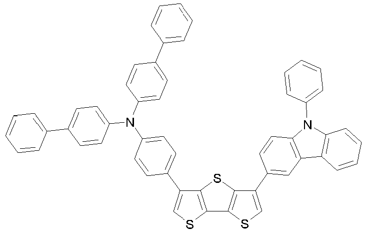 |
| 8l | 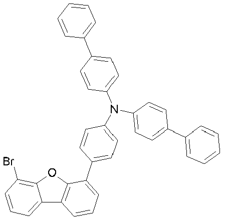 | 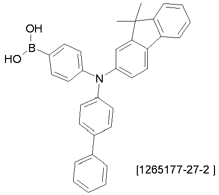 | 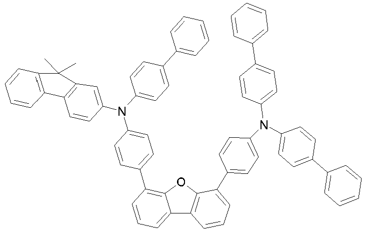 |
| 8m | 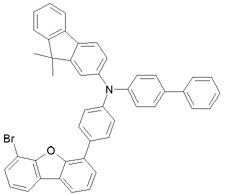 | 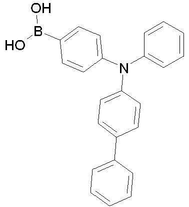 [1084334-86-0 ] | 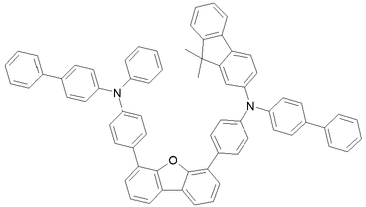 |

| | Ausbeute |
|---|---|
| 8a | 68% |
| 8b | 73% |
| 8c | 86% |
| 8d | 65% |
| 8e | 63% |
| 8f | 79% |
| 8g | 62% |
| 8h | 64% |
| 8i | 65% |
| 8j | 64% |
| 8k | 66% |
| 8l | 62% |
| 8m | 65% |

### Beispiel 9: Bis-biphenyl-4-yl-[4-(9,9'-diphenyl-9H,9'H-[1,2']bicarbazolyl-8-yl)-phenyl]-amin

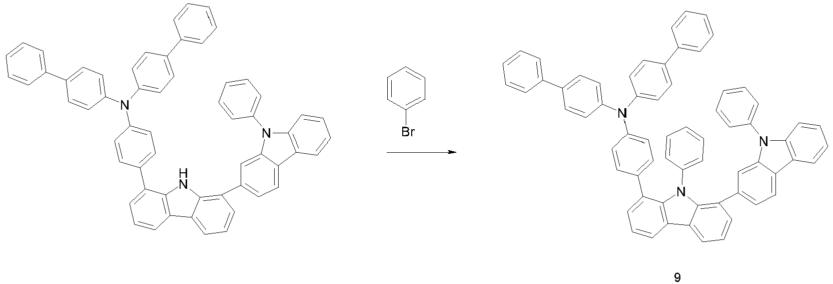

40 g (49,75 mmol) Bis-biphenyl-4-yl-[4-(9'-phenyl-9H,9'H-[1,2']bicarbazolyl-8-yl)-phenyl]-amin und 16,7 g (74,62 mmol) Brom-benzol werden in Toluol gelöst und via Schutzgaseinleitung entgast. Anschließend wird mit 4,9 mL (4,9 mmol, 1 M Lösung in Toluol) Tri-tert-butylphosphin, 633,7 mg (2,82 mmol) Pd(OAc)₂ und 10,2 g (105,87 mmol) t-BuONa versetzt. Die Feststoffe werden zuvor entgast, die Reaktionsmischung wird nachentgast und anschließend unter Rückfluss für 12 h gerührt. Die warme Reaktionslösung wird über Alox B (Aktivitätsstufe 1) filtriert, mit Wasser gewaschen, getrocknet und eingeengt. Die Ausbeute beträgt 29,9 g (33,98 mmol), entsprechend 68 % der Theorie. Der Rückstand wird aus Toluol umkristallisiert und abschließend im Hochvakuum (p = 5 x 10⁻⁵ mbar) sublimiert. Die Reinheit beträgt 99.9%.

Analog können folgende Verbindungen erhalten werden:

| | Edukt 1 | Edukt 2 | Produkt |
|---|---|---|---|
| 9a | 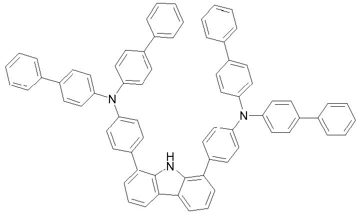 | 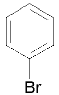 | 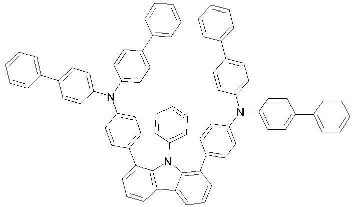 |
| 9b | 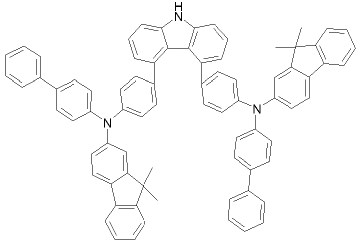 | 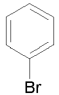 | 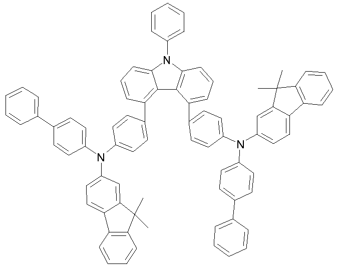 |
| 9c | 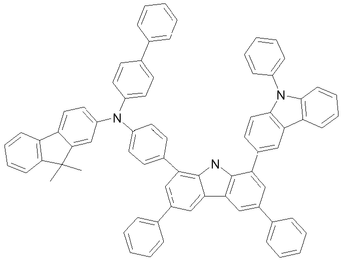 | 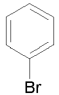 | 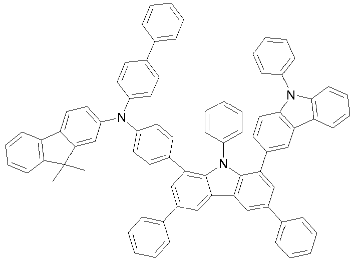 |
| 9d | 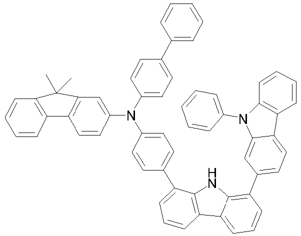 | 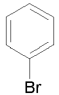 | 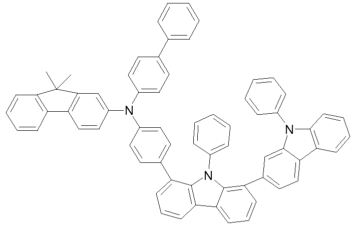 |
| 9e | 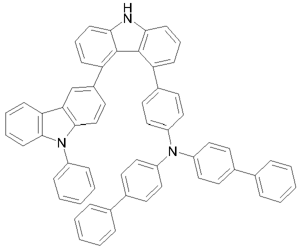 | 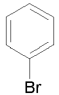 | 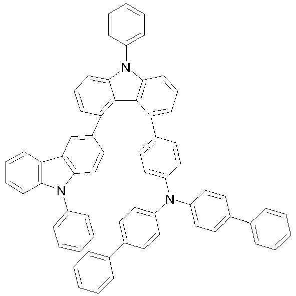 |

| | Ausbeute |
|---|---|
| 9a | 79% |
| 9b | 90% |
| 9c | 79% |
| 9d | 90% |
| 9e | 79% |

### Synthese von Vorstufen:

### Beispiel Int-10 : Bis-biphenyl-4-yl-(6-bromo-dibenzofuran-4-yl)-amin

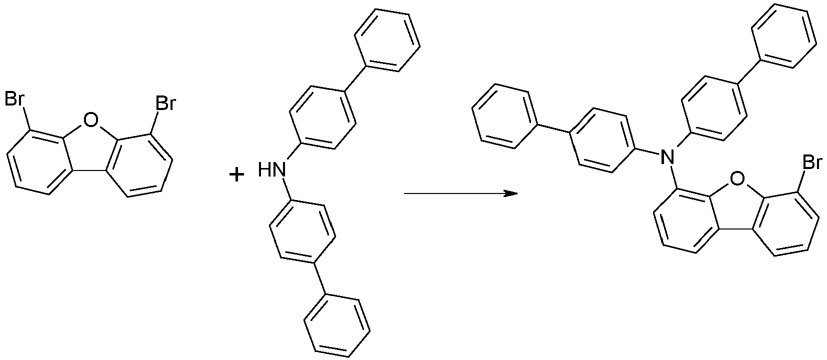

Ein Gemisch aus 16,3 g (50 mmol) 4,6-Dibromo-dibenzofuran, 19,2 g (60 mmol ) Bis-biphenyl-4-yl-amin, 7.7 g (80 mmol) Natrium-tert-butylat, 1.4 g (5 mmol) Tricyclohexylamin, 561 mg (2.5 mmol) Palladium(II)acetat und 300 ml Mesitylen wird 24 h unter Rückfluss erhitzt. Nach Erkalten versetzt man mit 200 ml Wasser, rührt 30 min. nach, trennt die org. Phase ab, filtriert diese über ein kurzes Celite-Bett und entfernt dann das Lösungsmittel im Vakuum. Der Rückstand wird fünfmal aus DMF umkristallisiert und abschließend zweimal fraktioniert sublimiert (p ca. 10⁻⁶ mbar). Ausbeute: 22,9 g (40 mmol), 81 %; Reinheit: 99.9 % n. HPLC.

Analog werden folgende Verbindungen erhalten:

| Bsp. | Edukt | Produkt | | Ausbeute |
|---|---|---|---|---|
| Int-10a | 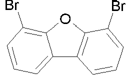 [201138-91-2] | 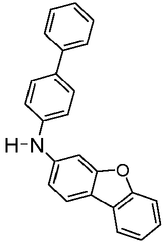 [1290039-85-8] | 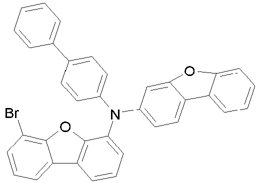 | 65 % |
| Int-10b | 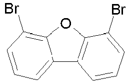 [201138-91-2] | 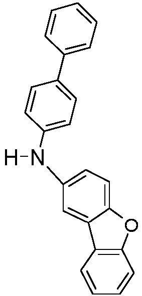 [1300028-94-7 ] | 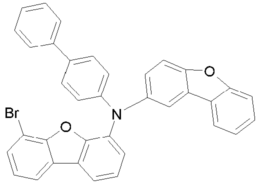 | 69% |
| Int-10c | 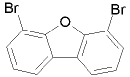 [201 138-91-2] | 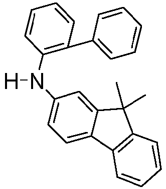 [1198395-24-2] | 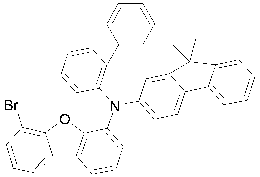 | 76 % |
| Int-10d | 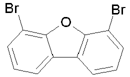 [201138-91-2] | 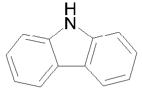 [86-74-8 ] | 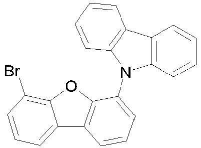 | 75 % |
| Int-10e | 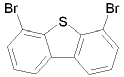 [669773-34-6] | 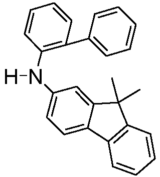 [1198395-24-2] | 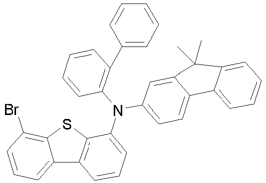 | 71% |

### Synthese von erfindungsgemäßen Verbindungen:

Analog können folgende Verbindungen durch zweite Addition mit entsprechenden Borosäuren erhalten werden:

| Bsp. | Edukt | Produkt | |
|---|---|---|---|
| 10f | 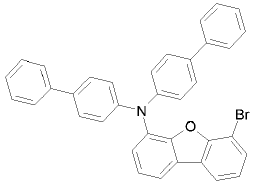 | 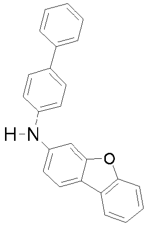 [1290039-85-8] | 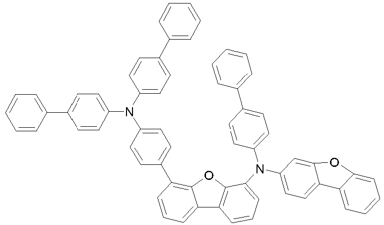 |
| 10g | 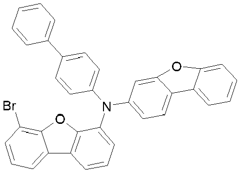 | 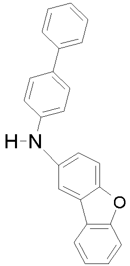 [1300028-94-7 ] | 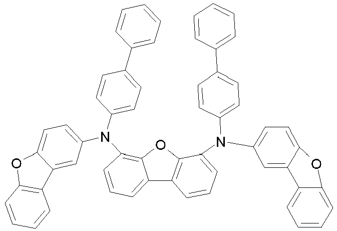 |
| 10h | 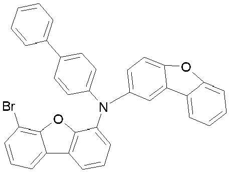 | 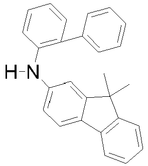 [1198395-24-2] | 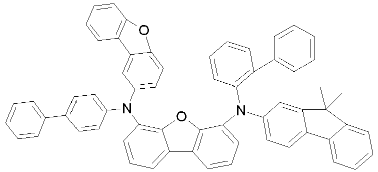 |
| 10i | 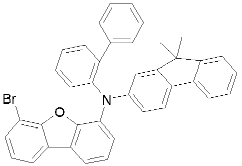 | 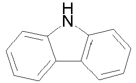 [86-74-8 ] | 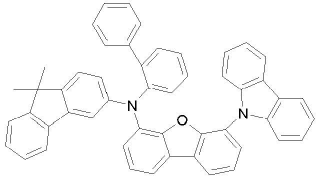 |
| 10j | 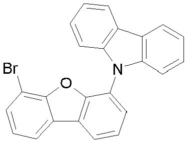 | 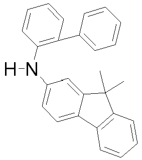 [1198395-24-2] | 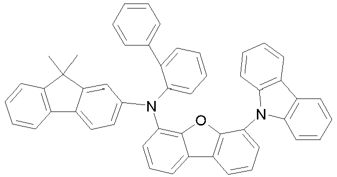 |

| Bsp. | Ausbeute |
|---|---|
| 10f | 65 % |
| 10g | 66% |
| 10h | 76 % |
| 10i | 75 % |
| 10j | 77% |

### B) Devicebeispiele: Herstellung der OLEDs

Die Herstellung der folgenden OLEDs sowie OLEDs nach dem Stand der Technik erfolgt nach einem allgemeinen Verfahren gemäß WO 04/058911, das auf die hier beschriebenen Gegebenheiten (Schichtdickenvariation, Materialien) angepasst wird.

In den folgenden nicht erfindungsgemäßen Beispielen E1 bis E5 und in dern Referenzbeispielen V1 bis V3 werden die Daten verschiedener OLEDs vorgestellt. Als Substrate werden Glasplättchen verwendet, die mit strukturiertem ITO (Indium Zinn Oxid) der Dicke 50 nm beschichtet sind. Die OLEDs haben prinzipiell folgenden Schichtaufbau: Substrat / p-dotierte Lochtransportschicht (HTL1) / Lochtransportschicht (HTL2) / p-dotierte Lochtransportschicht (HTL3) / Lochtransportschicht (HTL4) / Emissionsschicht (EML) / Elektronentransportschicht (ETL) / Elektroneninjektionsschicht (EIL) und abschließend eine Kathode. Die Kathode wird durch eine 100 nm dicke Aluminiumschicht gebildet. Die zur Herstellung der OLEDs benötigten Materialien sind in Tabelle 1 gezeigt, die verschiedenen Bauteilaufbauten in Tabelle 2.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H1:SEB1(5%) bedeutet hierbei, dass das Material H1 in einem Volumenanteil von 95% und SEB1 in einem Anteil von 5% in der Schicht vorliegt. Analog kann auch die Elektronentransportschicht oder die Lochinjektionsschichten aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe EQE @ 10 mA/cm² bezeichnet die externe Quanteneffizienz bei einer Stromdichte von 10mA/cm². LD80 @ 50 mA/cm² (Lebensdauer) ist die Zeit, bis zu die Helligkeit der OLED bei einer Starthelligkeit bei konstantem Strom von 50mA/cm² auf 80 % der Anfangsintensität abgefallen ist.

| Tabelle 1: Strukturen der verwendeten Materialien | | |
|---|---|---|
| 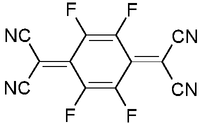 | 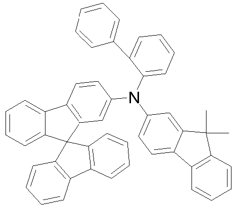 | 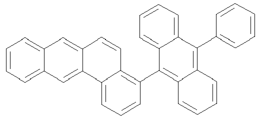 |
| F4TCNQ | HIM1 | H1 |
| 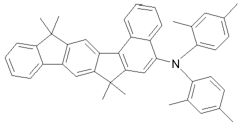 | 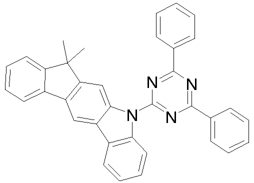 | 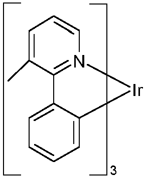 |
| SEB1 | H2 | TEG |
| 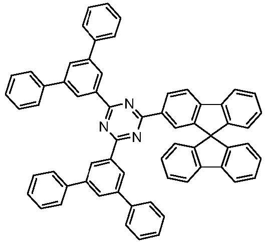 | 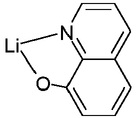 | 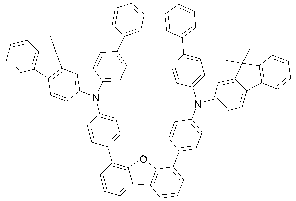 |
| ETM | LiQ | HTM1 |
| 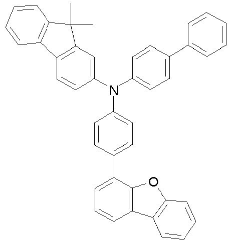 | 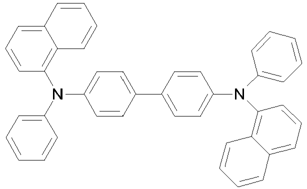 | 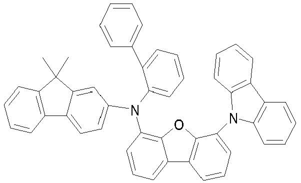 |
| HTMV1 | HTMV2 | HTM2 |
| 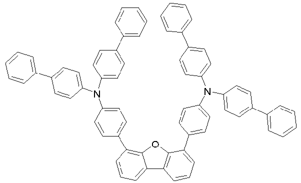 | 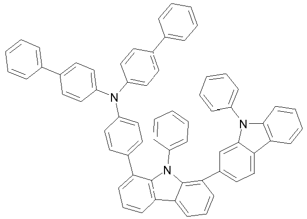 | |
| HTM3 | HTM4 | |

| Tabelle 2: Aufbau der Vorrichtungen | | | | | | | |
|---|---|---|---|---|---|---|---|
| **Bsp.** | **HTL1** | **HTL2** | **HTL3** | **HTL4** | **EML** | **ETL** | **EIL** |
| | Dicke / nm | Dicke / nm | Dicke / nm | Dicke / nm | Dicke / nm | Dicke / nm | Dicke / nm |
| V1 | HIM1: F4TCNQ (3%) 20 nm | HIM1 155 nm | HTMV1: F4TCNQ (3%) 20 nm | HTMV1 20 nm | H1:SEB1(5%) 20 nm | ETM(50%): LiQ(50%) 30 nm | LiQ 1 nm |
| E1 | HIM1: F4TCNQ (3%) 20 nm | HIM1 155 nm | HTM1: F4TCNQ (3%) 20 nm | HTM1 20 nm | H1:SEB1(5%) 20 nm | ETM(50%): LiQ(50%) 30 nm | LiQ 1 nm |
| V2 | HIM1: F4TCNQ (3%) 20 nm | HIM1 160 nm | HTMV1: F4TCNQ (3%) 20 nm | HTMV1 70 nm | H2:TEG(10%) 30 nm | ETM(50%): LiQ(50%) 40 nm | LiQ 1 nm |
| E2 | HIM1: F4TCNQ (3%) 20 nm | HIM1 160 nm | HTM1: F4TCNQ (3%) 20 nm | HTM1 70 nm | H2:TEG(10%) 30 nm | ETM(50%): LiQ(50%) 40 nm | LiQ 1 nm |
| V3 | HIM1: F4TCNQ (3%) 20 nm | HIM1 155 nm | HTMV2: F4TCNQ (3%) 20 nm | HTMV2 20 nm | H1:SEB1(5%) 20 nm | ETM(50%): LiQ(50%) 30 nm | LiQ 1 nm |
| E3 | HIM1: F4TCNQ (3%) 20 nm | HIM1 155 nm | HTM2: F4TCNQ (3%) 20 nm | HTM2 20 nm | H1:SEB1(5%) 20 nm | ETM(50%): LiQ(50%) 30 nm | LiQ 1 nm |
| E4 | HIM1: F4TCNQ (3%) 20 nm | HIM1 155 nm | HTM3: F4TCNQ (3%) 20 nm | HTM3 20 nm | H1:SEB1(5%) 20 nm | ETM(50%): LiQ(50%) 30 nm | LiQ 1 nm |
| E5 | HIM1: F4TCNQ (3%) 20 nm | HIM1 155 nm | HTM4: F4TCNQ (3%) 20 nm | HTM4 20 nm | H1:SEB1(5%) 20 nm | ETM(50%): LiQ(50%) 30 nm | LiQ 1 nm |

### Ergebnisse:

Die Beispiele zeigen die Verwendung der nicht erfindungsgemäßen Verbindung HTM1 als Lochtransportmaterial in einer Lochtransportschicht.

Es wird eine Vergleichsvorrichtung V1 hergestellt und mit der Vorrichtung E1 verglichen. Die Vorrichtung V1 hat hat eine Verbindung gemäß dem Stand der Technik HTMV1 in der Lochtransportschicht HTL3, die Vorrichtung E1 hat eine Lochtransportmaterial in der Lochtransportschicht HTL3. Beide Vorrichtungen E1 und V1 haben eine fluoreszierende Verbindung (SEB1) in der emittierenden Schicht.

Die Referenzvorrichtung V1 hat bei einer Stromdichte von 10 mA/cm² eine externe Quanteneffizienz von 7.8 % und eine Lebensdauer (LD80 @ 50 mA/cm²) von 290 h. Verglichen damit ist bei der Vorrichtung E1 sowohl die externe Quanteneffizienz bei einer Stromdichte von 10 mA/cm² mit 8.4 % höher, als auch die gemessene Lebensdauer (LD80 @ 50 mA/cm²) von 320 h.

Es wird eine Vergleichsvorrichtung V2 hergestellt und mit der Vorrichtung E2 verglichen. Die Vorrichtung V2 hat eine Verbindung gemäß dem Stand der Technik HTMV1 in der Lochtransportschicht HTL3, die Vorrichtung E2 hat eine Verbindung HTM1 als Lochtransportmaterial in der Lochtransportschicht HTL3. Beide Vorrichtungen E1 und V1 haben eine phosphoreszierende Verbindung (TEG) in der emittierenden Schicht.

Im Vergleich zu einer Referenzvorrichtung V2 zeigt die entsprechende Vorrichtung E2 sowohl eine höhere Quanteneffizienz (@ 2 mA/cm²) von 20.4 % gegenüber der Referenzvorrichtung V2 von 19.4% als auch eine höhere Lebensdauer (LD80 @ 20 mA/cm²) von 180 h gegenüber der Referenzvorrichtung E2 von 120 h.

Verglichen mit dem Referenzmaterial HTMV2 (6.2 %, 135 h)) zeigen die Materialien HTM2 (7.4 %, 270h), HTM3 (8.2%, 300h) und HTM4 (7.2%, 210 h) bessere externe Quanteneffizienzen bei 10 mA/cm² und eine bessere Lebendauer (LD80 bei 50 mA/cm²) bei Verwendung als Lochtransportmaterial in einer blau fluoreszierenden OLED.

Die Beispiele zeigen die überraschenden Vorteile bei der Verwendung der Verbindungen HTM1 bis HTM4 mit zwei Arylaminogruppen bzw. einer Arylaminogruppe und einer Carbazolgruppe "face to face" gebunden an eine zentrale Linkergruppe, verglichen mit der Verwendung einer Verbindung mit einer einzigen Arylaminogruppe HTMV1 bzw. der Verwendung der Diaminoverbindung HTMV2.

Die Beispiele zeigen Vorteile bei der Verwendung der Materialien als Lochtransportmaterial in Kombination mit fluoreszierenden und phosphoreszierenden emittierenden Schichten.

Verbindungen mit vergleichbar vorteilhaftem Effekt können auch als Matrixmaterialien in der emittierenden Schicht eingesetzt werden.

### C) Messung der Glasübergangstemperatur

Es werden T_{G}-Messungen durchgeführt gemäß Standard-Verfahren (durchgeführt an einem TA-Instruments Q2000-Serie-Gerät zur DSC-Messung). Dabei werden die folgenden Ergebnisse erhalten (Tabelle 3).

| Tabelle 3: T_{G}-Messungen | |
|---|---|
| Verbindung | Glasübergangstemperatur T_{G} |
| HTMV1 | 111 °C |
| HTM1 | 155 °C |
| HTM2 | 148 °C |
| HTM3 | 113 °C |
| HTM4 | 142 °C |

Es wurde gefunden, dass das Material HTM1 eine hohe Glasübergangstemperatur von 155 °C aufweist, was für die Verwendung in OLEDs sehr vorteilhaft ist. Auch die Verbindungen HTM2 und HTM4 weisen hohe Glasübergangstemperaturen auf. Die Glasübergangstemperatur T_{G} der Vergleichverbindung HTMV1 ist dagegen deutlich niedriger.

## Patentansprüche

1. Elektronische Vorrichtung, enthaltend Anode, Kathode sowie mindestens eine organische Schicht, welche mindestens eine Verbindung der Formel (I-f), (I-k) oder (I-I) enthält 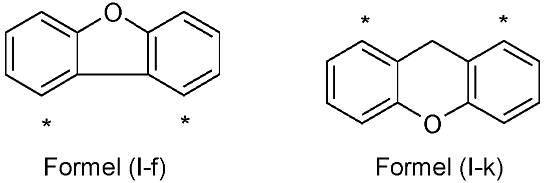 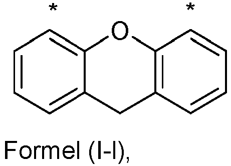 wobei für Formel (I-f) an die mit * gekennzeichneten Positionen eine Gruppe gewählt aus den Gruppen der Formeln 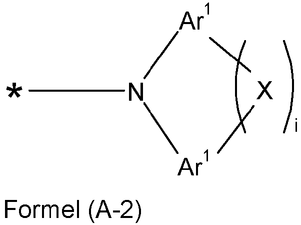 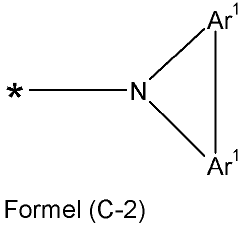 gebunden ist und wobei für Formeln (I-k) und (I-I) an die mit * gekennzeichneten Positionen eine Gruppe gewählt aus den Gruppen der Formeln 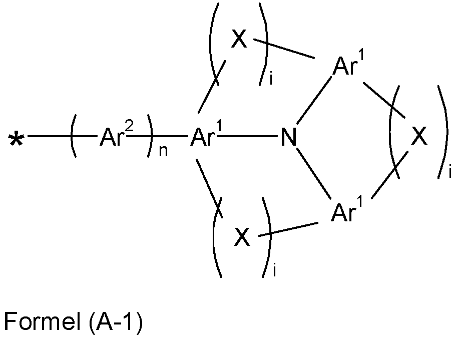 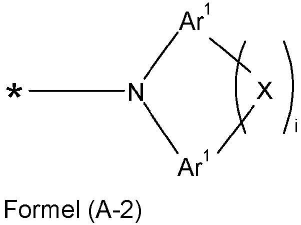 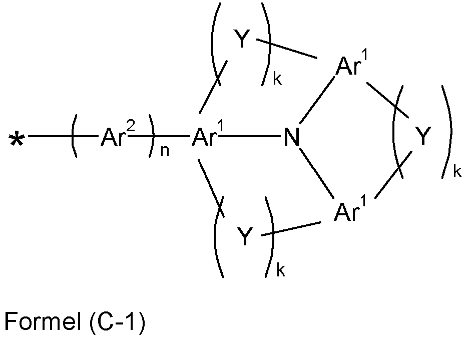 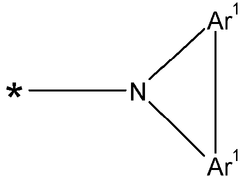 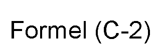 gebunden ist, wobei gilt:
die Verbindungen können an allen freien Positionen an den Formeln (l-f), (I-k) und (I-I) mit Resten R¹ substituiert sein, und
Ar¹ ist bei jedem Auftreten gleich oder verschieden gewählt aus Aryl- oder Heteroarylgruppen mit 6 bis 13 aromatischen Ringatomen, die mit einem oder mehreren Resten R¹ substituiert sein können;
Ar² ist bei jedem Auftreten gleich oder verschieden gewählt aus Aryl- oder Heteroarylgruppen mit 6 bis 13 aromatischen Ringatomen, die mit einem oder mehreren Resten R¹ substituiert sein können;
X ist bei jedem Auftreten gleich oder verschieden gewählt aus C(R¹)₂, Si(R¹)₂, C=O, O, S, S=O, SO₂ und NR¹;
Y ist eine Einfachbindung;
R¹ ist bei jedem Auftreten gleich oder verschieden H, D, F, C(=O)R², CN, Si(R²)₃, N(R²)₂, P(=O)(R²)₂, S(=O)R², S(=O)₂R², eine geradkettige Alkyl- oder Alkoxygruppe mit 1 bis 20 C-Atomen oder eine verzweigte oder cyclische Alkyl-, oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R² substituiert sein können und wobei eine oder mehrere CH₂-Gruppen in den oben genannten Gruppen durch - R²C=CR²-, -C=C-, Si(R²)₂, C=O, C=S, C=NR², -C(=O)O-, -C(=O)NR²-, NR², P(=O)(R²), -O-, -S-, SO oder SO₂ ersetzt sein können und wobei ein oder mehrere H-Atome in den oben genannten Gruppen durch D, F oder CN ersetzt sein können, oder ein aromatisches oder heteroaromatisches Ringsystem mit 5 bis 30 aromatischen Ringatomen, das durch einen oder mehrere Reste R² substituiert sein kann, oder eine Aryloxy- oder Heteroaryloxygruppe mit 5 bis 30 aromatischen Ringatomen, die durch einen oder mehrere Reste R² substituiert sein kann, wobei zwei oder mehr Reste R¹ miteinander verknüpft sein können und einen Ring bilden können;
R² ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können; dabei können zwei oder mehr Substituenten R² miteinander verknüpft sein und einen Ring bilden;
n ist bei jedem Auftreten gleich oder verschieden 0, 1, 2, 3 oder 4;
i ist bei jedem Auftreten gleich oder verschieden 0 oder 1;
k ist bei jedem Auftreten gleich oder verschieden 0 oder 1, wobei mindestens ein Index k pro Gruppe der Formel (C-1) gleich 1 sein muss;
und wobei an mindestens eine der mit * gekennzeichneten Positionen eine Gruppe gewählt aus Gruppen der Formeln (A-1) oder (A-2) gebunden sein muss,
und wobei weiterhin keine kondensierte Aryl- oder Heteroarylgruppe mit 14 oder mehr aromatischen Ringatomen in der Verbindung vorhanden ist.

2. Elektronische Vorrichtung nach Anspruch 1, **dadurch gekennzeichnet, dass** sie Formel (I-f) entspricht, und dass an beide mit * gekennzeichnete Positionen eine Gruppe gewählt aus Gruppen der Formeln (A-2) gebunden ist.

3. Elektronische Vorrichtung nach Anspruch 1 oder 2, **dadurch gekennzeichnet, dass** die Gruppe Ar¹ bei jedem Auftreten gleich oder verschieden gewählt ist aus Aryl- oder Heteroarylgruppen mit 6 bis 10 aromatischen Ringatomen, die mit einem oder mehreren Resten R¹ substituiert sein können.

4. Elektronische Vorrichtung nach einem oder mehreren der Ansprüche 1 oder 3, **dadurch gekennzeichnet, dass** die Gruppe Ar² bei jedem Auftreten gleich oder verschieden gewählt ist aus Aryl- oder Heteroarylgruppen mit 6 bis 10 aromatischen Ringatomen, die mit einem oder mehreren Resten R¹ substituiert sein können.

5. Elektronische Vorrichtung nach einem oder mehreren der Ansprüche 1 bis 4, **dadurch gekennzeichnet, dass** R¹ bei jedem Auftreten gleich oder verschieden H, D, F, CN, Si(R²)₃, N(R²)₂, eine geradkettige Alkyl- oder Alkoxygruppe mit 1 bis 20 C-Atomen oder eine verzweigte oder cyclische Alkyl- oder Alkoxygruppe mit 3 bis 20 C-Atomen ist, wobei die oben genannten Gruppen jeweils mit einem oder mehreren Resten R² substituiert sein können und wobei in den oben genannten Gruppen eine oder mehrere CH₂-Gruppen durch -C=C-, -R²C=CR²-, Si(R²)₂, C=O, C=NR², -NR²-, -O-, -S-, -C(=O)O- oder -C(=O)NR²-ersetzt sein können, oder ein aromatisches oder heteroaromatisches Ringsystem mit 5 bis 20 aromatischen Ringatomen ist, das mit einem oder mehreren Resten R² substituiert sein kann, wobei zwei oder mehr Reste R¹ miteinander verknüpft sein können und einen Ring bilden können.

6. Elektronische Vorrichtung nach einem oder mehreren der Ansprüche 1, 3 bis 5, **dadurch gekennzeichnet, dass** der Index n bei jedem Auftreten gleich oder verschieden 0 oder 1 ist.

7. Elektronische Vorrichtung nach einem oder mehreren der Ansprüche 1 bis 6, **dadurch gekennzeichnet, dass** der Index i bei jedem Auftreten gleich 0 ist.

8. Elektronische Vorrichtung nach einem oder mehreren der Ansprüche 1 bis 7, **dadurch gekennzeichnet, dass** sie einer Formel gewählt aus den Formeln (I-k) und (I-I) entspricht.

9. Elektronische Vorrichtung nach einem oder mehreren der Ansprüche 1 bis 8, **dadurch gekennzeichnet, dass** die Verbindungen der Formeln (I-f), (I-k) und (I-I) zusätzlich zu den Gruppen (A-1), (A-2), (C-1) und (C-2) nach Anspruch 1 keine weiteren Arylaminogruppen aufweisen.

10. Elektronische Vorrichtung nach einem oder mehreren der Ansprüche 1 bis 9, **dadurch gekennzeichnet, dass** die Verbindungen der Formeln (I-f), (I-k) und (I-l) zusätzlich zu den Gruppen (A-1), (A-2), (C-1) und (C-2) nach Anspruch 1 keine weiteren Carbazolgruppen aufweisen.

11. Elektronische Vorrichtung nach einem oder mehreren der Ansprüche 1 bis 10, **dadurch gekennzeichnet, dass** die Verbindungen der Formeln (I-k) und (I-I) sind, wobei an die mit * gekennzeichneten Positionen eine Gruppe gewählt aus den Gruppen der Formeln (A-1) und (A-2) nach Anspruch 1 gebunden ist, und wobei alle weiteren Gruppen definiert sind wie in Anspruch 1.

12. Elektronische Vorrichtung nach einem oder mehreren der Ansprüche 1 bis 11, **dadurch gekennzeichnet, dass** die elektronische Vorrichtung eine organische Elektrolumineszenzvorrichtung, OLED, ist.

13. Elektronische Vorrichtung nach Anspruch 12, **dadurch gekennzeichnet, dass** die organische Schicht enthaltend die Verbindung der Formel (I-f), (I-k) oder (I-I) eine Schicht mit lochtransportierender Funktion oder eine emittierende Schicht ist.

14. Verwendung einer elektronischen Vorrichtung nach einem oder mehreren der Ansprüche 1 bis 13 in Displays und/oder als Lichtquelle in Beleuchtungsanwendungen und/oder als Lichtquelle in medizinischen oder kosmetischen Anwendungen.

## Claims

1. Electronic device, comprising anode, cathode and at least one organic layer which comprises at least one compound of the formula (I-f), (I-k) or (I-I) 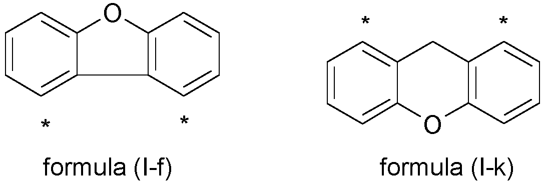 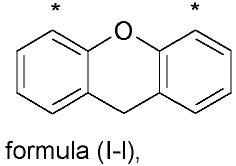 where, for formula (I-f), a group selected from the groups of the formulae 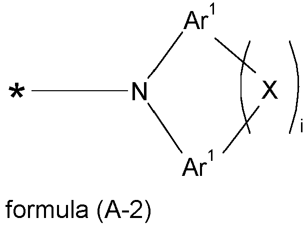 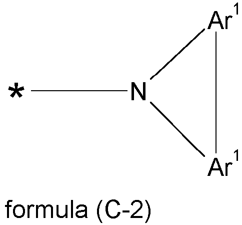 is bonded at the positions denoted by * and where, for formulae (I-k) and (I-l), a group selected from the groups of the formulae 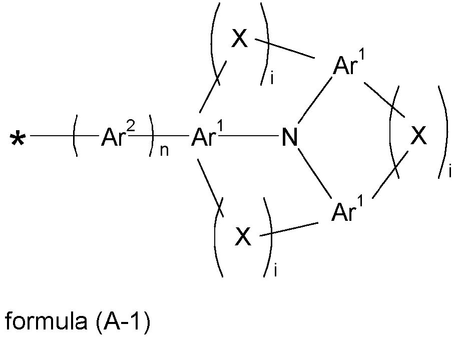 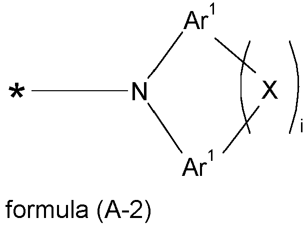 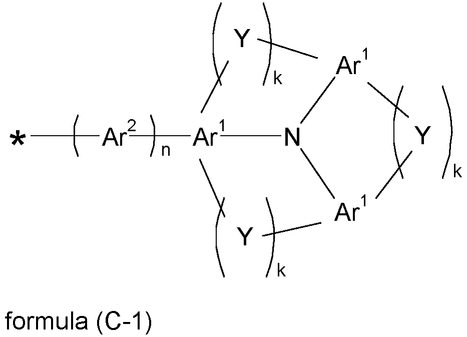 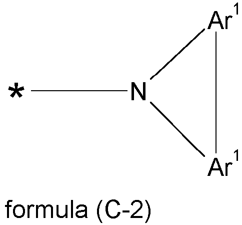 is bonded at the positions denoted by *, where:
the compounds may be substituted by radicals R¹ at all free positions on the formulae (I-f), (I-k) and (I-l), and
Ar¹ is selected on each occurrence, identically or differently, from aryl or heteroaryl groups having 6 to 13 aromatic ring atoms, which may be substituted by one or more radicals R¹;
Ar² is selected on each occurrence, identically or differently, from aryl or heteroaryl groups having 6 to 13 aromatic ring atoms, which may be substituted by one or more radicals R¹;
X is selected on each occurrence, identically or differently, from C(R¹)₂, Si(R¹)₂, C=O, O, S, S=O, SO₂ and NR¹;
Y is a single bond;
R¹ is on each occurrence, identically or differently, H, D, F, C(=O)R², CN, Si(R²)₃, N(R²)₂, P(=O)(R²)₂, S(=O)R², S(=O)₂R², a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may in each case be substituted by one or more radicals R² and where one or more CH₂ groups in the above-mentioned groups may be replaced by -R²C=CR²-, -C≡C-, Si(R²)₂, C=O, C=S, C=NR², -C(=O)O-, -C(=O)NR²-, NR², P(=O)(R²), -O-, -S-, SO or SO₂ and where one or more H atoms in the above-mentioned groups may be replaced by D, F or CN, or an aromatic or heteroaromatic ring system having 5 to 30 aromatic ring atoms, which may be substituted by one or more radicals R², or an aryloxy or heteroaryloxy group having 5 to 30 aromatic ring atoms, which may be substituted by one or more radicals R², where two or more radicals R¹ may be linked to one another and may form a ring;
R² is on each occurrence, identically or differently, H, D, F or an aliphatic, aromatic or heteroaromatic organic radical having 1 to 20 C atoms, in which, in addition, one or more H atoms may be replaced by D or F; two or more substituents R² may be linked to one another and may form a ring;
n is on each occurrence, identically or differently, 0, 1, 2, 3 or 4;
i is on each occurrence, identically or differently, 0 or 1;
k is on each occurrence, identically or differently, 0 or 1, where at least one index k per group of the formula (C-1) must be equal to 1;
and where a group selected from groups of the formulae (A-1) or (A-2) must be bonded at at least one of the positions denoted by *,
and where furthermore no condensed aryl or heteroaryl group having 14 or more aromatic ring atoms is present in the compound.

2. Electronic device according to Claim 1, **characterised in that** it corresponds to formula (I-f), and that a group selected from groups of the formula (A-2) is bonded at both positions denoted by *.

3. Electronic device according to Claim 1 or 2, **characterised in that** the group Ar¹ is selected on each occurrence, identically or differently, from aryl or heteroaryl groups having 6 to 10 aromatic ring atoms, which may be substituted by one or more radicals R¹.

4. Electronic device according to one or more of Claims 1 or 3, **characterised in that** the group Ar² is selected on each occurrence, identically or differently, from aryl or heteroaryl groups having 6 to 10 aromatic ring atoms, which may be substituted by one or more radicals R¹.

5. Electronic device according to one or more of Claims 1 to 4, **characterised in that** R¹ is on each occurrence, identically or differently, H, D, F, CN, Si(R²)₃, N(R²)₂, a straight-chain alkyl or alkoxy group having 1 to 20 C atoms or a branched or cyclic alkyl or alkoxy group having 3 to 20 C atoms, where the above-mentioned groups may in each case be substituted by one or more radicals R² and where one or more CH₂ groups in the above-mentioned groups may be replaced by
-C≡C-, -R²C=CR²-, Si(R²)₂, C=O, C=NR², -NR²-, -O-, -S-, -C(=O)O- or -C(=O)NR²-, or is an aromatic or heteroaromatic ring system having 5 to 20 aromatic ring atoms, which may be substituted by one or more radicals R², where two or more radicals R¹ may be linked to one another and may form a ring.

6. Electronic device according to one or more of Claims 1, 3 to 5, **characterised in that** the index n is on each occurrence, identically or differently, 0 or 1.

7. Electronic device according to one or more of Claims 1 to 6, **characterised in that** the index i is on each occurrence equal to 0.

8. Electronic device according to one or more of Claims 1 to 7, **characterised in that** it corresponds to a formula selected from the formulae (I-k) and (I-l).

9. Electronic device according to one or more of Claims 1 to 8, **characterised in that** the compounds of the formulae (I-f), (I-k) and (I-I) contain no further arylamino groups in addition to the groups (A-1), (A-2), (C-1) and (C-2) according to Claim 1.

10. Electronic device according to one or more of Claims 1 to 9, **characterised in that** the compounds of the formulae (I-f), (I-k) and (I-I) contain no further carbazole groups in addition to the groups (A-1), (A-2), (C-1) and (C-2) according to Claim 1.

11. Electronic device according to one or more of Claims 1 to 10, **characterised in that** the compounds are of the formulae (I-k) and (I-I), where a group selected from the groups of the formulae (A-1) and (A-2) according to Claim 1 is bonded at the positions denoted by *, and where all other groups are defined as in Claim 1.

12. Electronic device according to one or more of Claims 1 to 11, **characterised in that** the electronic device is an organic electroluminescent device, OLED.

13. Electronic device according to Claim 12, **characterised in that** the organic layer comprising the compound of the formula (I-f), (I-k) or (I-I) is a layer having a hole-transporting function or an emitting layer.

14. Use of an electronic device according to one or more of Claims 1 to 13 in displays and/or as light source in lighting applications and/or as light source in medical or cosmetic applications.

## Revendications

1. Dispositif électronique, comprenant une anode, une cathode et au moins une couche organique qui comprend au moins un composé de formule (I-f), (I-k) ou (I-l) 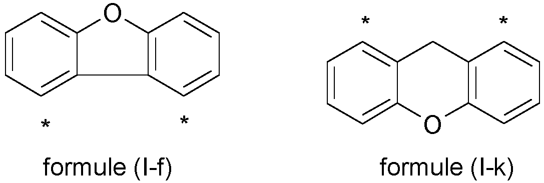 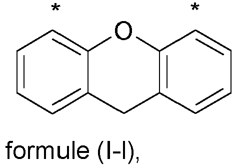 où, pour la formule (I-f), un groupement choisi parmi les groupements de formules 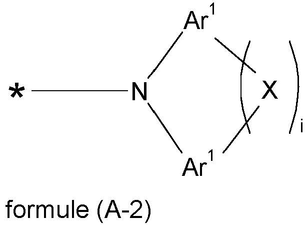 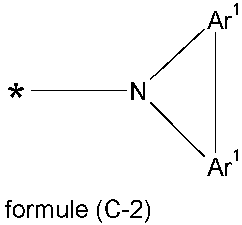 est lié au niveau des positions désignées par * et où, pour les formules (I-k) et (I-l), un groupement choisi parmi les groupements de formules 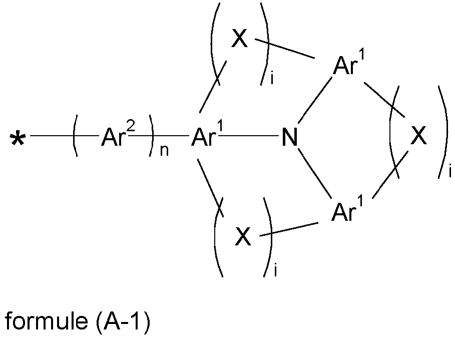 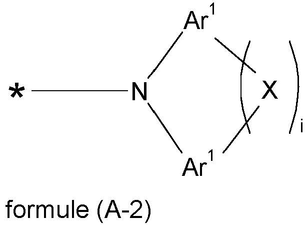 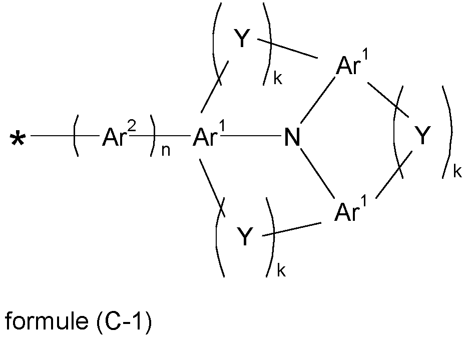 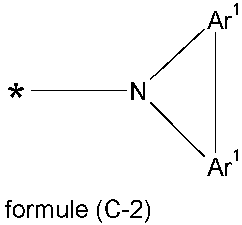 est lié au niveau des positions désignées par *, où :
les composés peuvent être substitués par des radicaux R¹ au niveau de toutes les positions libres dans les formules (I-f), (I-k) et (I-l), et
Ar¹ est choisi à chaque occurrence, de manière identique ou différente, parmi des groupements aryle ou hétéroaryle ayant de 6 à 13 atomes de cycle aromatique, qui peuvent être substitués par un ou plusieurs radicaux R¹ ;
Ar² est choisi à chaque occurrence, de manière identique ou différente, parmi des groupements aryle ou hétéroaryle ayant de 6 à 13 atomes de cycle aromatique, qui peuvent être substitués par un ou plusieurs radicaux R¹ ;
X est choisi à chaque occurrence, de manière identique ou différente, parmi C(R¹)₂, Si(R¹)₂, C=O, O, S, S=O, SO₂ et NR¹ ;
Y est une liaison simple ;
R¹ est à chaque occurrence, de manière identique ou différente, H, D, F, C(=O)R², CN, Si(R²)₃, N(R²)₂, P(=O)(R²)₂, S(=O)R², S(=O)₂R², un groupement alkyle ou alcoxy à chaîne linéaire ayant de 1 à 20 atomes de C ou un groupement alkyle ou alcoxy ramifié ou cyclique ayant de 3 à 20 atomes de C ou un groupement alcényle ou alcynyle ayant de 2 à 20 atomes de C, où les groupements susmentionnés peuvent dans chaque cas être substitués par un ou plusieurs radicaux R² et où un ou plusieurs groupements CH₂ dans les groupements susmentionnés peuvent être remplacés par -R²C=CR²-, -C=C-, Si(R²)₂, C=O, C=S, C=NR², -C(=O)O-, -C(=O)NR²-, NR², P(=O)(R²), -O-, -S-, SO ou SO₂ et où un ou plusieurs atomes de H dans les groupements susmentionnés peuvent être remplacés par D, F ou CN, ou un noyau aromatique ou hétéroaromatique ayant de 5 à 30 atomes de cycle aromatique, qui peut être substitué par un ou plusieurs radicaux R², ou un groupement aryloxy ou hétéroaryloxy ayant de 5 à 30 atomes de cycle aromatique, qui peut être substitué par un ou plusieurs radicaux R², où deux, ou plus, radicaux R¹ peuvent être liés les uns aux autres et peuvent former un cycle ;
R² est à chaque occurrence, de manière identique ou différente, H, D, F ou un radical organique aliphatique, aromatique ou hétéroaromatique ayant de 1 à 20 atomes de C, où, de plus, un ou plusieurs atomes de H peuvent être remplacés par D ou F ; deux, ou plus, substituants R² peuvent être liés les uns aux autres et peuvent former un cycle ;
n vaut à chaque occurrence, de manière identique ou différente, 0, 1, 2, 3 ou 4 ;
i vaut à chaque occurrence, de manière identique ou différente, 0 ou 1 ;
k vaut à chaque occurrence, de manière identique ou différente, 0 ou 1, où au moins un indice k par groupement de formule (C-1) doit être égal à 1 ;
et où un groupement choisi parmi les groupements de formules (A-1) ou (A-2) doit être lié au niveau d'au moins l'une parmi les positions désignées par *,
et où en outre aucun groupement aryle ou hétéroaryle condensé ayant 14, ou plus, atomes de cycle aromatique n'est présent dans le composé.

2. Dispositif électronique selon la revendication 1, **caractérisé en ce qu'**il correspond à la formule (I-f), et qu'un groupement choisi parmi les groupements de formule (A-2) est lié au niveau des deux positions désignées par *.

3. Dispositif électronique selon la revendication 1 ou 2, **caractérisé en ce que** le groupement Ar¹ est choisi à chaque occurrence, de manière identique ou différente, parmi des groupements aryle ou hétéroaryle ayant de 6 à 10 atomes de cycle aromatique, qui peuvent être substitués par un ou plusieurs radicaux R¹.

4. Dispositif électronique selon l'une ou plusieurs parmi les revendications 1 ou 3, **caractérisé en ce que** le groupement Ar² est choisi à chaque occurrence, de manière identique ou différente, parmi des groupements aryle ou hétéroaryle ayant de 6 à 10 atomes de cycle aromatique, qui peuvent être substitués par un ou plusieurs radicaux R¹.

5. Dispositif électronique selon l'une ou plusieurs parmi les revendications 1 à 4, **caractérisé en ce que** R¹ est à chaque occurrence, de manière identique ou différente, H, D, F, CN, Si(R²)₃, N(R²)₂, un groupement alkyle ou alcoxy à chaîne linéaire ayant de 1 à 20 atomes de C ou un groupement alkyle ou alcoxy ramifié ou cyclique ayant de 3 à 20 atomes de C, où les groupements susmentionnés peuvent dans chaque cas être substitués par un ou plusieurs radicaux R² et où un ou plusieurs groupements CH₂ dans les groupements susmentionnés peuvent être remplacés par -C≡C-, -R²C=CR²-, Si(R²)₂, C=O, C=NR², -NR²-, -O-, -S-, -C(=O)O- ou -C(=O)NR²-, ou est un noyau aromatique ou hétéroaromatique ayant de 5 à 20 atomes de cycle aromatique, qui peut être substitué par un ou plusieurs radicaux R², où deux, ou plus, radicaux R¹ peuvent être liés les uns aux autres et peuvent former un cycle.

6. Dispositif électronique selon l'une ou plusieurs parmi les revendications 1, 3 à 5, **caractérisé en ce que** l'indice n vaut à chaque occurrence, de manière identique ou différente, 0 ou 1.

7. Dispositif électronique selon l'une ou plusieurs parmi les revendications 1 à 6, **caractérisé en ce que** l'indice i est à chaque occurrence égal à 0.

8. Dispositif électronique selon l'une ou plusieurs parmi les revendications 1 à 7, **caractérisé en ce qu'**il correspond à une formule choisie parmi les formules (I-k) et (I-l).

9. Dispositif électronique selon l'une ou plusieurs parmi les revendications 1 à 8, **caractérisé en ce que** les composés de formules (I-f), (I-k) et (I-l) ne contiennent pas d'autres groupements arylamino outre les groupements (A-1), (A-2), (C-1) et (C-2) selon la revendication 1.

10. Dispositif électronique selon l'une ou plusieurs parmi les revendications 1 à 9, **caractérisé en ce que** les composés de formules (I-f), (I-k) et (I-l) ne contiennent pas d'autres groupements carbazole outre les groupements (A-1), (A-2), (C-1) et (C-2) selon la revendication 1.

11. Dispositif électronique selon l'une ou plusieurs parmi les revendications 1 à 10, **caractérisé en ce que** les composés répondent aux formules (I-k) et (I-l), où un groupement choisi parmi les groupements de formules (A-1) et (A-2) selon la revendication 1 est lié au niveau des positions désignées par *, et où tous les autres groupements sont tels que définis selon la revendication 1.

12. Dispositif électronique selon l'une ou plusieurs parmi les revendications 1 à 11, **caractérisé en ce que** le dispositif électronique est un dispositif électroluminescent organique, OLED.

13. Dispositif électronique selon la revendication 12, **caractérisé en ce que** la couche organique comprenant le composé de formule (I-f), (I-k) ou (I-l) est une couche ayant une fonction de transport de trous ou une couche émettrice.

14. Utilisation d'un dispositif électronique selon l'une ou plusieurs parmi les revendications 1 à 13 dans des affichages et/ou comme source lumineuse dans des applications d'éclairage et/ou comme source lumineuse dans des applications médicales ou cosmétiques.
